(19) Europäisches Patentamt / European Patent Office / Office européen des brevets

(11) EP 4 710 945 A1

(12) EUROPEAN PATENT APPLICATION
published in accordance with Art. 153(4) EPC

(43) Date of publication:
**18.03.2026 Bulletin 2026/12**

(21) Application number: **24803036.3**

(22) Date of filing: **09.05.2024**

(51) International Patent Classification (IPC):
***A61K 47/68*** (2017.01) ***C07D 491/22*** (2006.01)
***C07K 16/28*** (2006.01) ***A61P 35/00*** (2006.01)

(52) Cooperative Patent Classification (CPC):
**A61K 47/68; A61P 35/00; C07D 491/22; C07K 16/28**

(86) International application number:
**PCT/CN2024/091817**

(87) International publication number:
**WO 2024/230756 (14.11.2024 Gazette 2024/46)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **09.05.2023 CN 202310519224**
**10.10.2023 CN 202311314455**

(71) Applicants:
- **Shanghai Henlius Biotech, Inc.**
  **Shanghai 201210 (CN)**
- **Shanghai Henlius Biologics Co., Ltd.**
  **Shanghai 201616 (CN)**

(72) Inventors:
- **SHAN, Yongqiang**
  **Shanghai 201210 (CN)**
- **SONG, Hongbin**
  **Shanghai 201210 (CN)**
- **SONG, Ge**
  **Shanghai 201210 (CN)**
- **JIANG, Jiahao**
  **Shanghai 201210 (CN)**
- **ZHANG, Wentao**
  **Shanghai 201210 (CN)**

(74) Representative: **Plougmann Vingtoft a/s**
**Strandvejen 70**
**2900 Hellerup (DK)**

Remarks:
The complete document including Reference Table(s) and the Sequence Listing(s) can be downloaded from the EPO website

# (54) ANTI-EPIDERMAL GROWTH FACTOR RECEPTOR (EGFR) ANTIBODY-DRUG CONJUGATE AND USE THEREOF

(57) An antibody-drug conjugate (ADC) and the use thereof. Specifically, the present invention relates to a novel anti-epidermal growth factor receptor (EGFR) antibody-drug conjugate, and a method of using same and the use thereof in the treatment of EGFR-related diseases or disorders.

EP 4 710 945 A1

## Description

The present application claims priority to Chinese Patent Application No. CN 202310519224.2 filed on May 9, 2023 and Chinese Patent Application No. CN 202311314455.6 filed on October 10, 2023.

## Technical Field

The present application belongs to the pharmaceutical field and relates to an antibody-drug conjugate (ADC) and the use thereof. Specifically, the present application relates to a novel anti-epidermal growth factor receptor antibody-drug conjugate, and a method of using same and the use thereof in the treatment of EGFR-related diseases or disorders.

## Background Art

Human epidermal growth factor receptor (also known as HER-1 or Erb-B1, and referred to herein as "EGFR") is a 170 kDa transmembrane receptor encoded by the c-erbB proto-oncogene and exhibits intrinsic tyrosine kinase activity (Modjtahedi et al., Br. J. Cancer 73: 228-235 (1996); Herbst and Shin, Cancer 94: 1593-1611(2002)). EGFR is a member of the epidermal growth factor receptor family and plays an important role in cell growth, development, and differentiation. EGFR regulates a variety of cellular processes through tyrosine kinase-mediated signal transduction pathways, including but not limited to activation of signal transduction pathways that control cell proliferation, differentiation, cell survival, apoptosis, angiogenesis, mitogenesis, and metastasis (Atalay et al., Ann. Oncology 14: 1346-1363 (2003); Tsao and Herbst, Signal 4: 4-9 (2003); Herbst and Shin, Cancer 94: 1593-1611 (2002); Modjtahedi et al., Br. J. Cancer 73: 228-235 (1996)).

EGFR is highly expressed in a variety of solid tumors, and among drugs targeting EGFR, several blockbuster drugs such as osimertinib have emerged over the past three decades. However, since small molecule therapies often lead to mutations in target genes, leading to treatment failure, there is a continuous need to develop a new generation of anti-mutation small molecule drugs as EGFR inhibitors. Among antibody drugs, cetuximab was approved in 2004 and found effective for colorectal cancer, nasopharyngeal cancer, etc. However, it is also necessary to combine cetuximab with chemotherapy to enhance therapeutic efficacy.

Therefore, there is an urgent need in the art for humanized anti-epidermal growth factor receptor drugs with higher biological activity, especially drugs that are efficacious in patients, e.g., those with KRAS mutant or TKI resistance, such as antibody-drug conjugates, so as to further improve efficacy and reduce side effects. The development of macromolecular drugs targeting EGFR, as an important kinase target, has also presented novel drug molecules with different anti-cancer mechanisms of action. EGFR-targeted antibody-drug conjugates are one of the hotspots in the development of new-generation targeted drugs. An antibody-drug conjugate (ADC) consists of a monoclonal antibody (Antibody) that targets a tumorspecific antigen or a tumor-associated antigen, which monoclonal antibody is conjugated to a small molecule toxin (Payload) having high anti-cancer activity via a linker (Linker). The antibody-drug conjugate has dual advantages, i.e., both the high targeting ability of the monoclonal antibody drug and the high activity of the cytotoxin in a tumor tissue, so that the advantages of the antibody drug and the small molecule drug payload work synergistically to more efficiently and specifically kill tumor cells. Antibody-drug conjugates have less toxic and side effects than chemotherapeutic drugs alone and better efficacy than traditional antibody-based oncology drugs.

## Summary of the Invention

The present application relates to an antibody-drug conjugate of formula (I) as defined herein and a pharmaceutical composition comprising the antibody-drug conjugate. The present application is characterized by a method for treating or preventing an EGFR-related disease or disorder, comprising administering to a subject in need thereof a therapeutically effective amount of the antibody-drug conjugate of formula (I), the prodrug, pharmaceutically acceptable salt, or solvate thereof, or the solvate of the pharmaceutically acceptable salt, as defined herein. The method of the present application can be used for treating or preventing an EGFR-related disease or disorder.

A first aspect of the present application relates to an antibody-drug conjugate, a prodrug, pharmaceutically acceptable salt, or solvate thereof, or a solvate of the pharmaceutically acceptable salt, wherein the antibody-drug conjugate has a structure of formula (I):

$$TP\text{-}[L1\text{-}L2\text{-}L3\text{-}D]_k \qquad (I)$$

wherein:

TP is a targeting moiety selectively binding or recognizing EGFR;

L1 is an extension unit connecting TP and L2;
L2 is an optional amino acid residue or a short peptide consisting of 2-10 amino acid residues;
L3 is a spacer element;
D is a biologically active molecule; and
k represents any numerical value between about 0.1 and about 10.0.

Another aspect of the present application relates to a pharmaceutical composition comprising the antibody-drug conjugate of formula (I), the prodrug, pharmaceutically acceptable salt, or solvate thereof, or the solvate of the pharmaceutically acceptable salt, and optionally, a pharmaceutically acceptable carrier, diluent, or excipient.

A further aspect of the present application relates to a method for treating an EGFR-related disease or disorder. The method comprises administering to a subject in need thereof the antibody-drug conjugate of formula (I), the prodrug, pharmaceutically acceptable salt, or solvate thereof, or the solvate of the pharmaceutically acceptable salt, or a pharmaceutical composition comprising same. The EGFR-related disease or disorder is selected from epidermal cancer, colon cancer, rectal cancer, head and neck cancer, lung cancer, ovarian cancer, cervical cancer, bladder cancer, esophageal cancer, breast cancer, kidney cancer, prostate cancer, gastric cancer, pancreatic cancer, and glioma.

A further aspect of the present application relates to the use of the antibody-drug conjugate of formula (I), the prodrug, pharmaceutically acceptable salt, or solvate thereof, or the solvate of the pharmaceutically acceptable salt, or the pharmaceutical composition comprising same in the preparation of a medicament.

The present application further provides an antibody-drug conjugate and composition with improved potency and/or anti-tumor activity as compared with anti-EGFR-ADCs known in the prior art.

The details of the present application are set forth in the accompanying description below. Although methods and materials similar or equivalent to those described herein may be used in the practice or testing of the present application, illustrative methods and materials are now described. Other features, objects, and advantages of the present application will be apparent from the description and the claims. In the description and the appended claims, singular forms also include plural forms, unless the context clearly dictates otherwise. Unless otherwise defined, all technical and scientific terms used herein have the same meanings as commonly understood by those of ordinary skill in the field to which the present application belongs. All patents and publications cited in the description are incorporated herein by reference in their entirety.

The contents of all references (including literature references, granted patents, published patent applications, and co-pending patent applications) cited throughout the present application are expressly incorporated herein by reference in their entirety. Unless defined otherwise, all technical and scientific terms used herein have the meanings as commonly understood by those of ordinary skill in the art.

## Brief Description of the Drawings

FIG. 1 is a graph showing the affinity of an anti-EGFR antibody-drug conjugate for EGFR-positive cells A549 and SW480. (A) Affinity of anti-EGFR antibody-drug conjugate MAB07-LP62 (DAR4 or DAR8) for A549 cells. (B) Affinity of anti-EGFR antibody-drug conjugate MAB07-LP62 (DAR8) for SW480 cells. Note: MAB07 represents an anti-EGFR antibody, the amino acid sequence of which is set forth in the Sequence Listing; MAB07-LP62 represents an antibody-drug conjugate formed by conjugation of MAB07 to LP62; DAR represents the loaded drug : antibody ratio; IgG1 represents a human IgG1, κ isotype control.

FIG. 2 is a graph showing that the anti-EGFR antibody-drug conjugate is internalized by EGFR-positive cells A549 as the incubation time increases. Note: MAB07 represents an anti-EGFR antibody, the amino acid sequence of which is set forth in the Sequence Listing; MAB07-LP62 represents an antibody-drug conjugate formed by conjugation of MAB07 to LP62; DAR represents the loaded drug : antibody ratio; IgG1 represents: a human IgG1, κ isotype control.

FIG. 3 is a graph showing the cell killing activity of the anti-EGFR antibody-drug conjugate MAB07-LP62 (DAR8). (A) Cell killing activity of MAB07-LP62 (DAR8) on an NCI-H1993 cell line. (B) Cell killing activity of MAB07-LP62 (DAR8) on an EBC-1 cell line. Note: MAB07 represents an anti-EGFR antibody, the amino acid sequence of which is set forth in the Sequence Listing; MAB07-LP62 represents an antibody-drug conjugate formed by conjugation of MAB07 to LP62; DAR represents the loaded drug : antibody ratio.

FIG. 4 is a graph showing the cross-binding activity of the anti-EGFR antibody-drug conjugate to EGFRs derived from different species. (A) MAB07-LP62 (DAR8) binding to human EGFR ECD. (B) MAB07-LP62 (DAR8) binding to cynomolgus EGFR ECD. (C) MAB07-LP62 (DAR8) binding to rat EGFR ECD. (D) MAB07-LP62 (DAR8) binding to mouse EGFR ECD. Note: MAB07 represents an anti-EGFR antibody, the amino acid sequence of which is set forth in the Sequence Listing; MAB07-LP62 represents an antibody-drug conjugate formed by conjugation of MAB07 to LP62; DAR represents the loaded drug : antibody ratio; IgG1 represents: a human IgG1, κ isotype control.

FIG. 5 is a graph showing the anti-tumor activity of the anti-EGFR antibody-drug conjugate in a BALB/c nude mouse model subcutaneously xenografted with a human epidermal cancer cell line A431. Note: MAB07 represents an anti-EGFR antibody, the amino acid sequence of which is set forth in the Sequence Listing; MAB07-LP62 represents an antibody-drug conjugate formed by conjugation of MAB07 to LP62; Cetuximab-LP62 represents an antibody-drug conjugate formed by conjugation of Cetuximab to LP62; IgG1-LP62 represents an antibody-drug conjugate formed by conjugation of IgG1 to LP62; and DAR represents the loaded drug : antibody ratio.

FIG. 6 is a graph showing the anti-tumor activity of the anti-EGFR antibody-drug conjugate in a BALB/c nude mouse model subcutaneously xenografted with a human non-small cell lung cancer cell line NCI-H1993. Note: MAB07 represents an anti-EGFR antibody, the amino acid sequence of which is set forth in the Sequence Listing; MAB07-LP62 represents an antibody-drug conjugate formed by conjugation of MAB07 to LP62; MAB07-VC-MMAE represents an antibody-drug conjugate formed by conjugation of MAB07 to linker-toxin MC-VC-PABC-MMAE; MAB07-GGFG-DXD represents an antibody-drug conjugate formed by conjugation of MAB07 to linker-toxin GGFG-DXD; A 1.3 represents toxin compound A 1.3 of Example 1, which is a free loaded drug; DAR represents the loaded drug : antibody ratio.

FIG. 7 is a graph showing the anti-tumor activity of the anti-EGFR antibody-drug conjugate in a BALB/c nude mouse model subcutaneously xenografted with a human non-small cell lung cancer cell line EBC-1. Note: MAB07 represents an anti-EGFR antibody, the amino acid sequence of which is set forth in the Sequence Listing; MAB07-LP62 represents an antibody-drug conjugate formed by conjugation of MAB07 to LP62; MAB07-VC-MMAE represents an antibody-drug conjugate formed by conjugation of MAB07 to linker-toxin MC-VC-PABC-MMAE; MAB07-GGFG-DXD represents an antibody-drug conjugate formed by conjugation of MAB07 to linker-toxin GGFG-DXD; A 1.3 represents toxin compound A 1.3 of Example 1, which is a free loaded drug; DAR represents the loaded drug : antibody ratio.

FIG. 8 is a graph showing the anti-tumor effect of the anti-EGFR antibody-drug conjugate in a male BALB/c nude mouse model subcutaneously xenografted with HuPrime® lung cancer LU3075. Note: MAB07 represents an anti-EGFR antibody, the amino acid sequence of which is set forth in the Sequence Listing; MAB07-LP62 represents an antibody-drug conjugate formed by conjugation of MAB07 to LP62; and DAR represents the loaded drug : antibody ratio.

FIG. 9 is a graph showing the anti-tumor effect of the anti-EGFR antibody-drug conjugate in a human lung cancer LD1-0025-200717 PDX xenograft NCG mouse model. Note: MAB07 represents an anti-EGFR antibody, the amino acid sequence of which is set forth in the Sequence Listing; MAB07-LP62 represents an antibody-drug conjugate formed by conjugation of MAB07 to LP62; serplulimab is an anti-PD-1 antibody, the amino acid sequence of which is set forth in the Sequence Listing; IgG1 represents a human IgG1, κ isotype control; IgG1-LP62 represents an antibody-drug conjugate formed by conjugation of IgG1 to LP62, with a DAR value of 8; and DAR represents the loaded drug : antibody ratio.

FIG. 10 is a graph showing the anti-tumor activity of the anti-EGFR antibody-drug conjugate in an NCG mouse model having humanized PBMC immune system in mice and subcutaneously allografted with human colon cancer HT-29 cells. Note: MAB07 represents an anti-EGFR antibody, the amino acid sequence of which is set forth in the Sequence Listing; MAB07-LP62 represents an antibody-drug conjugate formed by conjugation of MAB07 to LP62; serplulimab is an anti-PD-1 antibody, the amino acid sequence of which is set forth in the Sequence Listing; and DAR represents the loaded drug : antibody ratio.

FIG. 11 is a graph showing the anti-tumor effect of the anti-EGFR antibody-drug conjugate in a human head and neck squamous carcinoma LD1-2023-411020 PDX xenograft NCG mouse model. Note: MAB07-LP62 represents an antibody-drug conjugate formed by conjugation of MAB07 to LP62; DAR represents the loaded drug : antibody ratio.

FIG. 12 is a graph showing the anti-tumor effect of the anti-EGFR antibody-drug conjugate in a human gastric cancer LD1-0017-411335PDX xenograft NU/NU mouse model. Note: MAB07 represents an anti-EGFR antibody, the amino acid sequence of which is set forth in the Sequence Listing; MAB07-LP62 represents an antibody-drug conjugate formed by conjugation of MAB07 to LP62; A 1.3 represents toxin compound A 1.3 of Example 1, which is a free loaded drug; and DAR represents the loaded drug : antibody ratio.

FIG. 13 is a graph showing the anti-tumor effect of the anti-EGFR antibody-drug conjugate in an NOD/SCID mouse model subcutaneously xenografted with a human esophageal cancer KYSE-150 cell line. Note: IgG1 represents a human IgG1, κ isotype control; MAB07 represents an anti-EGFR antibody, the amino acid sequence of which is set forth in the Sequence Listing; MAB07-LP62 represents an antibody-drug conjugate formed by conjugation of MAB07 to LP62; and DAR represents the loaded drug : antibody ratio.

## Detailed Description of the Invention

The present invention provides a novel anti-EGFR antibody-drug conjugate. The anti-EGFR antibody-drug conjugate exhibits good affinity for EGFR-positive cells, can be effectively internalized into the EGFR-positive cells, and

even exhibits a good cell killing effect on tumor cells that are insensitive to anti-EGFR antibodies or EGFR small molecule inhibitors. Furthermore, the anti-EGFR antibody-drug conjugate exhibits excellent tumor inhibitory activity in a variety of tumor models, including osimertinib- and/or Enhertu-resistant tumor models.

For the sake of clarity and not as a limitation, the detailed description of the present invention is divided into the following subsections:

1. Definitions;
2. Antibody-drug conjugate;
3. Method of use;
4. Pharmaceutical formulation; and
5. Article of manufacture and kit.

### 1. Definitions

In the present invention, the scientific and technical terms used herein have the meanings as commonly understood by those skilled in the art unless otherwise specified. In addition, the terms and laboratory operation steps related to the protein and nucleic acid chemistry, molecular biology, cell and tissue culture, microbiology and immunology used herein are all terms and conventional steps that are widely used in the corresponding art. Moreover, for better understanding of the present invention, definitions and explanations of related terms are provided below.

The term "EGFR" means that human epidermal growth factor receptor (also referred to as HER-1 or Erb-B1) is a 170 kDa transmembrane receptor encoded by the c-erbB proto-oncogene and exhibits intrinsic tyrosine kinase activity (Modjtahedi et al., Br. J. Cancer 73: 228-235 (1996); Herbst and Shin, Cancer 94: 1593-1611 (2002)). SwissProt database entry P00533 provides a human EGFR sequence. EGFR regulates a variety of cellular processes through tyrosine kinase-mediated signal transduction pathways, including but not limited to activation of signal transduction pathways that control cell proliferation, differentiation, cell survival, apoptosis, angiogenesis, mitogenesis, and metastasis (Atalay et al., Ann. Oncology 14: 1346-1363 (2003); Tsao and Herbst, Signal 4: 4-9 (2003); Herbst and Shin, Cancer 94: 1593-1611 (2002); Modjtahedi et al., Br. J. Cancer 73: 228-235 (1996)).

The term "antibody" is used in its broadest sense and encompasses various antibody structures, including but not limited to monoclonal antibodies, polyclonal antibodies, multispecific antibodies (e.g., bispecific antibodies), full-length antibodies, and antigen-binding fragments thereof, as long as they exhibit the desired antigen-binding activity. The term "antibody moiety" refers to a full-length antibody or an antigen-binding fragment thereof.

The full-length antibody comprises two heavy chains and two light chains. The variable regions of the heavy chains and light chains are responsible for antigen binding. The variable domains of a heavy chain and a light chain may be referred to as "$V_H$" and "$V_L$", respectively. The variable regions in the two chains generally contain three highly variable loops termed complementarity determining regions (CDRs) (light chain (LC) CDRs including LC-CDR1, LC-CDR2, and LC-CDR3, and heavy chain (HC) CDRs including HC-CDR1, HC-CDR2, and HC-CDR3). The CDR boundaries of the antibodies and antigen-binding fragments disclosed herein can be defined or identified by the following conventions: Kabat, Chothia, or Al-Lazikani (Al-Lazikani 1997; Chothia 1985; Chothia 1987; Chothia 1989; Kabat 1987; and Kabat 1991). The three CDRs of the heavy chain or light chain are interposed between flanking segments termed framework regions (FRs), which are more highly conserved than the CDRs and form a scaffold that supports the hypervariable loops. The constant regions of the heavy chain and light chain are not involved in antigen binding but exhibit various effector functions. Antibodies are assigned to classes based on the amino acid sequences of the constant regions of the heavy chains of the antibodies. The five major classes or isotypes of antibodies are IgA, IgD, IgE, IgG, and IgM, which are characterized by the presence of $\alpha$, $\delta$, $\varepsilon$, $\gamma$, and $\mu$ heavy chains, respectively. Several of the major antibody classes are divided into subclasses such as IgG1 ($\gamma$1 heavy chain), IgG2 ($\gamma$2 heavy chain), IgG3 ($\gamma$3 heavy chain), IgG4 ($\gamma$4 heavy chain), IgA1 ($\alpha$1 heavy chain), or IgA2 ($\alpha$2 heavy chain).

The term "antigen-binding fragment" as used herein refers to an antibody fragment, including, for example, bispecific antibodies, Fab, Fab', F(ab')2, an Fv fragment, a disulfide-stabilized Fv fragment (dsFv), (dsFv)2, bispecific dsFv (dsFv-dsFv'), a disulfide-stabilized bispecific antibody (ds bispecific antibody), single-chain Fv (scFv), an scFv dimer (bivalent diabody), a multispecific antibody consisting of an antibody moiety comprising one or more CDRs, a camelized single domain antibody, a nanobody, a domain antibody, a bivalent domain antibody, or any other antibody fragment that binds to an antigen but does not contain a complete antibody structure. An antigen-binding fragment can bind to the same antigen that the parent antibody or parent antibody fragment (e.g., parent scFv) binds to. In some embodiments, an antigen-binding fragment may comprise one or more CDRs from a particular human antibody, wherein the one or more CDRs are grafted to framework regions from one or more different human antibodies.

As used herein, the term "CDR" or "complementarity determining region" is intended to mean non-contiguous antigen binding sites found within the variable region of a heavy chain and/or light chain polypeptide. These specific regions have been described in: Kabat et al., J. Biol. Chem., 252:6609-6616 (1977); Kabat et al., the U.S. Department of

Health and Human Services, "Sequences of proteins of immunological interest" (1991); Chothia et al., J. Mol. Biol. 196:901-917 (1987); Al-Lazikani B. et al., J. Mol. Biol., 273: 927-948 (1997); MacCallum et al., J. Mol. Biol. 262:732-745 (1996); Abhinandan and Martin, Mol. Immunol., 45: 3832-3839 (2008); Lefranc M.P. et al., Dev. Comp. Immunol., 27: 55-77 (2003); and Honegger and Plückthun, J. Mol. Biol., 309:657-670 (2001), in which the definition includes overlaps or subsets of amino acid residues upon mutual alignment. Nevertheless, any one of the definitions is used to mean that a CDR of an antibody or a grafted antibody or a variant thereof is intended to fall within the scope of the term as defined and used herein. Amino acid residues encompassing the CDRs as defined by each of the above references are set forth below in Table 1 for comparison. CDR prediction algorithms and interfaces are known in the art, including, for example, Abhinandan and Martin, Mol. Immunol., 45: 3832-3839 (2008); Ehrenmann F. et al., Nucleic Acids Res., 38: D301-D307 (2010); and Adolf-Bryfogle J. et al., Nucleic Acids Res., 43: D432-D438 (2015). The contents of the references cited in this paragraph are incorporated herein by reference in their entireties for use in the present application and for possible inclusion in one or more claims herein.

**Table 1: CDR definitions**

| | **Kabat[1]** | **Chothia[2]** | **MacCallum[3]** | **IMGT[4]** | **AHo[5]** |
|---|---|---|---|---|---|
| $V_H$ CDR1 | 31-35 | 26-32 | 30-35 | 27-38 | 25-40 |
| $V_H$ CDR2 | 50-65 | 53-55 | 47-58 | 56-65 | 58-77 |
| $V_H$ CDR3 | 95-102 | 96-101 | 93-101 | 105-117 | 109-137 |
| $V_L$ CDR1 | 24-34 | 26-32 | 30-36 | 27-38 | 25-40 |
| $V_L$ CDR2 | 50-56 | 50-52 | 46-55 | 56-65 | 58-77 |
| $V_L$ CDR3 | 89-97 | 91-96 | 89-96 | 105-117 | 109-137 |

[1]Residue numbering follows the nomenclature of Kabat et al., supra.
[2]Residue numbering follows the nomenclature of Chothia et al., supra.
[3]Residue numbering follows the nomenclature of MacCallum et al., supra.
[4]Residue numbering follows the nomenclature of Lefranc et al., supra.
[5]Residue numbering follows the nomenclature of Honegger and Plückthun, supra.

The expression "variable domain residue numbering as in Kabat" or "amino acid position numbering as in Kabat", and variations thereof, refer to a numbering system used for heavy chain variable domains or light chain variable domains from antibody compilation by Kabat et al., supra. Using this numbering system, the actual linear amino acid sequence may contain shortened or inserted fewer or additional amino acids corresponding to an FR or hypervariable region (HVR) of a variable domain. For example, a heavy chain variable domain may comprise a single amino acid insertion (residue 52a according to Kabat) after residue 52 of H2 and inserted residues (e.g., residues 82a, 82b, and 82c according to Kabat) after heavy chain FR residue 82. The Kabat numbering of residues in a given antibody can be determined by aligning the sequence of the antibody with a "standard" Kabat-numbered sequence across regions of homology.

Unless otherwise specified herein, amino acid residues encompassing the CDRs of a full-length antibody are defined according to the Kabat nomenclature of Kabat et al., supra, and the numbering of residues in an immunoglobulin heavy chain, e.g., an Fc region, refers to numbering by the EU index as described in Kabat et al., supra, except that the amino acid residues of CDRs encompassing any consensus sequence are defined according to the Kabat nomenclature, wherein modifications are based on experimental conditions. The "EU index as described in Kabat" refers to the residue numbering of a human IgG1 EU antibody.

"Framework" or "FR" residues are those variable domain residues other than the CDR residues as defined herein.

A "humanized" form of a non-human (e.g., rodent) antibody is a chimeric antibody that contains a minimal sequence derived from a non-human antibody. In most cases, a humanized antibody is a human immunoglobulin (receptor antibody) in which residues from the hypervariable region (HVR) of a recipient are replaced by residues from a hypervariable region (donor antibody) with the desired antigen specificity, affinity, and capacity in a non-human species (e.g., a mouse, a rat, a rabbit, or a non-human primate). In some examples, framework region (FR) residues of a human immunoglobulin are replaced by corresponding non-human residues. In addition, a humanized antibody can comprise residues not found in the recipient antibody or the donor antibody. These modifications are made to further improve the performance of the antibody. In general, a humanized antibody will comprise at least one, and typically substantially both, of the two variable domains, wherein all or substantially all of the hypervariable loops correspond to those of a non-human immunoglobulin, and all or substantially all of the FRs are those of the human immunoglobulin sequence. A humanized antibody will also optionally comprise at least a portion of an immunoglobulin constant region (Fc), typically at least a

portion of a human immunoglobulin. For further details, see Jones et al., Nature, 321: 522-525, (1986); Riechmann et al., Nature, 332:323-329 (1988); and Presta, Curr. Op. Struct. Biol. 2:593-596 (1992).

A "human antibody" is an antibody that has an amino acid sequence corresponding to that of an antibody produced in a human and/or has been made using any technique as disclosed herein for making human antibodies. This definition of a human antibody specifically excludes a humanized antibody comprising non-human antigen-binding residues. Human antibodies can be produced using various techniques known in the art, including phage display libraries. Hoogenboom and Winter, J. Mol. Biol., 227:381 (1991); and Marks et al., J. Mol. Biol., 222:581 (1991). Also useful for the preparation of human monoclonal antibodies are methods described in Cole et al., Monoclonal Antibodies and Cancer Therapy, Alan R. Liss, p. 77 (1985); and Boerner et al., J. Immunol., 147(1):86-95 (1991). See also van Dijk and van de Winkel, Curr. Opin. Pharmacol., 5: 368-74 (2001). Human antibodies can be prepared by administering an antigen to a transgenic animal that has been modified to produce such antibodies in response to antigenic challenge, but whose endogenous loci have been disabled, e.g., immunized xenomice (see, e.g., U.S. Patent Nos. 6,075,181 and 6,150,584 for XENOMOUSE™ technology). See also, for example, Li et al., Proc. Natl. Acad. Sci. USA 103:3557-3562 (2006) for human antibodies generated via human B cell hybridoma technology.

"Percent (%) amino acid sequence identity" or "homology" with respect to the polypeptide and antibody sequences identified herein is defined as the percentage of amino acid residues in a candidate sequence that are identical to the amino acid residues in the polypeptide being compared, after sequence alignment (considering any conservative substitutions as part of the sequence identity). Alignment for purposes of determining percent amino acid sequence identity can be achieved by using various methods within techniques in the art, for instance, by using publicly available computer software such as BLAST, BLAST-2, ALIGN, Megalign (DNASTAR), or MUSCLE software. Those skilled in the art can determine appropriate parameters for measuring alignment, including any algorithms needed to achieve maximal alignment over the full-length of the sequences being compared. However, for purposes herein, % amino acid sequence identity values are generated using the sequence alignment computer program MUSCLE (Edgar, R.C., Nucleic Acids Research 32(5):1792-1797, 2004; and Edgar, R.C., BMC Bioinformatics 5(1):113, 2004).

"Homologous" refers to the sequence similarity or sequence identity between two polypeptides or between two nucleic acid molecules. When a position in both of two sequences being compared is occupied by the same base or amino acid monomer subunit, e.g., if a position in each of two DNA molecules is occupied by adenine, then the molecules are homologous at that position. The percent of homology between two sequences is a function of the number of matching or homologous positions shared by the two sequences divided by the number of the positions being compared times 100. For example, if 6 of 10 positions in two sequences are matched or homologous, the two sequences are 60% homologous. By way of example, the DNA sequences ATTGCC and TATGGC share 50% homology. Generally, comparison is made when two sequences are aligned to give maximum homology.

The term "constant domain" refers to a portion of an immunoglobulin molecule that has a more conserved amino acid sequence relative to the other portion of the immunoglobulin, i.e., a variable domain, which contains an antigen-binding site. A constant domain contains $C_H1$, $C_H2$, and $C_H3$ domains (collectively referred to as $C_H$) of a heavy chain and a CHL (or $C_L$) domain of a light chain.

A "light chain" of an antibody (immunoglobulin) from any mammalian species can be assigned to one of two clearly distinct types, called kappa ("κ") and lambda ("λ"), respectively, based on the amino acid sequence of the constant domain thereof.

The "CH1 domain" (also referred to as "C1" of "H1" domain) is usually from about amino acid 118 to about amino acid 215 (EU numbering system).

A "hinge region" is generally defined as a region in IgG that corresponds to Glu216 to Pro230 of human IgG1 (Burton, Molec. Immunol., 22:161-206 (1985)). Hinge regions of other IgG isotypes may be aligned with an IgG1 sequence by placing the first and last cysteine residues forming inter-heavy-chain S-S bonds in the same positions.

The "CH2 domain" of a human IgG Fc region (also referred to as "C2" domain) is usually from about amino acid 231 to about amino acid 340. The CH2 domain is unique in that it is not closely paired with another domain. Instead, two N-linked branched carbohydrate chains are interposed between the two CH2 domains of an intact native IgG molecule. It has been speculated that the carbohydrate may provide a substitute for domain-domain pairing and help stabilize the CH2 domain. Burton, Molec Immunol., 22:161-206 (1985).

The "CH3 domain" (also referred to as "C2" domain) comprises a residue region in the Fc region proximal to the C-terminus of the CH2 domain (i.e., from about amino acid residue 341 to the C-terminus of the antibody sequence (typically at residue 446 or 447 in IgG)).

The term "Fc region" or "fragment crystallizable region" herein is used to define a C-terminal region of an immunoglobulin heavy chain, including native sequence Fc regions and variant Fc regions. Although the boundaries of the Fc region of an immunoglobulin heavy chain might vary, the human IgG heavy chain Fc region is usually defined to extend from an amino acid residue at position Cys226, or from Pro230, to the carboxyl-terminus thereof. The C-terminal lysine (residue 447 according to the EU numbering system) of the Fc region may be removed, for example, during production or purification of the antibody, or by recombinantly engineering the nucleic acid encoding a heavy chain of the antibody.

Accordingly, a composition of intact antibodies may include antibody populations from which all K447 residues have been removed, antibody populations from which no K447 residues have been removed, and antibody populations having a mixture of antibodies with and without the K447 residue. Suitable native sequence Fc regions for use in the antibodies described herein include human IgG1, IgG2 (IgG2A, IgG2B), IgG3, and IgG4.

"Fc receptor" or "FcR" describes a receptor that binds the Fc region of an antibody. A preferred FcR is a native human FcR. Moreover, a preferred FcR is one which binds an IgG antibody (γ receptor) and includes receptors of the FcγRI, FcγRII, and FcyRIII subclasses, including allelic variants and alternatively spliced forms of these receptors, FcγRII receptors include FcγRIIA ("activating receptor") and FcγRIIB ("inhibitory receptor"), which have similar amino acid sequences that differ primarily in the cytoplasmic domains thereof. Activating receptor FcyRIIA contains an immunoreceptor tyrosine-based activation motif (ITAM) in its cytoplasmic domain. Inhibitory receptor FcγRIIB contains an immunoreceptor tyrosine-based inhibition motif (ITIM) in its cytoplasmic domain. (See M. Daëron, Annu. Rev. Immunol. 15:203-234 (1997)). FcR is reviewed in Ravetch and Kinet, Annu. Rev. Immunol. 9: 457-92 (1991); Capel et al., Immunomethods 4: 25-34 (1994); and de Haas et al., J. Lab.Clin. Med. 126: 330-41 (1995). The term "FcR" herein encompasses other FcRs, including those to be identified in the future.

As used herein, the terms "specifically binds", "specifically recognizing", and "is specific for" refer to measurable and reproducible interactions, such as binding between a target and an antibody or antibody moiety, which is determinative of the presence of the target in the presence of a heterogeneous population of molecules, including biological molecules. For example, an antibody or antibody moiety that specifically recognizes a target (which can be an epitope) is an antibody or antibody moiety that binds this target with greater affinity, greater avidity, greater readiness, and/or greater duration than its binding to other targets. In some embodiments, the extent of binding of an antibody to an unrelated target is less than about 10% of the extent of binding of the antibody to the target as measured, e.g., by radioimmunoassay (RIA). In some embodiments, the dissociation constant of an antibody that specifically binds to a target is ($K_D$) $\leq 10^{-5}$ M, $\leq 10^{-6}$ M, $\leq 10^{-7}$ M, $\leq 10^{8}$M, $\leq 10^{-9}$ M, $\leq 10^{-10}$ M, $\leq 10^{-11}$ M, or $\leq 10^{-12}$ M. In some embodiments, an antibody specifically binds to an epitope of a protein that is conserved among proteins of various species. In some embodiments, specific binding can include, but does not require, exclusive binding. The binding specificity of an antibody or antigen-binding domain can be determined experimentally by a method known in the art. Such methods include, but are not limited to, Western blots, ELISA-, RIA-, ECL-, IRMA-, EIA-, BIACORE™-assays and peptide scans.

As used herein, the term "cytotoxic agent" refers to a substance that inhibits or prevents cell functions and/or causes cell destruction. The term is intended to include: radioisotopes, e.g., the radioisotopes $At^{211}$, $I^{131}$, $I^{125}$, $Y^{90}$, $Re^{186}$, $Re^{188}$, $Sm^{153}$ , $Bi^{212}$, $P^{32}$, $C^{60}$, and Lu; chemotherapeutic agents; and toxins, such as small molecule toxins or enzymatic toxins of bacterial, fungal, plant, or animal origin, including synthetic analogs and derivatives thereof.

A "linker", "linker unit", or "linker element" refers to a chemical module comprising a covalent bond or chain of atoms that covalently links an antibody or targeting moiety to a drug module.

As used herein, the terms "antibody-drug conjugate", "antibody conjugate", "conjugate", "immunoconjugate", and "ADC" are used interchangeably and refer to a compound or derivative thereof linked to an antibody, e.g., an anti-EGFR antibody, as defined by the following general formula: Ab-(L-D)k, in which Ab = antibody moiety (i.e., antibody or antigen-binding fragment), L = linker moiety, D = drug moiety, and k = the number of drug moieties conjugated per antibody moiety.

Among currently commercially available antibody-drug conjugates, there are two main methods for conjugating an antibody to a linker: (1) Lysine is the most common site of attachment in antibodies, and an ε-amino group thereof can react with an activated carboxyl group in the linker to form an amide bond. Currently, there have been techniques that achieve site-directed conjugation by activating a carboxyl group in the linker with an activating group and then forming an amide bond with the ε-amino group of a specific lysine in the antibody. (2) The sulfhydryl groups (SH) of cysteines in an antibody all exist in the form of disulfide bonds. Opening the disulfide bond in the antibody can provide a plurality of free sulfhydryl groups as sites of conjugation. Conjugation to the sulfhydryl groups of the antibody is done either by a Michael addition reaction between a free sulfhydryl group on the antibody and maleimide, or by double Michael addition reactions between a specific substrate and the free sulfhydryl group on the antibody to form a structurally unique sulfur bridge bond.

The three-letter and one-letter codes of amino acids as used herein are as described in J. boil. Chem. 1968, 243, 3558.

As used herein, the term "halogen" refers to fluorine, chlorine, bromine, or iodine.

As used herein, the term "alkyl" refers to a saturated aliphatic hydrocarbon group, which is a linear or branched group containing 1 to 20 carbon atoms, preferably alkyl containing 1 to 12 carbon atoms, more preferably alkyl containing 1 to 10 carbon atoms, and most preferably alkyl containing 1 to 6 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof, etc. More preferably, the alkyl is a lower alkyl group containing 1 to 6 carbon atoms, and non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. The alkyl group may be substituted or unsubstituted. When substituted, the substituent may be substituted at any available point of attachment, and the substituent is preferably one or more groups independently selected from alkyl, alkenyl, alkynyl, alkoxy, alkylthio, alkylamino, halogen, sulfhydryl, hydroxy, nitro, amino, cycloalkyl, heterocycloalkyl, aryl, heteroaryl, cycloalkoxy, heterocycloalkoxy, cycloalkylthio, heterocycloalkylthio, and oxo.

As used herein, the term "haloalkyl" refers to alkyl substituted with one or more halogen, wherein the alkyl is as defined above.

As used herein, the term "cyano" refers to -CN.

As used herein, the term "sulfonic acid group" refers to the group $-SO_3H$.

As used herein, the term "carboxy" refers to -C(O)OH.

As used herein, the term "alkoxy" refers to -O-(alkyl) and -O-(cycloalkyl), in which the alkyl or cycloalkyl is as defined above. Non-limiting examples of alkoxy include: methoxy, ethoxy, propoxy, butoxy, cyclopropoxy, cyclobutoxy, cyclopentoxy, and cyclohexoxy. The alkoxy can be optionally substituted or unsubstituted, and when it is substituted, the substituent is preferably one or more groups independently selected from alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, and heterocycloalkylthio.

The term "alkenyl" refers to an alkyl compound containing at least one carboncarbon double bond in the molecule, wherein the alkyl is as defined above, and the alkenyl has 2 to 10 (e.g., 2, 3, 4, 5, 6, 7, 8, 9, or 10) carbon atoms (i.e., $C_{2-10}$ alkenyl). The alkenyl is preferably an alkenyl group having 2 to 6 carbon atoms (i.e., $C_{2-6}$ alkenyl). The alkenyl may be substituted or unsubstituted. When it is substituted, the substituent is preferably selected from one or more of alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkynyl" refers to an alkyl compound containing at least one carboncarbon triple bond in the molecule, wherein the alkyl is as defined above, and the alkenyl has 2 to 10 (e.g., 2, 3, 4, 5, 6, 7, 8, 9, or 10) carbon atoms (i.e., $C_{2-10}$ alkynyl). The alkynyl is preferably an alkynyl group having 2 to 6 carbon atoms (i.e., $C_{2-6}$ alkynyl). The alkynyl may be substituted or unsubstituted. When it is substituted, the substituent is preferably selected from one or more of alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

As used herein, the term "deuterated alkyl" refers to alkyl substituted with one or more halogen, wherein the alkyl is as defined above.

The present invention further comprises various deuterated forms of formula (I). Each available hydrogen atom attached to a carbon atom can be independently replaced by a deuterium atom. Those skilled in the art can synthesize deuterated forms of formula (I) by reference to relevant literatures. Commercially available deuterated starting materials may be used in preparing deuterated forms of formula (I), or they may be synthesized using conventional techniques employing deuterated reagents, non-limiting examples of which include: deuterated borane, a solution of a trideuterated borane in tetrahydrofuran, deuterated lithium aluminum hydride, deuterated iodoethane, deuterated iodomethane, etc.

As used herein, the term "DAR" refers to the loaded drug : antibody ratio, which represents the average number of cytotoxic drugs loaded per antibody, or may also represent the ratio of the amount of the drug to the amount of the antibody. The drug loading may range from 0 to 12, preferably 1 to 10 cytotoxic drugs (D) conjugated per antibody (Ab). In an embodiment of the present invention, the DAR is denoted as k, which can be an average value of 1, 2, 3, 4, 5, 6, 7, 8, 9, or 10, by way of example, without limitation. The average number of drugs per ADC molecule after a conjugation reaction can be characterized by conventional methods such as UV/visible spectroscopy, mass spectrometry, ELISA assay, and HPLC.

In one embodiment of the present invention, the cytotoxic drug is conjugated to an exposed interchain cysteine sulfhydryl group (-SH) of an antibody and/or a cysteine sulfhydryl group (-SH) resulting from site-directed mutagenesis via a linker unit. Generally, the number of drug molecules that can be conjugated to the antibody in a conjugation reaction will be less than or equal to the theoretically maximum value.

The following non-limiting methods can be used to control the loading of a ligand-cytotoxic drug conjugate and include:

(1) controlling the molar ratio of a linker-loaded drug to a reducing agent to a monoclonal antibody,
(2) controlling the reaction time, pH value, and temperature, and
(3) selecting different reaction reagents and the content thereof.

As used herein, the term "solvated form" or "solvate" means that the ligand (or targeting moiety)-drug conjugate of the present invention forms a pharmaceutically acceptable solvate with one or more solvent molecules, non-limiting examples of which include water, ethanol, acetonitrile, isopropanol, DMSO, ethyl acetate, dimethylacetamide (DMAC), etc.

The phrase "pharmaceutically acceptable salt" as used herein refers to a pharmaceutically acceptable organic or inorganic salt of an antibody-drug conjugate. Exemplary salts include, but are not limited to, sulfate, citrate, acetate, oxalate, chloride, bromide, iodide, nitrate, bisulfate, phosphate, acid phosphate, isonicotinate, lactate, salicylate, acid citrate, tartrate, oleate, tanninate, pantothenate, bitartrate, ascorbate, succinate, maleate, gentisate, fumarate, gluconate, glucuronate, sugar acid salt, formate, benzoate, glutamate, methanesulfonate, ethanesulfonate, benzenesulfonate, p-toluenesulfonate, and pamoate (i.e., 1,1'-methylene-bis-(2-hydroxy-3-naphthoate)). Pharmaceutically acceptable salts may involve the inclusion of another molecule, such as an acetate ion, a succinate ion, or other counterions. The counterions can be any organic or inorganic module that stabilizes the charge of the parent compound. In addition, pharmaceutically acceptable salts may have more than one charged atom in their structure. Where a variety of charged atoms act as a constituent of a pharmaceutically acceptable salt, there may be a variety of counterions. Thus, a pharmaceutically acceptable salt may have one or more charged atoms and/or one or more counterions.

A "pharmaceutically acceptable solvate" refers to a combination of one or more solvent molecules and an ADC or a salt thereof. Examples of solvents that form pharmaceutically acceptable solvates include, but are not limited to, water, isopropanol, ethanol, methanol, DMSO, ethyl acetate, acetic acid, and ethanolamine.

As used herein, "treatment" or "treating" is an approach for obtaining beneficial or desired results, including clinical results. For purposes of the present application, the beneficial or desired clinical results include, but are not limited to, one or more of: alleviating one or more symptoms resulting from the disease, diminishing the extent of the disease, stabilizing the disease (e.g., preventing or delaying the worsening of the disease), preventing or delaying the spread (e.g., metastasis) of the disease, preventing or delaying the recurrence of the disease, delaying or slowing the progression of the disease, ameliorating the disease state, providing a remission (partial or total) of the disease, decreasing the dose of one or more other drugs required to treat the disease, delaying the progression of the disease, increasing or ameliorating the quality of life, increasing body weight gain, and/or prolonging survival. Also encompassed by "treatment" is a reduction of pathological consequence of cancer (such as, for example, tumor volume). The method of the present application contemplates any one or more of these aspects of treatment.

In the context of cancer, the term "treatment" includes any one or all of: inhibiting the growth of cancer cells, inhibiting the replication of cancer cells, reducing the overall tumor burden, and ameliorating one or more symptoms related to the disease.

The term "inhibition" or "inhibit" refers to the reduction or cessation of any phenotypic characteristic, or the reduction or cessation of the incidence, extent or likelihood of that characteristic. "Reducing" or "inhibiting" refers to reducing, lowering, or preventing activity, function and/or amount as compared with a reference. In certain embodiments, "reducing" or "inhibiting" refers to the ability to cause an overall reduction of 20% or more. In another embodiment, "reducing" or "inhibiting" refers to the ability to cause an overall reduction of 50% or more. In yet another embodiment, "reducing" or "inhibiting" refers to the ability to cause an overall reduction of 75%, 85%, 90%, 95%, or more.

As used herein, "reference" refers to any sample, standard, or level used for comparison purposes. The reference can be obtained from healthy and/or nondiseased samples. In certain embodiments, the reference may be obtained from an untreated sample. In certain embodiments, the reference is obtained from a nondiseased or untreated sample of an individual. In some examples, the reference is obtained from one or more healthy individuals other than the individual or patient.

As used herein, "delaying the development of a disease" refers to delaying, impeding, slowing, retarding, stabilizing, inhibiting, and/or delaying the development of a disease, such as cancer. The delay may vary in the length of time, depending on the history of the disease and/or the individual being treated. It would be obvious to those skilled in the art that a sufficient or significant delay may actually include prevention, since the individual does not develop the disease. For example, it may delay advanced cancer (e.g., the development of metastasis).

As used herein, the "prevention" includes providing prevention against the occurrence or recurrence of a disease in an individual who may be predisposed to the disease but has not yet been diagnosed with the disease.

As used herein, an "inhibitory" function or activity is a decrease in function or activity as compared with the same condition other than the condition or parameter of interest, or alternatively as compared with another condition. For example, antibodies that inhibit tumor growth reduce the rate of tumor growth as compared with the rate of tumor growth in the absence of the antibodies.

The terms "subject", "individual", and "patient" are used interchangeably herein to refer to a mammal, including, but not limited to, human, bovine, horse, feline, canine, rodent, or primate. In some embodiments, the individual is a human.

An "effective amount" of an agent refers to an amount effective, at doses and for periods of time necessary, to achieve the desired therapeutic or prophylactic result. The specific dose may vary depending on one or more of: the

selected particular agent, the dosage regimen to be followed, whether to be administered in combination with other compounds, the time of administration, the tissue to be imaged, and the physical delivery system that carries it.

The "therapeutically effective amount" of a substance/molecule, agonist, or antagonist of the present application may vary depending on factors such as the disease state, age, sex, and body weight of the individual, and the ability of the substance/molecule, agonist, or antagonist to elicit the desired response in the individual. A therapeutically effective amount is also an amount in which any toxic or detrimental effects of the substance/molecule, agonist, or antagonist are outweighed by the therapeutically beneficial effects. A therapeutically effective amount may be delivered in one or more administrations.

A "prophylactically effective amount" refers to an amount effective, at doses and for periods of time necessary, to achieve the desired prophylactic result. Typically, but not necessarily, since a prophylactic dose is used in a subject prior to or at an earlier stage of a disease, the prophylactically effective amount will be less than the therapeutically effective amount.

The terms "pharmaceutical formulation" and "pharmaceutical composition" refer to a preparation in a form that allows the biological activity of one or more active ingredients to be effective and does not comprise other components that are unacceptably toxic to the individual to which the formulation is to be administered. Such formulations may be sterile.

A "pharmaceutically acceptable carrier" refers to a non-toxic solid, semi-solid, or liquid filler, diluent, encapsulating material, formulation auxiliary, or carrier conventional in the art for use with a therapeutic agent, both of which together constitute a "pharmaceutical composition" for administration to an individual. The pharmaceutically acceptable carrier is non-toxic to the recipient at the dose and concentration employed and is compatible with the other ingredients of the formulation. A pharmaceutically acceptable carrier is suitable for the formulation employed.

Herein, KRAS gene has the same meaning as K-RAS gene, a member of the RAS gene family, which encodes K-ras protein and is related to the generation, proliferation, migration, diffusion and angiogenesis of various tumors. Common mutation sites therein are codon 12 and codon 13 of exon 2 and codon 61 of exon 3 in the K-RAS gene, wherein there are 7 mutation hotspots: G12C, G12R, G12S, G12V, G12D, G12A, and G13V/D, and these seven mutations account for 90% or more. In one embodiment of the present invention, the tumor is a tumor with KRAS gene mutation related to EGFR expression.

Herein, BRAF (v-raf murine sarcoma viral oncogene homolog B1) gene is a proto-oncogene and is a member of the RAF family. BRAF mutations occur in approximately 8% of human tumors, and the vast majority of BRAF gene mutations are in the form of BRAF V600E mutation, which results in the continuous activation of the downstream MEK/ERK signaling pathway, which is critical for tumor growth proliferation and invasive metastasis. In one embodiment of the present invention, the tumor is a tumor with BRAF gene mutation related to EGFR expression.

It should be understood that where a chemical name is inconsistent with the chemical structure in the present application, if any, the chemical structure prevails.

It should be understood that embodiments of the present application described herein include "consisting" and/or "consisting essentially of' embodiments.

Reference to "about" a value or parameter herein includes (and describes) variations that are directed to that value or parameter per se. For example, description referring to "about X" includes description of "X".

As used herein, reference to "not" being a value or parameter generally means and describes a "different" value or parameter. For example, where a method is not used for treating type X cancer, it means that the method is used for treating cancers other than type X.

As used herein, the term "about X-Y" has the same meaning as "about X to about Y".

As used herein, modifying the amount of an ingredient or reactant in the present invention with the term "about" is a variation in the amount of a numerical value that may occur, for example, in typical measurements and liquid treatment procedures used for preparing concentrates or actually used solutions; in occasional errors in these procedures; in differences in manufacturing, sources, or the purity of ingredients used for preparing the composition or carrying out the method; etc. The term "about" also includes amounts that differ due to different equilibrium conditions relative to a composition resulting from a particular starting mixture. Whether or not modified by the term "about", equivalents of amounts are included in the claims. In one embodiment, the term "about" means being within 10% of the reported numerical value, preferably within 5% of the reported numerical value.

It will be understood by those skilled in the art that when a numerical value is recited in the claims, whether or not it is prefixed with "about", the actual value of the numerical value may vary by 10% ($\pm$ 10%) from the recited value, preferably by 5% ($\pm$ 5%).

As used herein and in the appended claims, the singular forms "a", "an", "or", and "the" include plural referents unless the context clearly dictates otherwise.

## 2. Antibody-drug conjugate

An anti-EGFR antibody can be conjugated to a cytotoxic or cytostatic moiety (including a pharmaceutically

compatible salt thereof) to form an antibody-drug conjugate (ADC). Particularly suitable moieties for conjugation to the antibody are cytotoxic agents (e.g., chemotherapeutic agents), prodrug converting enzymes, radioisotopes or compounds or toxins (these moieties are collectively referred to as therapeutic agents). For example, the anti-EGFR antibody can be conjugated to a cytotoxic agent such as a chemotherapeutic agent or a toxin (e.g., a cytostatic agent or a cell killer, such as, for example, abrin, ricin A, Pseudomonas exotoxin, or diphtheria toxin).

The anti-EGFR antibody can be conjugated to a prodrug converting enzyme. The prodrug converting enzyme can be recombinantly fused with or chemically conjugated to the antibody by using a known method. Exemplary prodrug converting enzymes are carboxypeptidase G2, β-glucuronidase, penicillin-V-amidase, penicillin-G-amidase, β-lactamase, β-glucosidase, nitroreductase, and carboxypeptidase A.

Techniques for conjugating therapeutic agents to proteins, particularly to antibodies, are well known. (See, for example, Arnon et al., "Monoclonal Antibodies For Immunotargeting Of Drugs In Cancer Therapy", in Monoclonal Antibodies And Cancer Therapy (Reisfeld et al. eds., Alan R. Liss, Inc., 1985); Hellstrom et al., "Antibodies For Drug Delivery", in Controlled DrugDelivery (Robinson et al. eds., Marcel Dekker, Inc., 2nd edition, 1987); Thorpe, "Antibody-Carriers Of Cytotoxic Agents In Cancer Therapy: A Review", in Monoclonal Antibodies'84: Biological And Clinical Applications (Pinchera et al. eds., 1985); "Analysis, Results, and Future Prospective of the Therapeutic Use of Radiolabeled Antibody In Cancer Therapy", in Monoclonal Antibodies For CancerDetection And Therapy (Baldwin et al. eds., Academic Press, 1985); and Thorpe et al., 1982, Immunol. Rev. 62:119-58. See also, for example, PCT Publication No. WO 89/12624.)

The therapeutic agent can be conjugated in a manner that reduces the activity thereof unless it is cleaved from the antibody (e.g., by hydrolysis, by antibody degradation, or by a cleaving agent). Such a therapeutic agent is attached to the antibody by using a cleavable linker that is sensitive to cleavage in the intracellular environment or the tumor microenvironment of EGFR-expressing cancer cells such that it is cleaved from the antibody when the conjugate is in that environment (e.g., in endosomes or for example due to pH sensitivity or protease sensitivity in lysosomal environments, in caveolae environments or in tumor microenvironment).

Typically, the ADC comprises a linker region between the therapeutic agent and the anti-EGFR antibody. As described above, typically, the linker is cleavable under intracellular conditions or in the tumor microenvironment such that the cleavage of the linker occurs in the intracellular environment (e.g., in lysosomes or endosomes or caveolae) or in the tumor microenvironment, leading to release of the therapeutic agent from the antibody. The linker can be, for example, a peptidyl linker that is cleaved by intracellular peptidases or proteases, including lysosomal or endosomal proteases. Typically, the peptidyl linker has a length of at least two amino acids or at least three amino acids. Cleaving agents may include cathepsins B and D, plasmin, etc. (see, e.g., Dubowchik and Walker, 1999, Pharm. Therapeutics 83: 67-123). The most typical peptidyl linkers are peptidyl linkers cleavable by enzymes present in EGFR-expressing cells or within the tumor microenvironment. For example, peptidyl linkers cleavable by thiol-dependent protease cathepsin-B, which can be highly expressed in cancer tissues, can be used (e.g., a linker containing a Phe-Leu or Gly-Phe-Leu-Gly peptide). Other such linkers are described, for example, in U.S. Patent No. 6,214,345. In a specific embodiment, a peptidyl linker cleavable by an intracellular protease includes a Val-Cit linker or a Phe-Lys dipeptide (see, e.g., U.S. Patent 6,214,345, which describes the synthesis of doxorubicin using the Val-Cit linker). One advantage of using intracellular proteolysis to release the therapeutic agent is that the agent typically decays during conjugation and the serum stability of the conjugate is generally high.

The cleavable linker can be pH-sensitive, i.e., sensitive to hydrolysis at certain pH values. Typically, pH-sensitive linkers are hydrolyzable under acidic conditions. For example, acid-labile linkers that are hydrolyzable in lysosomes (e.g., hydrazones, hemicarbazones, thiosemicarbazones, cis-aconitic amide, orthoesters, acetals, and ketals) can be used. (See, e.g., U.S. Patent Nos. 5,122,368; 5,824,805; and 5,622,929; Dubowchik and Walker, 1999, Pharm. Therapeutics 83:67-123; and Neville et al., 1989, Biol. Chem. 264:14653-14661). Such linkers are relatively stable under neutral pH conditions, such as those in blood, but are not stable below pH 5.5 or 5.0 (the approximate pH of lysosomes). In certain embodiments, a hydrolyzable linker is a thioether linker (such as a thioether attached to a therapeutic agent via an acylhydrazone bond) (see, e.g., U.S. Patent No. 5,622,929).

Other linkers, such as disulfide linkers, are cleavable under reducing conditions. Disulfide linkers include those that can be formed with SATA (N-succinimidyl-S-acetylthioacetate), SPDP (N-succinimidyl-3-(2-pyridyldithio)propionate), SPDB (N-succinimidyl-3-(2-pyridyldithio)butyrate) and SMPT (N-succinimidyl-oxycarbonyl-α-methyl-α-(2-pyridyldithio)toluene), SPDB and SMPT. See, e.g., Thorpe et al., 1987, Cancer Res. 47:5924-5931; Wawrzynczak et al., Immunoconjugates: Antibody Conjugates in Radioimagery and Therapy of Cancer (C. W. Vogel eds, Oxford U. Press, 1987. See also U.S. Patent No. 4,880,935.)

The linker may also be a malonate linker (Johnson et al., 1995, Anticancer Res. 15: 1387-93), a maleimidobenzoyl linker (Lau et al., 1995, Bioorg-Med-Chem. 3 (10):1299-1304), or 3'-N-amide analogs (Lau et al., 1995, Bioorg-Med-Chem. 3 (10):1305-12). The linker may also be a malonate linker (Johnson et al., 1995, Anticancer Res. 15: 1387-93), a maleimidobenzoyl linker (Lau et al., 1995, Bioorg-Med-Chem. 3 (10):1299-1304), or 3'-N-amide analogs (Lau et al., 1995, Bioorg-Med-Chem. 3 (10):1305-12).

The linker may also be a non-cleavable linker, such as a maleimido-alkylene-or maleimido-aryl linker directly attached to a therapeutic agent (e.g., a drug). The active drug-linker is released by the degradation of the antibody.

Typically, the linker is substantially insensitive to the circulatory system environment, which means that when the ADC is present in plasma, no more than about 20%, typically no more than about 15%, more typically no more than about 10%, even more typically no more than about 5%, no more than about 3%, or no more than about 1% of the linker in an ADC sample is cleaved.

Whether the linker is substantially insensitive to the circulatory system environment can be determined, for example, by: incubating both (a) ADC ("ADC sample") and (b) an equimolar amount of an unconjugated antibody or therapeutic agent ("control sample") independently with plasma for a predetermined period of time (e.g., 2, 4, 8, 16, or 24 hours), and then comparing the amount of the unconjugated antibody or therapeutic agent present in the ADC sample as measured, for example, by high performance liquid chromatography, with the amount present in the control sample.

The anti-EGFR antibody can be conjugated to the linker via a heteroatom in the antibody. Such heteroatoms can be present in their natural state on the antibody, or can be introduced into the antibody. In some aspects, the anti-EGFR antibody will be conjugated to the linker via the nitrogen atom of the lysine residue. In other aspects, the anti-EGFR antibody will be conjugated to the linker via the sulfur atom of the cysteine residue. The cysteine residue can be a residue that is naturally occurring or engineered into the antibody. Methods for conjugating linkers and druglinkers to antibodies via lysine and cysteine residues are known in the art.

Exemplary antibody-drug conjugates also include camptothecin-based antibody-drug conjugates (i.e., the drug component is a camptothecin drug). Camptothecin is a topoisomerase inhibitor that has been proved to have anti-cancer activity. Typically, the camptothecin-based antibody-drug conjugate comprises a linker between the camptothecin drug and the anti-EGFR antibody. The linker may be, for example, a cleavable linker (e.g., a peptidyl linker or a carbohydrate linker) or a non-cleavable linker (e.g., a linker released by the degradation of the antibody). The synthesis and structure of exemplary camptothecin drug linkers are described in PCT/US19/025968, which is incorporated herein for all purposes by reference in its entirety.

Other exemplary antibody-drug conjugates include maytansinoid antibody-drug conjugates (i.e., the drug component is a maytansinoid drug) and benzodiazepine antibody-drug conjugates (i.e., the drug component is a benzodiazepine (e.g., a pyrrolo[l,4]benzodiazepine dimer (PBD dimer), an indolobenzodiazepine dimer, and an oxazolidinebenzodiazepine dimer)).

Useful classes of cytotoxic agents conjugated to anti-EGFR antibodies include, for example, anti-tubulin agents, DNA minor groove binders, DNA replication inhibitors, chemotherapeutic sensitizers, etc. Other exemplary classes of cytotoxic agents include anthracyclines, auristatins, camptothecins, duocarmycin, etoposide, maytansinoids, and vinca alkaloids. Some exemplary cytotoxic agents include camptothecins (e.g., irinotecan and DXD), auristatins (e.g., auristatin T, auristatin E, AFP, monomethylauristatin F (MMAF), lipophilic monomethylauristatin F, monomethylauristatin E (MMAE)), DNA minor groove binders (e.g., enediynes and lexitropsins), duocarmycin, taxanes (e.g., taxol and docetaxel), vinca alkaloids, nicotinamide phosphoribosyltransferase inhibitors (NAMPTi), Tubulysin M, doxorubicin, morpholino-doxorubicin, and cyclomorpholino-doxorubicin.

The cytotoxic agent may be a chemotherapeutic agent such as, for example, doxorubicin, taxol, melphalan, vinca alkaloids, methotrexate, mitomycin C, or etoposide. The agent may also be an analog of CC-1065, calicheamicin, maytansine, a dolastatin 10 analog, rhizoxin, or palytoxin.

The cytotoxic agent may also be auristatin. The auristatin can be an auristatin E derivative, such as an ester formed between auristatin E and a keto acid. For example, auristatin E can react with p-acetylbenzoic acid or benzoylvaleric acid to produce AEB and AEVB, respectively. Other typical auristatins include auristatin T, AFP, MMAF, and MMAE. The synthesis and structure of various auristatins are described, for example, in US 2005-0238649 and US 2006-0074008.

The present invention is a novel EGFR-targeted antibody-drug conjugate (ADC) consisting essentially of three moieties, namely a monoclonal antibody targeting EGFR, a cytotoxic small molecule drug (payload), and a tumor microenvironmentsensitive linker (Linker), wherein the loaded drug is a novel topoisomerase (TOP) I inhibitor that is more tumor-sensitive. After entering the body, the anti-EGFR antibody-drug conjugate of the present invention can enter the cancer cell by specifically binding to highly expressed EGFR on the tumor cells via the antibody moiety thereof, followed by antigen-mediated internalization of the drug. During the capture and transport of the anti-EGFR antibody-drug conjugate of the present invention by lysosomes inside the tumor cells, the linker of the anti-EGFR antibody-drug conjugate is cleaved to efficiently release the payload in a special environment within the tumors, whereupon the loaded small molecule drug exerts a cytotoxic effect and can thus kill the cancer cells. Due to the relatively high lipophilicity, some payloads can exert a "bystander effect", enabling their action after entering cells surrounding cancer cells. This anti-cancer mechanism of action is also effective against low-antigen expression or heterogeneous tumor cells.

In some aspects, the present invention provides an antibody-drug conjugate, a prodrug, pharmaceutically acceptable salt, or solvate thereof, or a solvate of the pharmaceutically acceptable salt, wherein the antibody-drug conjugate has a structure of formula (I):

$$TP\text{-}[L1\text{-}L2\text{-}L3\text{-}D]_k \qquad (I)$$

wherein:

TP is a targeting moiety selectively binding or recognizing EGFR;
L1 is an extension unit connecting TP and L2, wherein TP is connected to L1 via an active group (e.g., sulfhydryl or amino group), preferably wherein L1 is conjugated to an exposed interchain cysteine sulfhydryl group (-SH) in TP and/or a cysteine sulfhydryl group (-SH) resulting from site-directed mutagenesis;
L2 is an optional amino acid residue or a short peptide consisting of 2-10 amino acid residues;
L3 is a spacer element;
D is a biologically active molecule; and
k represents any numerical value between 0.1 and 10.0.

With reference to the antibody-drug conjugate, the subscript k represents the drug loading and, depending on the context, may represent the molecular number of the drug-linker molecules attached to the individual antibody molecule; therefore, it is an integer value; alternatively, k can represent the average drug loading and therefore can be an integer or non-integer value. The average drug loading represents the average number of drug-linker molecules per antibody in a population. Usually, but not always, when referring to antibodies, such as monoclonal antibodies, we refer to a population of antibody molecules. In a composition comprising a population of antibody-drug conjugate molecules, the average drug loading is an important mass attribute, as it determines the amount of the drug that can be delivered to the target cell. The percentage of unconjugated antibody molecules in the composition is included in the average drug loading value.
In a preferred aspect of the present invention, when referring to a composition comprising a population of antibody-drug conjugate compounds, the average drug loading is from 1 to about 16, preferably about 2 to about 14, more preferably from about 2 to about 10.
For MMAE and camptothecin ADCs, such as those exemplified herein, a preferred average drug loading is about 2, 4, or 8, and a particularly preferred average drug loading is about 8. In one embodiment, a preferred average drug loading for an MMAE ADC is 2 or 4. In one embodiment, a preferred average drug loading for a camptothecin ADC is 4 or 8. In an exemplary embodiment, the drug-linker is conjugated to a cysteine residue at a reduced interchain disulfide. In some aspects, the actual drug loading per individual antibody molecule in a population of antibody-drug conjugate compounds is from 1 to 10 (or from 6 to 10 or from 6 to 8), with the predominant drug loading being 8.
In some embodiments of formula (I), the TP is an antibody or an antigen-binding fragment thereof.
In some embodiments of formula (I), the antigen-binding fragment is selected from a full-length immunoglobulin, a single-chain Fv (scFv) fragment, an Fab fragment, an Fab' fragment, $F(ab')_2$, an Fv fragment, a disulfide-stabilized Fv fragment (dsFv), $(dsFv)_2$, an Fv-Fc fusion, an scFv-Fc fusion, an scFv-Fv fusion, a diabody, a tribody, a tetrabody, or any combination thereof.
In some embodiments of formula (I), the TP is a humanized antibody or an antigen-binding fragment thereof.
In some embodiments of formula (I), the TP comprises a heavy chain variable region (VH) and a light chain variable region (VL), wherein the heavy chain variable region (VH) comprises heavy chain complementarity determining region 1 (HCDR1), HCDR2, and HCDR3 contained in SEQ ID NO: 7, and the light chain variable region (VL) comprises light chain complementarity determining region 1 (LCDR1), LCDR2, and LCDR3 contained in SEQ ID NO: 8.
In some embodiments of formula (I), the amino acid sequence of HCDR1 is SEQ ID NO: 1, the amino acid sequence of HCDR2 is SEQ ID NO: 2, and the amino acid sequence of HCDR3 is SEQ ID NO: 3; and the amino acid sequence of LCDR1 is SEQ ID NO: 4, the amino acid sequence of LCDR2 is SEQ ID NO: 5, and the amino acid sequence of LCDR3 is SEQ ID NO: 6.
In some embodiments of formula (I), the TP comprises an Fc sequence of human IgG.
In some embodiments of formula (I), the IgG is selected from IgG1, IgG2, IgG3, and IgG4.
In some embodiments of formula (I), the TP comprises a heavy chain variable region (VH), and the amino acid sequence of the heavy chain variable region (VH) has at least 70%, at least 80%, at least 90%, at least 95%, at least 99%, or 100% identity to SEQ ID NO: 7.
In some embodiments of formula (I), the TP comprises a light chain variable region (VL), and the amino acid sequence of the light chain variable region (VL) has at least 70%, at least 80%, at least 90%, at least 95%, at least 99%, or 100% identity to SEQ ID NO: 8.
In some embodiments of formula (I), the TP comprises a heavy chain (HC), and the amino acid sequence of the heavy chain (HC) has at least 70%, at least 80%, at least 90%, at least 95%, at least 99%, or 100% identity to SEQ ID NO: 9.
In some embodiments of formula (I), the TP comprises a light chain (LC), and the amino acid sequence of the light chain (LC) has at least 70%, at least 80%, at least 90%, at least 95%, at least 99%, or 100% identity to SEQ ID NO: 10.
In some embodiments of formula (I), the TP is a multispecific antibody.

In some embodiments of formula (I), the TP is an afucosylated antibody.

In some embodiments of formula (I), the Fc region comprises a C-terminal lysine.

In some embodiments of formula (I), the Fc region comprises a deletion of the C-terminal lysine.

In some embodiments of formula (I), the L1 is selected from

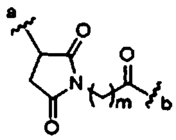 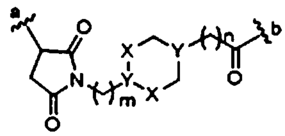 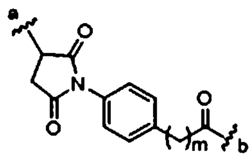

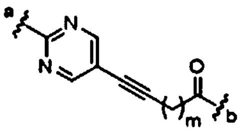 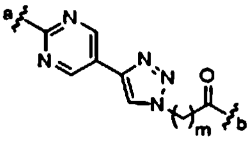 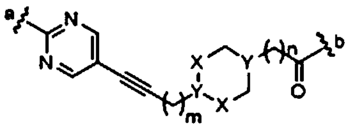

wherein L1 is connected to TP at position a via an S atom and to L2 at position b, m is any integer between 1 and 10, n is any integer between 1 and 10, and X and Y are each independently C, O, S, or N.

In some embodiments of formula (I), the L1 is

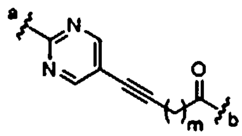

wherein L1 is connected to TP at position a via an S atom and to L2 at position b, and m is selected from 1, 2, 3, 4, or 5.

In some embodiments of formula (I), the L1 is

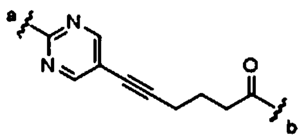

wherein L1 is connected to TP at position a via an S atom and to L2 at position b.

In some embodiments, L1 is connected to a cysteine of TP (more specifically, the sulfhydryl group of the cysteine) at position a.

In some embodiments of formula (I), the L2 is selected from

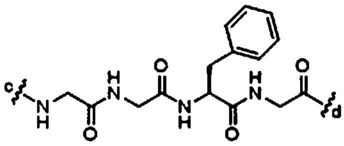 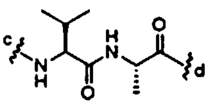 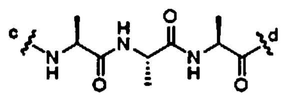

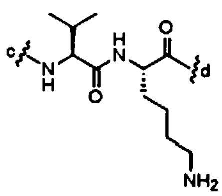 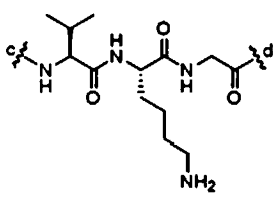 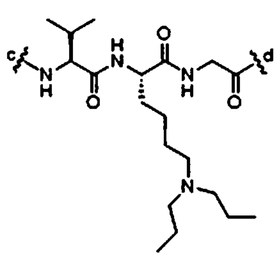

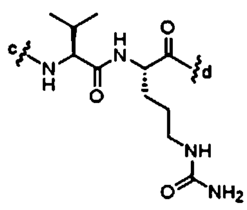
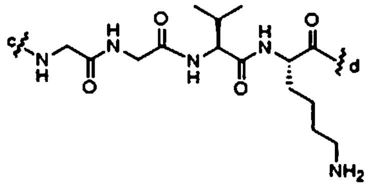
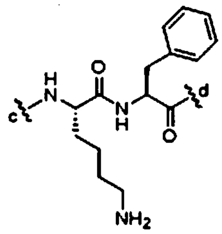

wherein L2 is connected to L1 at position c and to L3 at position d.

In some embodiments of formula (I), the L2 is

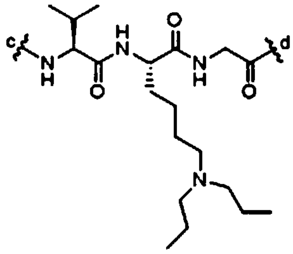

wherein L2 is connected to L1 at position c and to L3 at position d.

In some embodiments of formula (I), the L3 is a single bond or a group selected from:

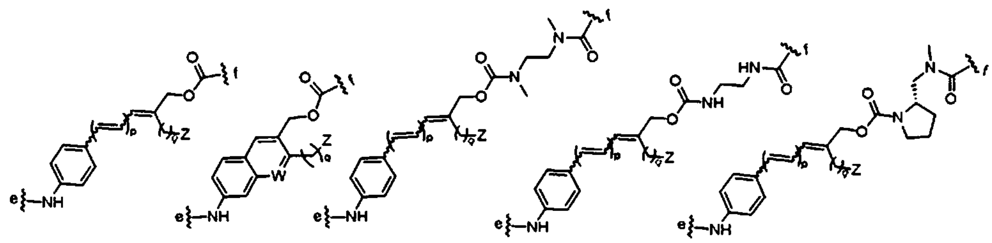

wherein L3 is connected to L2 at position e and to D at position f,
p and q are each independently an integer between 0 and 10,
W is C, O, S, or N, and
Z is selected from hydrogen, halogen, $C_1$-$C_{20}$ alkane, $C_1$-$C_{20}$ haloalkane, cyano, sulfonic acid, carboxylic acid, $C_1$-$C_{20}$ alkoxy, $C_2$-$C_{10}$ unsaturated alkane, a natural amino acid, an unnatural amino acid, and a short peptide consisting of the above amino acids.

In some embodiments of formula (I), Z is selected from

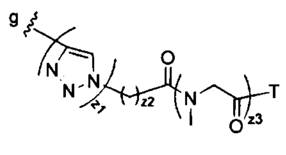
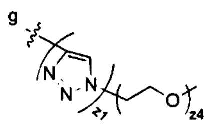
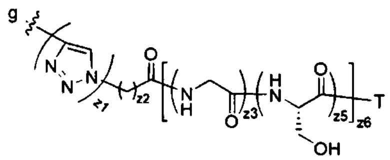

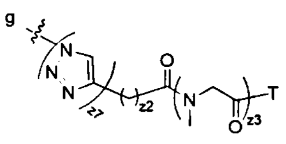
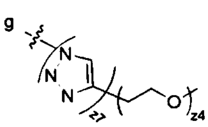
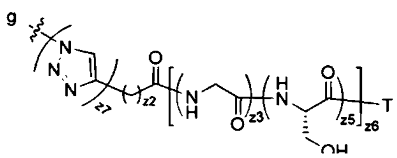

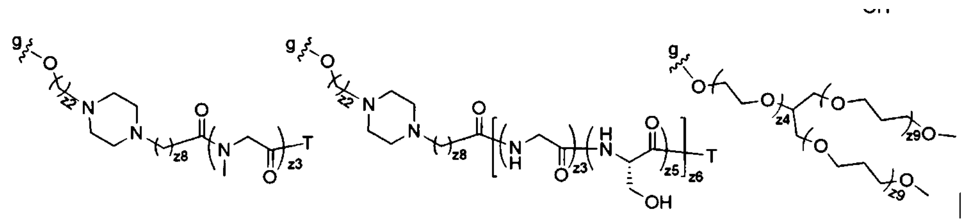

wherein Z is connected to the remainder of L3 at position g, z1-z9 are each independently an integer between 0 and 10, and T is $NH_2$ or OH.

In some embodiments of formula (I), D is a cytotoxic agent.

In some embodiments of formula (I), D is selected from

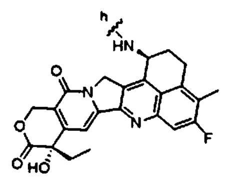
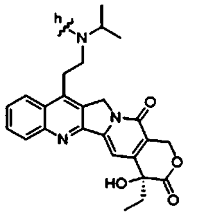
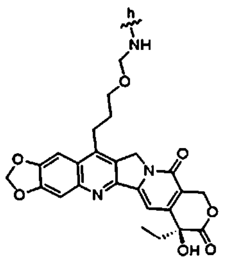
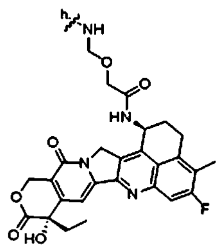

wherein D is connected to L3 at position h.

In some embodiments of formula (I), the antibody-drug conjugate is selected from:

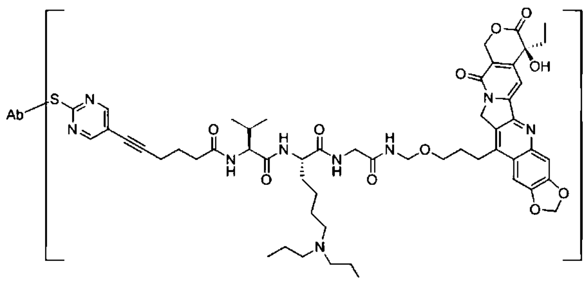
(A-1)

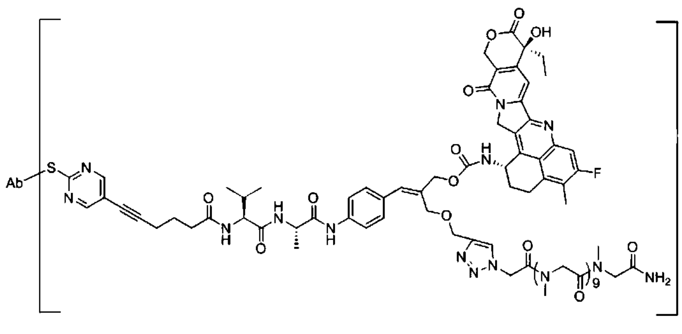
(A-2)

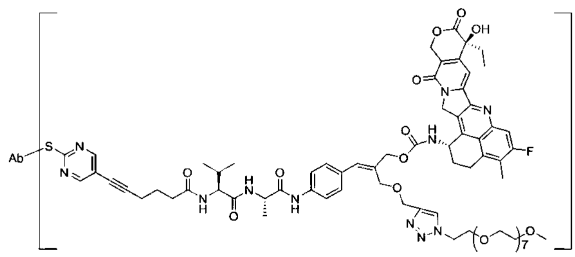
(A-3)

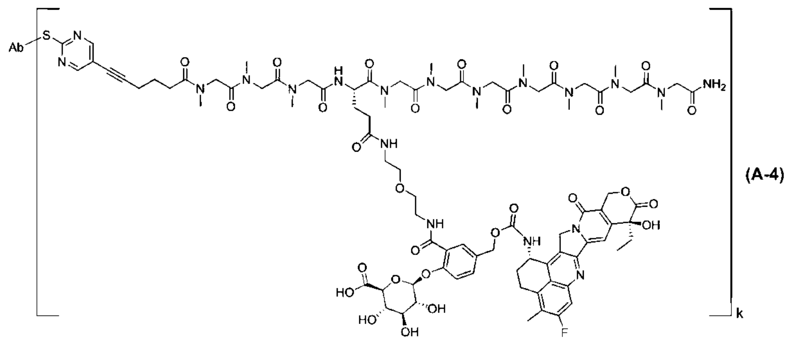

(A-4)

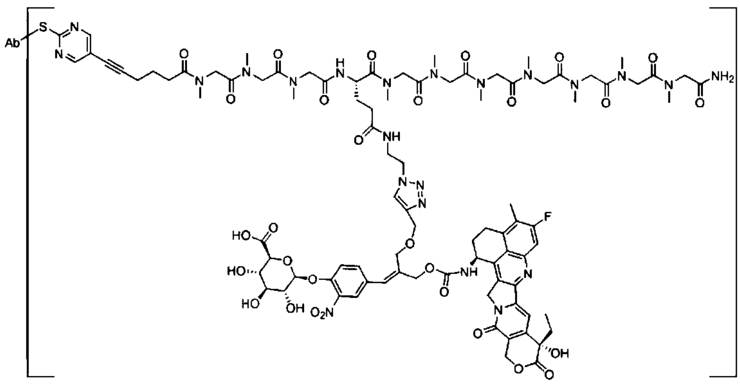

(A-5)

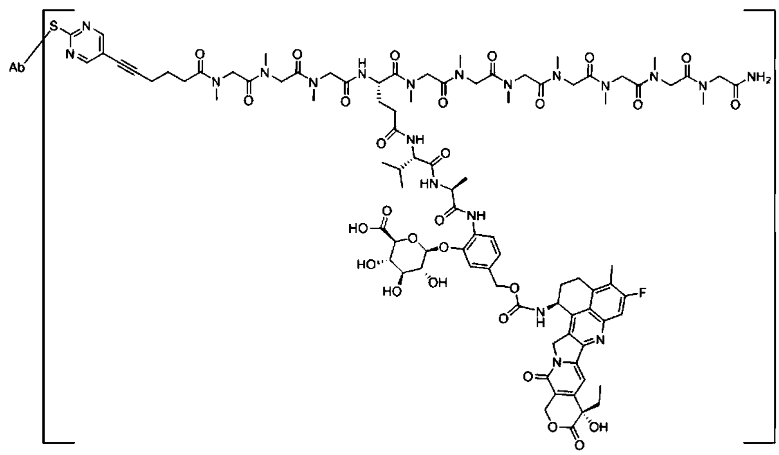

(A-6)

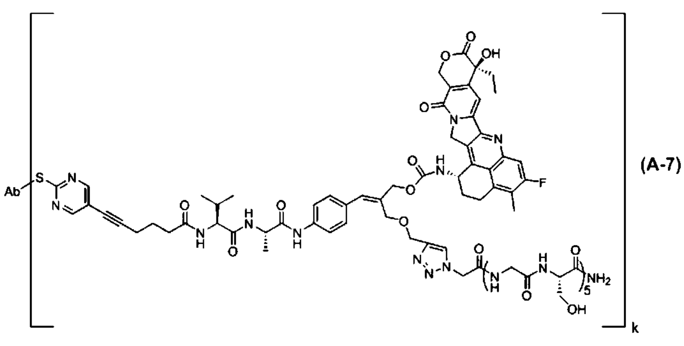

(A-7)

Ab represents the antibody or antigen-binding fragment thereof as defined in the previous embodiments.

In some embodiments of formula (I), k is 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6. 1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, 10.0, or in a

range between any two thereof. In further embodiments, k is 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6. 1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, or in a range between any two thereof. In further embodiments, k is 4.0, 6.0, or 8.0.

## 3. Method of use

The anti-EGFR antibody-drug conjugate of the present invention can be used for treating cancer. Some of such cancers show detectable EGFR levels measured at the protein (e.g., by immunoassay using one of the exemplified antibodies) or mRNA level. Some of such cancers show elevated EGFR levels relative to the same type of non-cancerous tissue, preferably from the same patient. Optionally, the level of EGFR in cancer is measured prior to treatment.

Examples of cancers that are related to EGFR expression and amenable to treatment include epidermal cancer, colon cancer, rectal cancer, head and neck cancer, lung cancer, ovarian cancer, cervical cancer, bladder cancer, esophageal cancer, breast cancer, kidney cancer, prostate cancer, gastric cancer, pancreatic cancer, and glioma. In some embodiments, the antibody or antibody-drug conjugate of the present invention is used for treating epidermal cancer. In some embodiments, the antibody or antibody-drug conjugate of the present invention is used for treating colon cancer. In some embodiments, the antibody or antibody-drug conjugate of the present invention is used for treating rectal cancer. In some embodiments, the antibody or antibody-drug conjugate of the present invention is used for treating head and neck cancer. In some embodiments, the antibody or antibody-drug conjugate of the present invention is used for treating lung cancer. In some embodiments, the antibody or antibody-drug conjugate of the present invention is used for treating osimertinib-resistant lung cancer. In some embodiments, the antibody or antibody-drug conjugate of the present invention is used for treating non-small cell lung cancer. In some embodiments, the antibody or antibody-drug conjugate of the present invention is used for treating small cell lung cancer. In some embodiments, the antibody or antibody-drug conjugate of the present invention is used for treating ovarian cancer. In some embodiments, the antibody or antibody-drug conjugate of the present invention is used for treating cervical cancer. In some embodiments, the antibody or antibody-drug conjugate of the present invention is used for treating bladder cancer. In some embodiments, the antibody or antibody-drug conjugate of the present invention is used for treating esophageal cancer. In some embodiments, the antibody or antibody-drug conjugate of the present invention is used for treating breast cancer. In some embodiments, the antibody or antibody-drug conjugate of the present invention is used for treating kidney cancer. In some embodiments, the antibody or antibody-drug conjugate of the present invention is used for treating breast cancer. In some embodiments, the antibody or antibody-drug conjugate of the present invention is used for treating prostate cancer. In some embodiments, the antibody or antibody-drug conjugate of the present invention is used for treating gastric cancer. In some embodiments, the antibody or antibody-drug conjugate of the present invention is used for treating Enhertu-resistant gastric cancer. In some embodiments, the antibody or antibody-drug conjugate of the present invention is used for treating pancreatic cancer. In some embodiments, the antibody or antibody-drug conjugate of the present invention is used for treating glioma. The treatment can be applied to patients with these types of primary or metastatic tumors. The treatment can also be applied to patients refractory to conventional treatment or who have relapsed after responding to such treatment.

The antibody-drug conjugate (ADC) of the present invention may also be used in combination with other TKI inhibitors or PD1/PDL1 inhibitors for the treatment of various diseases or disorders, such as those characterized by EGFR overexpression. Typical diseases or hyperproliferative disorders include benign or malignant tumors, including epidermal cancer, colon cancer, rectal cancer, head and neck cancer, lung cancer, ovarian cancer, cervical cancer, bladder cancer, esophageal cancer, breast cancer, kidney cancer, prostate cancer, gastric cancer, pancreatic cancer, and glioma. In one embodiment, the tumor or cancer is a tumor or cancer with a KRAS gene mutation and/or a BRAF gene mutation. In further embodiments, the tumor or cancer with the KRAS gene mutation and/or the BRAF gene mutation is a colon cancer, rectal cancer, lung cancer, or pancreatic cancer with the KRAS gene mutation and/or the BRAF gene mutation. In one embodiment, the PD1 inhibitor is an anti-PD1 antibody. In a further embodiment, the anti-PD1 antibody is serplulimab.

The antibody-drug conjugate of the present invention is administered in an effective regimen that means a dose, route of administration, and frequency of administration which can delay onset, reduce severity, inhibit further exacerbation, and/or ameliorate at least one sign or symptom of cancer. If the patient had already had cancer, the regimen may be referred to as a therapeutically effective regimen. If the patient is at increased risk of developing cancer relative to the general population, but has not experienced symptoms, the regimen may be referred to as a prophylactically effective regimen. In some cases, therapeutic or prophylactic efficacy can be observed in individual patients relative to historical controls or past experience in the same patient. In other cases, therapeutic or prophylactic efficacy in preclinical or clinical trials can be demonstrated relative to a control population of untreated patients.

An exemplary dose of the antibody-drug conjugate may be 1 μg/kg to 15 mg/kg (e.g., 0.1 mg/kg to 20 mg/kg), e.g., 1 mg/kg to 7.5 mg/kg, or 2 mg/kg to 7.5 mg/kg, or 3 mg/kg to 7.5 mg/kg of the body weight of the subject, or 0.1-20 mg/kg of the body weight, or 0.5-5 mg/kg of the body weight (e.g., 0.5 mg/kg, 1 mg/kg, 2 mg/kg, 3 mg/kg, 4 mg/kg, 5 mg/kg, 6 mg/kg, 7 mg/kg, 8 mg/kg, 9 mg/kg, or 10 mg/kg), or as a fixed dose of 10-1500 mg or 200-1500 mg. In some methods, the patient is administered a dose of at least 1 mg/kg, at least 1.5 mg/kg, at least 2 mg/kg, at least 3 mg/kg, at least 8 mg/kg, or at least 10

mg/kg, once every one week, once every three weeks, or less frequently. The dose depends on factors such as the frequency of administration, the condition of the patient and the response, if any, to the previous treatment, whether the treatment is prophylactic or therapeutic, and whether the disorder is acute or chronic.

The administration may be parenteral, intravenous, oral, subcutaneous, intraarterial, intracranial, intrathecal, intraperitoneal, local, intranasal, or intramuscular. The administration can also be directly localized to the tumor. It is preferably administered into the systemic circulation by intravenous or subcutaneous administration. Intravenous administration may be performed, for example, by infusion over a period of time (e.g., 30-90 min) or by a single bolus injection.

The frequency of administration depends on factors such as the half-life of the conjugate in circulation, the condition of the patient, and the route of administration. The frequency may be once a day, once a week, once a month, once a quarter, or at irregular intervals in response to changes in the condition in the patient or the progression of the cancer being treated. During a continuous course of treatment, an exemplary frequency of intravenous administration is between twice a week and once a quarter, but the administration may also be at a higher or lower frequency. During a continuous course of treatment, other exemplary frequencies of intravenous administration are between once a week or three times every four weeks, but the administration may also be at a higher or lower frequency. For subcutaneous administration, exemplary dosing frequencies are once a day to once a month, but the administration may also be at a higher or lower frequency.

The number of doses administered depends on the nature of the cancer (e.g., presenting acute or chronic symptoms) and the response of the disorder to the treatment. Between 1 and 10 doses are usually sufficient for acute disorders or acutely exacerbated chronic disorders. Sometimes, single bolus doses (optionally in separate forms) are sufficient for acute disorders or acutely exacerbated chronic disorders. Recurrence or acute exacerbation of acute disorders can be treated repeatedly. For chronic disorders, antibodies may be administered at regular intervals, e.g., once a week, once every two weeks, once a month, once a quarter, or once every six months for at least 1, 5, or 10 years or the lifespan of the patient.

Pharmaceutical compositions for parenteral administration are preferably sterile and substantially isotonic and are manufactured under GMP conditions. Pharmaceutical compositions may be provided in a unit dosage form (i.e., a dose for a single administration). Pharmaceutical compositions may be formulated using one or more physiologically acceptable carriers, diluents, excipients, or auxiliaries. The formulation depends on the chosen route of administration. For injection, the antibody can be formulated in an aqueous solution, preferably in a physiologically compatible buffer such as Hank's solution, Ringer's solution, or normal saline, or acetate buffer (to reduce discomfort at the injection site). The solution may contain formulation agents such as suspending agents, stabilizers and/or dispersing agents. Alternatively, before use, the antibody may be in a lyophilized form for constitution in a suitable vehicle (e.g., sterile pyrogen-free water). The concentration of the antibody in a liquid formulation may be, for example, 1-100 mg/ml, such as 10 mg/ml.

Treatment with the antibody-drug conjugate of the present invention may be combined with immunotherapy, chemotherapy, radiation, stem cell therapy, surgery, or other therapies that are effective for the disorder being treated. Useful classes of other agents that can be administered with EGFR-targeted antibody-drug conjugates as described herein include, for example, antibodies targeting other receptors expressed on cancer cells (e.g., anti-PDL1 antibodies), immunotherapeutic agents (e.g., anti-PD1 antibodies), anti-tubulin agents (e.g., auristatins), DNA minor groove binders, DNA replication inhibitors, alkylating agents (e.g., platinum complexes such as cisplatin, mono(platinum), bis(platinum) and trinuclear platinum complexes, and carboplatin), anthracyclines, antibiotics, antifolates, antimetabolites, chemotherapeutic sensitizers, duocarmycin, etoposide, fluorinated pyrimidines, ionophores, lexitropsins, nitrosourea, platinols, preforming compounds, purine antimetabolites, puromycin, radiosensitizers, steroids, taxanes, topoisomerase inhibitors, vinca alkaloids, etc.

Treatment with an antibody-drug conjugate, optionally in combination with any of the other agents or regimens described above, can increase the median progression-free survival or overall survival time of a patient with a tumor (e.g., epidermal cancer, colon cancer, rectal cancer, head and neck cancer, lung cancer, ovarian cancer, cervical cancer, bladder cancer, esophageal cancer, breast cancer, kidney cancer, prostate cancer, gastric cancer, pancreatic cancer, and glioma), especially in the case of recurrent or refractory tumors, by at least 30% or 40%, but preferably by 50%, 60% to 70%, or even 100% or longer, as compared with the same treatment (e.g., chemotherapy) without the antibody-drug conjugate. Additionally or alternatively, a treatment comprising the antibody-drug conjugate (e.g., standard chemotherapy) can increase the complete response rate, partial response rate, or objective response rate (complete + partial) of a patient with a tumor by at least 30% or 40%, but preferably by 50%, 60% to 70%, or even 100%, as compared with the same treatment (e.g., chemotherapy) without the antibody-drug conjugate.

## 4. Pharmaceutical formulation

Although the antibody-drug conjugate described herein may be used alone (e.g., by administration), it is generally preferably present in a composition or formulation.

In one aspect, the composition is a pharmaceutical composition (e.g., formulation, preparation, or agent) comprising the antibody-drug conjugate described herein and a pharmaceutically acceptable carrier, diluent, or excipient.

In one aspect, the composition is a pharmaceutical composition comprising at least one of the antibody-drug conjugates described herein and one or more other pharmaceutically acceptable ingredients well known to those skilled in the art, including but not limited to pharmaceutically acceptable carriers, diluents, excipients, adjuvants, fillers, buffers, preservatives, antioxidants, lubricants, stabilizers, solubilizers, surfactants (e.g., wetting agents), masking agents, colorants, flavorings, and sweeteners.

In one aspect, the composition further comprises other active agents, such as other therapeutic or prophylactic agents.

Suitable carriers, diluents, excipients, etc. can be found in standard pharmaceutical literatures. See, for example, Handbook of Pharmaceutical Additives, 2nd edition (M. Ash and I. Ash eds.), 2001 (Synapse Information Resources, Inc., Endicott, New York, U.S., Remington's Pharmaceutical Sciences, 20th edition, Lippincott, Williams & Wilkins, 2000; and Handbook of Pharmaceutical Excipients, 2nd edition, 1994.

Another aspect of the present invention relates to a method for preparing a pharmaceutical composition, which comprises mixing at least one [11C]-radiolabeled antibody-drug conjugate or antibody-drug conjugate-like compound as defined herein with one or more other pharmaceutically acceptable ingredients (e.g., carriers, diluents, and excipients) well known to those skilled in the art. If formulated as discrete units (e.g., tablets), each unit contains a predetermined amount (dose) of the active compound.

As used herein, the term "pharmaceutically acceptable" relates to compounds, ingredients, materials, compositions, dosage forms, etc., which are, within the scope of sound medical judgment, suitable for contact with the tissue of the subject in question (e.g., a human), without undue toxicity, irritation, allergic reactions, or other problems or complications, which is commensurate with a rational benefit/risk ratio. Each carrier, diluent, excipient, etc. must also be "acceptable" in the sense of being compatible with the other ingredients of the formulation.

Formulations can be prepared by any method well known in the pharmaceutical field. Such methods include the step of associating the active compound with a carrier that constitutes one or more auxiliary ingredients. Generally, formulations are prepared by uniformly and intimately associating the active compound with a carrier (e.g., a liquid carrier or a finely divided solid carrier) and then shaping the product as needed.

The formulation may be prepared to provide rapid or slow release; immediate, delayed, timed, or sustained release; or a combination thereof.

Formulations suitable for parenteral administration (e.g., by injection) include aqueous or non-aqueous, isotonic, pyrogen-free sterile liquids (e.g., solutions or suspensions), wherein the active ingredient is dissolved, suspended or otherwise provided (e.g., in liposomes or other microparticles). Such liquids may additionally contain other pharmaceutically acceptable ingredients, such as antioxidants, buffers, preservatives, stabilizers, bacteriostats, suspending agents, thickeners, and solutes, which can make the formulation isotonic with the blood (or other relevant bodily fluids) of the intended recipient. Examples of excipients include, for example, water, alcohols, polyols, glycerol, vegetable oils, etc. Examples of suitable isotonic carriers for such formulations include a sodium chloride injection, Ringer's solution, or lactated Ringer's injection. Typically, the concentration of the active ingredient in the liquid is from about 1 ng/ml to about 10 μg/ml, for example from about 10 ng/ml to about 1 μg/ml. The formulation may be present in unit dose or multi-dose sealed containers (e.g., ampoules and vials) and may be stored under freeze-drying (lyophilization) conditions, requiring only the addition of a sterile liquid carrier (e.g., water) for injection (immediately) before use. Temporary injection solutions and suspensions can be prepared from sterile powders, granules, and tablets.

## 5. Article of manufacture and kit

In another aspect, an article of manufacture or kit comprising an anti-EGFR antibody-drug conjugate described herein is provided. The article of manufacture or kit may also comprise instructions for use of the anti-EGFR antibody-drug conjugates described herein in the methods of the present invention. Therefore, in certain embodiments, the article of manufacture or kit comprises instructions for use of the anti-EGFR antibody-drug conjugate described herein in a method for treating cancer (e.g., epidermal cancer, colon cancer, rectal cancer, head and neck cancer, lung cancer, ovarian cancer, cervical cancer, bladder cancer, esophageal cancer, breast cancer, kidney cancer, prostate cancer, gastric cancer, pancreatic cancer, and glioma), the method comprising administering to the subject an effective amount of the anti-EGFR antibody-drug conjugate described herein. In some embodiments, the subject is a human.

The article of manufacture or kit can further comprise a container. Suitable containers include, for example, bottles, vials (e.g., dual-compartment vials), injectors (e.g., single-compartment or dual-compartment injectors), and test tubes. In some embodiments, the container is a vial. The container can be made of various materials, such as glass or plastic. The container accommodates the formulation.

The article of manufacture or kit may also comprise a label or package insert on or associated with the container that may indicate instructions for reconstitution and/or use of the formulation. The label or package insert may further

indicate that the formulation is useful or intended for subcutaneous, intravenous (e.g., intravenous infusion), or other mode of administration to treat cancer (e.g., epidermal cancer, colon cancer, rectal cancer, head and neck cancer, lung cancer, ovarian cancer, cervical cancer, bladder cancer, esophageal cancer, breast cancer, kidney cancer, prostate cancer, gastric cancer, pancreatic cancer, and glioma) in a subject. The container accommodating the formulation can be a disposable vial or a multi-use vial, which allows repeated administration of the reconstituted formulation. The article of manufacture or kit may also comprise a second container containing a suitable diluent. The article of manufacture or kit may also comprise other materials desired from a commercial, therapeutic, and user perspective, including other buffers, diluents, filters, needles, syringes, and package inserts printed with instructions for use.

The article of manufacture or kit herein also optionally comprises a container containing a second agent, wherein the anti-EGFR antibody-drug conjugate is a first agent, and the article of manufacture or kit further comprises instructions on a label or package insert for treating a subject with an effective amount of the second agent. In some embodiments, the second agent is used for eliminating or reducing the severity of one or more adverse events.

In some embodiments, the anti-EGFR antibody-drug conjugate is present in the container as a lyophilized powder. In some embodiments, the lyophilized powder is in a hermetically sealed container such as a vial, ampoule, or pouch, wherein the container indicates the amount of the active agent. In the case of administration of the drug by injection, an ampoule of sterile water for injection or normal saline may, for example, optionally be provided as part of the kit so that the ingredients may be mixed prior to administration. Such kits may also comprise, if desired, one or more of various conventional drug components, such as, for example, a container having one or more pharmaceutically acceptable carriers, an additional container, etc. This is clear to those skilled in the art. The kit may also comprise printed instructions as inserts or labels indicating the amount of components to be administered, administration guidelines, and/or instructions for mixing the components.

**Sequence Listing**

| SEQ ID NO | Gene Name | Amino acid sequence |
|---|---|---|
| 1. | MAB07 heavy chain CDR1 | NYGVH |
| 2. | MAB07 heavy chain CDR2 | VIWSGGNTDYGNEFTS |
| 3. | MAB07 heavy chain CDR3 | ALDYYDYEFAY |
| 4. | MAB07 light chain CDR1 | RASQSIGTNIH |
| 5. | MAB07 light chain CDR2 | YASESIS |
| 6. | MAB07 light chain CDR3 | QQNNNWPTS |
| 7. | MAB07 heavy chain variable region | EVQLVESGGGLVQPGGSLRLSCAASGFSLTNYGVHWVRQAPGKGLEWLGVIWSGGNTDYGNEFTSRFTISRDNAKNSLYLQMNSLRAEDTAVYYCARALDYYDYEFAYWGQGTMVTVSS |
| 8. | MAB07 light chain variable region | EIVLTQSPATLSLSPGERATLSCRASQSIGTNIHWYQQKPGQAPRLLIKYASESISGIPARFSGSGSGTDFTLTISSLEPEDFAVYYCQQNNNWPTSFGGGTKVEIKRTV |

(continued)

| SEQ ID NO | Gene Name | Amino acid sequence |
|---|---|---|
| 9. | MAB07 heavy chain | EVQLVESGGGLVQPGGSLRLSCAASGFSLTNYGVHWVRQAPGKGLEWLGVIWSGGNTDYGNEFTSRFTISRDNAKNSLYLQMNSLRAEDTAVYYCARALDYYDYEFAYWGQGTMVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 10. | MAB07 light chain | EIVLTQSPATLSLSPGERATLSCRASQSIGTNIHWYQQKPGQAPRLLIKYASESISGIPARFSGSGSGTDFTLTISSLEPEDFAVYYCQQNNNWPTSFGGGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |

(continued)

| SEQ ID NO | Gene Name | Amino acid sequence |
|---|---|---|
| 11. | Cetuximab heavy chain | QVQLKQSGPGLVQPSQSLSITCTVSGFSLTNYGVHWVRQSPGKGLEWLGVIWSGGNTDYNTPFTSRLSINKDNSKSQVFFKMNSLQSNDTAIYYCARALTYYDYEFAYWGQGTLVTVSAASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 12. | Cetuximab light chain | DILLTQSPVILSVSPGERVSFSCRASQSIGTNIHWYQQRTNGSPRLLIKYASESISGIPSRFSGSGSGTDFTLSINSVESEDIADYYCQQNNNWPTTFGAGTKLELKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |
| 13. | Human EGFR ECD | LEEKKVCQGTSNKLTQLGTFEDHFLSLQRMFNNCEVVLGNLEITYVQRNYDLSFLKTIQEVAGYVLIALNTVERIPLENLQIIRGNMYYENSYALAVLSNYDANKTGLKELPMRNLQEILHGAVRFSNNPALCNVESIQWRDIVSSDFLSNMSMDFQNHLGSCQKCDPSCPNGSCWGAGEENCQKLTKIICAQQCSGRCRGKSPSDCCHNQCAAGCTGPRESDCLVCRKFRDEATCKDTCPPLMLYNPTTYQMDVNPEGKYSFGATCVKKCPRNYVVTDHGSCVRACGADSYEMEEDGVRKCKKCEGPCRKVCNGIGIGEFKDSLSIN |

(continued)

| SEQ ID NO | Gene Name | Amino acid sequence |
|---|---|---|
| | | ATNIKHFKNCTSISGDLHILPVAFRGDSFTHTPPL DPQELDILKTVKEITGFLLIQAWPENRTDLHAFE NLEIIRGRTKQHGQFSLAVVSLNITSLGLRSLKEI SDGDVIISGNKNLCYANTINWKKLFGTSGQKTK IISNRGENSCKATGQVCHALCSPEGCWGPEPRD CVSCRNVSRGRECVDKCNLLEGEPREFVENSEC IQCHPECLPQAMNITCTGRGPDNCIQCAHYIDGP HCVKTCPAGVMGENNTLVWKYADAGHVCHLC HPNCTYGCTGPGLEGCPTNGPKIPS |
| 14. | Cynomolgus EGFR ECD | LEEKKVCQGTSNKLTQLGTFEDHFLSLQRMFNN CEVVLGNLEITYVQRNYDLSFLKTIQEVAGYVLI ALNTVERIPLENLQIIRGNMYYENSYALAVLSN YDANKTGLKELPMRNLQEILHGAVRFSNNPALC NVESIQWRDIVSSEFLSNMSMDFQNHLGSCQKC DPSCPNGSCWGAGEENCQKLTKIICAQQCSGRC RGKSPSDCCHNQCAAGCTGPRESDCLVCRKFR DEATCKDTCPPLMLYNPTTYQMDVNPEGKYSF GATCVKKCPRNYVVTDHGSCVRACGADSYEM EEDGVRKCKKCEGPCRKVCNGIGIGEFKDTLSIN ATNIKHFKNCTSISGDLHILPVAFRGDSFTHTPPL DPQELDILKTVKEITGFLLIQAWPENRTDLHAFE NLEIIRGRTKQHGQFSLAVVSLNITSLGLRSLKEI SDGDVIISGNKNLCYANTINWKKLFGTSSQKTKI ISNRGENSCKATGQVCHALCSPEGCWGPEPRDC VSCQNVSRGRECVDKCNVLEGEPREFVENSECI QCHPECLPQVMNITCTGRGPDNCIQCAHYIDGP HCVKTCPAGVMGENNTLVWKYADAGHVCHLC HPNCTYGCTGPGLEGCARNGPKIPS |

(continued)

| SEQ ID NO | Gene Name | Amino acid sequence |
|---|---|---|
| 15. | Rat EGFR ECD | LEEKKVCQGTSNRLTQLGTFEDHFLSLQRMFNNCEVVLGNLEITYVQRNYDLSFLKTIQEVAGYVLIALNTVERIPLENLQIIRGNALYENTYALAVLSNYGTNKTGLRELPMRNLQEILIGAVRFSNNPILCNMETIQWRDIVQDVFLSNMSMDVQRHLTGCPKCDPSCPNGSCWGRGEENCQKLTKIICAQQCSRRCRGRSPSDCCHNQCAAGCTGPRESDCLVCHRFRDEATCKDTCPPLMLYNPTTYQMDVNPEGKYSFGATCVKKCPRNYVVTDHGSCVRACGPDYYEVEEDGVSKCKKCDGPCRKVCNGIGIGEFKDTLSINATNIKHFKYCTAISGDLHILPVAFKGDSFTRTPPLDPRELEILKTVKEITGFLLIQAWPENWTDLHAFENLEIIRGRTKQHGQFSLAVVGLNITSLGLRSLKEISDGDVIISGNRNLCYANTINWKKLFGTPNQKTKIMNNRAEKDCKATNHVCNPLCSSEGCWGPEPTDCVSCQNVSRGRECVDKCNILEGEPREFVENSECIQCHPECLPQTMNITCTGRGPDNCIKCAHYVDGPHCVKTCPSGIMGENNTLVWKFADANNVCHLCHANCTYGCAGPGLKGCQQPEGPKIPS |

(continued)

| SEQ ID NO | Gene Name | Amino acid sequence |
|---|---|---|
| 16. | Mouse EGFR ECD | LEEKKVCQGTSNRLTQLGTFEDHFLSLQRMYN NCEVVLGNLEITYVQRNYDLSFLKTIQEVAGYV LIALNTVERIPLENLQIIRGNALYENTYALAILSN YGTNRTGLRELPMRNLQEILIGAVRFSNNPILCN MDTIQWRDIVQNVFMSNMSMDLQSHPSSCPKC DPSCPNGSCWGGGEENCQKLTKIICAQQCSHRC RGRSPSDCCHNQCAAGCTGPRESDCLVCQKFQ DEATCKDTCPPLMLYNPTTYQMDVNPEGKYSF GATCVKKCPRNYVVTDHGSCVRACGPDYYEVE EDGIRKCKKCDGPCRKVCNGIGIGEFKDTLSINA TNIKHFKYCTAISGDLHILPVAFKGDSFTRTPPLD PRELEILKTVKEITGFLLIQAWPDNWTDLHAFEN LEIIRGRTKQHGQFSLAVVGLNITSLGLRSLKEIS DGDVIISGNRNLCYANTINWKKLFGTPNQKTKI MNNRAEKDCKAVNHVCNPLCSSEGCWGPEPRD CVSCQNVSRGRECVEKCNILEGEPREFVENSECI QCHPECLPQAMNITCTGRGPDNCIQCAHYIDGP HCVKTCPAGIMGENNTLVWKYADANNVCHLC HANCTYGCAGPGLQGCEVWPSGPKIPS |

(continued)

| SEQ ID NO | Gene Name | Amino acid sequence |
|---|---|---|
| 17. | Serplulimab heavy chain | QVQLVESGGGLVKPGGSLRLSCAASGFTFSNYGMSWIRQAPGKGLEWVSTISGGGSNIYYADSVKGRFTISRDNAKNSLYLQMNSLRAEDTAVYYCVSYYYGIDFWGQGTSVTVSSASTKGPSVFPLAPCSRSTSESTAALGCLVKDYFPEPVTVSWNSGALTSGVHTFPAVLQSSGLYSLSSVVTVPSSSLGTKTYTCNVDHKPSNTKVDKRVESKYGPPCPPCPAPEFLGGPSVFLFPPKPKDTLMISRTPEVTCVVVDVSQEDPEVQFNWYVDGVEVHNAKTKPREEQFNSTYRVVSVLTVLHQDWLNGKEYKCKVSNKGLPSSIEKTISKAKGQPREPQVYTLPPSQEEMTKNQVSLTCLVKGFYPSDIAVEWESNGQPENNYKTTPPVLDSDGSFFLYSRLTVDKSRWQEGNVFSCSVMHEALHNHYTQKSLSLSLGK |
| 18. | Serplulimab light chain | DIQMTQSPSSLSASVGDRVTITCKASQDVTTAVAWYQQKPGKAPKLLIYWASTRHTGVPSRFSGSGSGTDFTLTISSLQPEDFATYYCQQHYTIPWTFGGGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |

## Examples

The following examples are merely illustrative of the presently disclosed subject matter and should not be considered as limitations in any way.

Reagents and materials:

Organic solvents such as dimethyl sulfoxide were purchased from Spectrum Chemical Mfg Corp, and TCEP-HCl reagent from Thermo Fisher, etc.

IgG1 is a human IgG1, κ isotype control purchased from Beijing Sino Biological, Inc.

pH-sensitive Zenon™ pHrodo™ iFL IgG labeling reagent was purchased from Thermo Fisher.

CellTiter-Glo solution was purchased from Promega.

A549 and NCI-H1993 cell lines were purchased from ATCC, SW480 cell line was purchased from the Cell Bank of the Chinese Academy of Sciences, EBC-1 cell line was purchased from the JCRB Cell Bank, and various PDX transplants were established and maintained by Shanghai Lide Biotech Co., Ltd.

Preparation scheme:

The structures of compounds described in the following examples were determined by nuclear magnetic resonance hydrogen spectroscopy ($^1$HNMR) or liquid chromatography-mass spectrometry (LC-MS).

Bruker 400 MHz nuclear magnetic resonance instrument was used for the measurement of nuclear magnetic resonance hydrogen spectroscopy ($^1$HNMR); the solvent for measurement was deuterated methanol (CD3OD), deuterated chloroform (CDCl3), or hexadeuterated dimethyl sulfoxide (DMSO-d6); and the internal standard substance is tetramethylsilane (TMS).

The abbreviations in nuclear magnetic resonance (NMR) spectra used in the examples are shown below.

s: singlet; d: doublet; t: triplet; q: quartet; dd: double doublet; qd: quarter doublet; ddd: double doubledoublet; ddt: double doubletriplet; dddd: double doubledouble doublet; m: multiplet; br: broad; J: coupling constant; Hz: Hertz; DMSO-d6: deuterated dimethyl sulfoxide. Δ values were expressed in ppm.

Detection by liquid chromatography-mass spectrometry (LC-MS) was carried out using model Agilent 6120B Agilent (ESI) mass spectrometer.

**Example 1. Preparation of linker-toxin (LP62)**

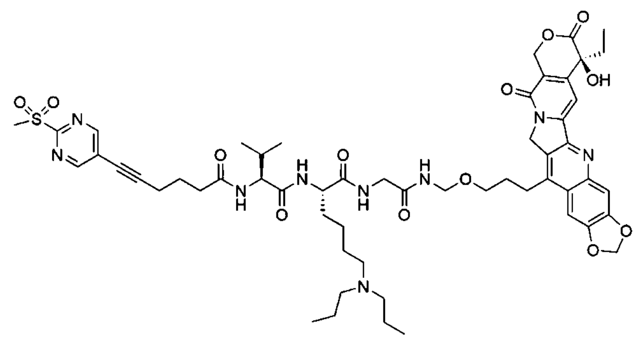

LP62

**Example 1.1: Synthesis of (*S*)-7-ethyl-7-hydroxy-14-(3-hydroxypropyl)-10,13-dihydro-11*H*-[1,3]dioxolo[4,5-*g*] pyrano[3',4',6,7]indolizino[1,2-*b*]quinoline-8,11(7*H*)-dione (A1.3)**

The synthesis route for compound A1.3 is as shown in scheme 1 below:

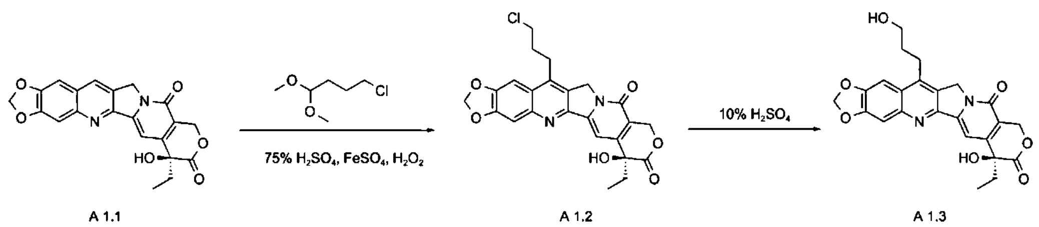


Scheme 1: Preparation of active compound A1.3

The specific steps were as follows:

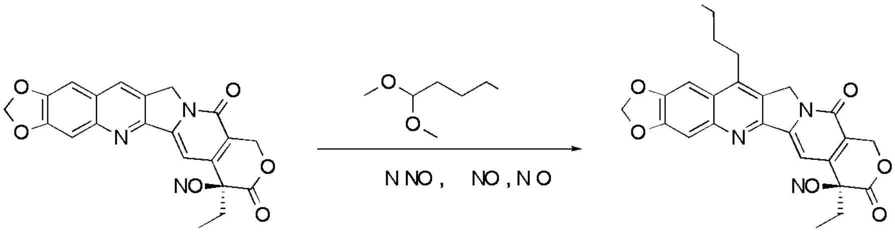


O .    O .

Step 1: Under ice bath conditions, to a solution of (*S*)-7-ethyl-7-hydroxy-10,13-dihydro-11*H*-[1,3]dioxolo[4,5-*g*] pyrano[3',4',6,7]indolizino[1,2-b]quinoline-8,11(7H)-dione (**A1.1**, 500 mg) in 75% sulfuric acid (5 mL) was added ferrous sulfate heptahydrate (570 mg of ferrous sulfate heptahydrate dissolved in 1 mL of water) and 4,4-dimethoxychlorobutane (3.89 g). The reaction liquid was stirred for three minutes, and hydrogen peroxide (29%, 2.5 mL) was then dropwise added under ice bath conditions. After the dropwise addition was complete, the reaction liquid was reacted under stirring at 0°C for 5 minutes, then warmed to room temperature, and reacted under stirring for 3 hours. The reaction liquid was diluted with water (50 mL) and extracted with ethyl acetate (80 mL × 2). The organic phase was washed with a saturated aqueous sodium chloride solution and then dried over anhydrous sodium sulfate. The organic phase was evaporated under reduced pressure and then concentrated to give a crude product, which was purified using preparative liquid chromatography equipped with a C18 column (acetonitrile/0.05% aqueous formic acid solution; 5%-60%) to finally give the target compound (**A1.2**, as a yellow solid, 400 mg, yield: 67%).

LC-MS (ESI) $[M+H]^+$: 468.9:

$^1$HNMR (400 Hz, DMSO-*d6*) δ7.65 (s,1H), 7.51 (s,1H),7.24 (s, 1H), 6.50 (s,1H), 6.30 (s, 2H), 5.42 (s, 2H), 5.26 (s, 2H), 3.81(d, *J* = 5.9 Hz, 2H), 3.22 (s, 2H), 1.98 (d, *J* = 6.7 Hz, 4H), 0.88(t, *J* = 7.2 Hz, 3H).

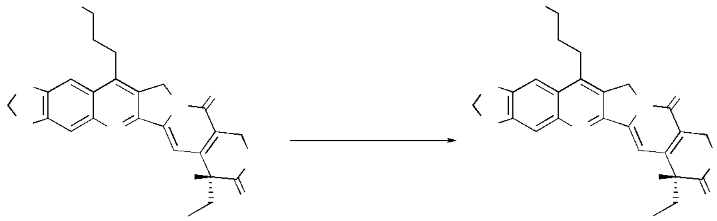

.    .

Step 2: (S)-7-ethyl-7-hydroxy-14-(3-chloropropyl)-10,13-dihydro-11H-1,3]dioxolo[4,5-g]pyrano[3',4',6,7]indolizino[1,2-b]quinoline-8,11(7H)-dione (A1.2, 100 mg, 0.213 mmol) was dissolved in a 10% sulfuric acid solution (5 mL), and the reaction liquid was reacted at 110°C for 48 hours. To the reaction liquid was added a saturated sodium bicarbonate solution (30 mL), and the reaction liquid was extracted with dichloromethane (10 mL × 5), dried over anhydrous sodium sulfate, suction-filtered, and concentrated under reduced pressure to give a crude product. The crude product was subjected to preparative purification by high performance liquid chromatography (acetonitrile/water, with 0.05% formic acid) to give (S)-7-ethyl-7-hydroxy-14-(3-hydroxypropyl)-10,13-dihydro-11*H*-[1,3]dioxolo[4,5-g]pyrano[3',4',6,7]indolizino[1,2-*b*]quinoline-8,11(7*H*)-dione (**A1.3**, 1.78 mg).

LC-MS (ESI) $[M+H]^+$: 451.0:

1HNMR (400 Hz, DMSO-*d6*) δ7.63 (s,1H), 7.50 (s,1H),7.24 (s, 1H), 6.48 (s, 1H), 6.28 (s, 2H), 5.47-5.37 (m, 2H), 5.32-5.19 (m, 2H), 3.51-3.46 (m, 2H), 3.17-3.13 (m, 2H), 1.92-1.76 (m, 4H), 0.90-0.84 (m, 3H).

Example 1.2: Synthesis of compound A2.3

The synthesis route for compound A2.3 is as shown in scheme 2 below:

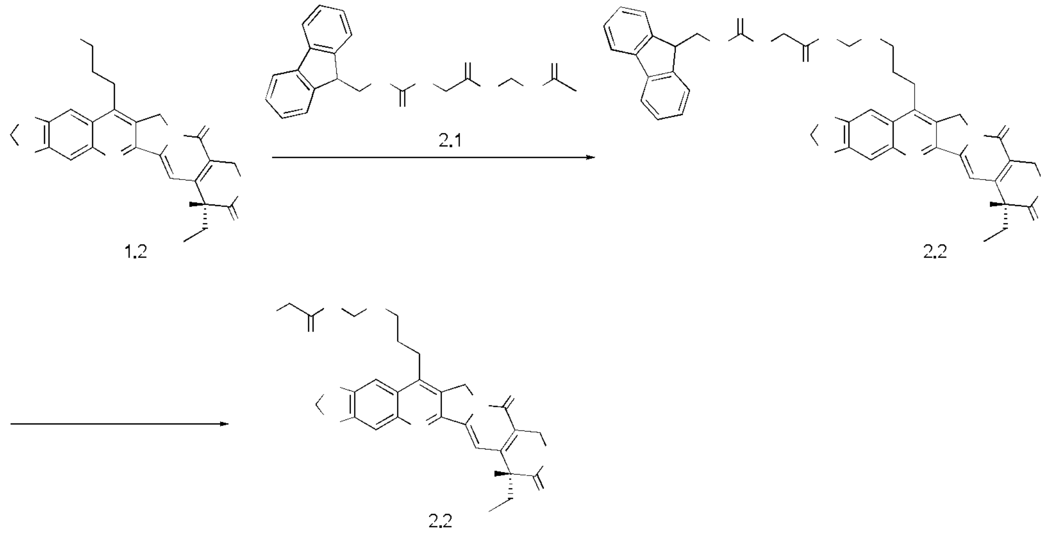


Scheme 2: Synthesis route of compound A2.3

The specific steps for the synthesis of compound A2.3 were as follows:

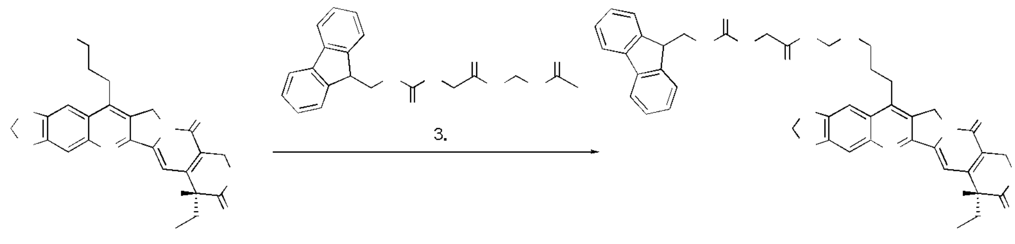


.3 3.3

Step 1: Compound A2.1 (368 mg), compound A1.3 (440 mg), and pyridinium p-toluenesulfonate (PPTS, 25 mg) were refluxed in dichloromethane (20 mL) at 40°C for 20 hours and then washed separately with an aqueous sodium bicarbonate solution and an aqueous hydrochloric acid solution, and the organic solvent was removed under reduced pressure to give a crude product. The crude product was separated and purified by column chromatography (dichloromethane:methanol = 10 : 1) to give the target compound A2.2 (240 mg).

LC-MS (ESI) $[M+H]^+$: 747:

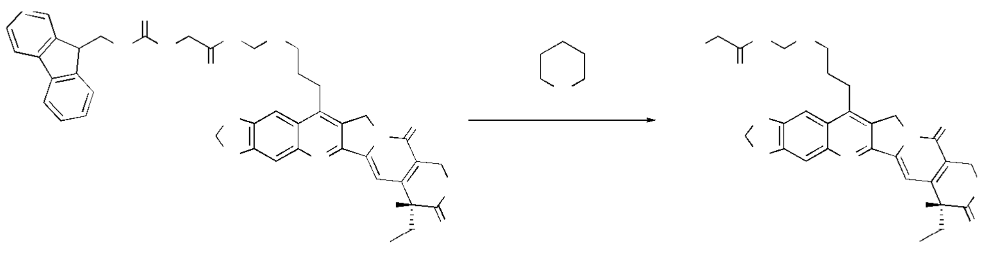

2.2 2.

Step 2: A2.2 (240 mg) was dissolved in DMF (5 mL), and piperidine (1 mL) was added. The mixture was stirred at room temperature for 20 minutes. Low-boiling-point components were removed under pressure, and the residue was used directly in the next step of synthesis. A small amount of the crude product was purified by reverse-phase chromatography (acetonitrile/0.05% FA in water: 5%-50%) to give the target compound A2.3.

LC-MS (ESI) $[M+H]^+$: 525:

$^1$H NMR (400 Hz, DMSO-d6) δ9.13 (t,1H), 8.04 (br, 2H), 7.58 (s, 1H), 7.51 (s, 1H), 7.25 (s, 1H), 6.29 (s, 2H), 5.43 (s, 2H), 5.21 (s, 2H), 4.65 (d, 2H), 3.63 (m, 2H), 3.53 (m, 2H), 3.11 (m, 2H), 1.87 (m, 4H), 0.88 (t, 3H).

**Example 1.3: Synthesis of compound A3.5**

The synthesis route for compound A3.5 is as shown in scheme 3 below:

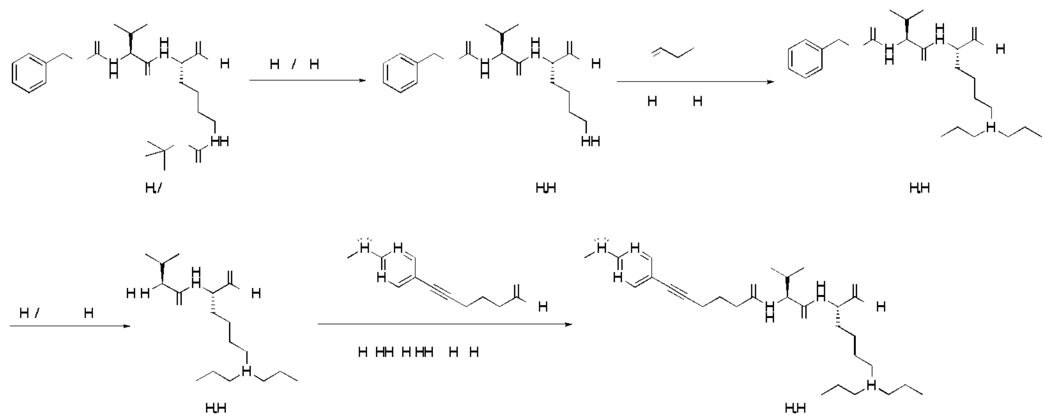

Scheme 3: Synthesis route of compound A3.5

The specific synthesis steps for compound A3.5 were as follows:

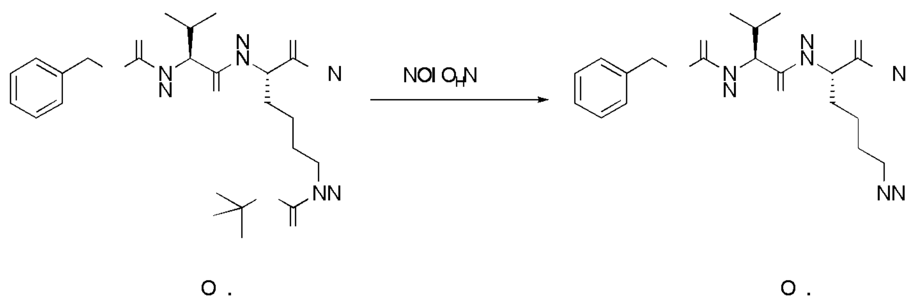

Step 1: To compound A3.1 (10.0 g) was added a solution of hydrogen chloride-dioxane (dioxane, $C_4H_8O_2$) (100 mL), and the mixture was reacted at room temperature for 2 hours. The reaction liquid was concentrated under reduced pressure to give the target compound A3.2 (8.0 g) as a white solid.

LC-MS (ESI) $[M+H]^+$: 380.1

$^1$H NMR (400 MHz, DMSO-d6) δ 8.18 (d, *J*= 7.4 Hz, 1H), 7.40-7.30 (m, 5H), 7.26 (d, *J* = 8.8 Hz, 1H), 5.08-4.99 (m, 2H), 4.23-4.11 (m, 1H), 3.94-3.88 (m, 1H), 2.78-2.73 (m, 2H), 2.03-1.94 (m, 1H), 1.77-1.51 (m, 4H), 1.44-1.31 (m, 2H), 0.87 (dd, *J*= 17.3, 6.6 Hz, 6H).

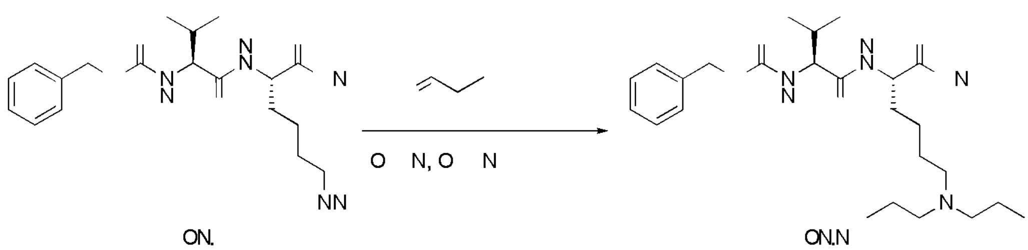

Step 2: Compound A3.2 (5.0 g, 12 mmol) was dissolved in dichloromethane (100 mL), and n-propanal (4.2 g, 72.3 mmol) was added to the reaction liquid. The reaction was stirred at room temperature for 10 minutes, and sodium triacetoxyborohydride (12.8 g, 60.25 mmol) was then added to the reaction liquid. The reaction was stirred at room temperature for 1 hour. LC-MS showed that the reaction was complete. A saturated aqueous ammonium chloride solution was added to the reaction liquid and stirred for 1 hour. After rotary drying and filtration, the filtrate was purified by reverse separation using a C18 column to give the target compound A3.3 (4.57 g, yield 82.0%). The compound A3.3 was a white solid.

LC-MS (ESI) [M+H]$^+$: 464.0:
$^1$H NMR(400 MHz, DMSO-d6) δ 7.81 (d, *J*= 7.3 Hz, IH), 7.38-7.28 (m, 5H), 5.04 (d, *J* = 1.7 Hz,2H),4.12- 4.02 (m, IH), 3.94-3.82 (m, 1H), 2.65-2.52 (m, 6H), 2.06-1.94 (m, 1H), 1.76-1.64 (m, 1H), 1.64-1.53 (m, 1H), 1.52-1.40 (m, 6H), 1.34-1.18 (m, 2H), 0.92-0.80 (m, 12H).

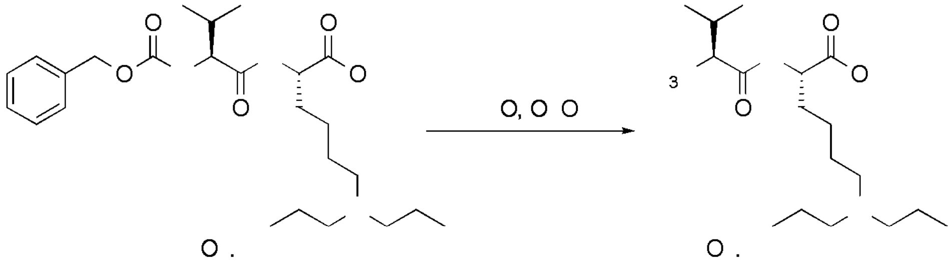


Step 3: Compound 3.3 (2.0 g, 4.32 mmol) was dissolved in methanol (80 mL) at room temperature, and Pd/C (0.16 g) was then added to the reaction liquid. The reaction was stirred at room temperature under hydrogen for 12 hours. After LC-MS showed that the reaction was complete, the reaction liquid was filtered and concentrated under reduced pressure to give the target compound A3.4 (1.2 g, yield 85.5%) as a white solid.

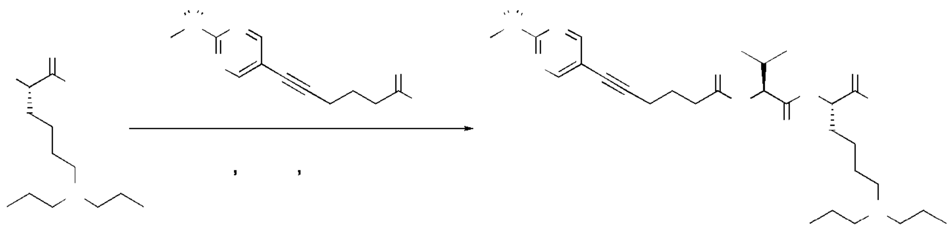

Step 4: 6-(2-(Methylsulfonyl)pyrimidin-5-yl)hex-5-ynoic acid (100 mg, 0.373 mmol) was dissolved in DMF (1 mL), and HATU (142 mg, 0.373 mmol) and *N,N*-diisopropylethylamine (120 mg, 0.93 mmol) were then added. The system was stirred for 30 minutes, and compound A3.4 (122 mg, 0.371 mmol) was then added. The reaction liquid was stirred at room temperature for 1 hour. After the reaction was complete as detected by LC-MS, the reaction liquid was directly purified by reverse phase chromatography on a C18 column (acetonitrile and a 0.05% formic acid aqueous solution system) to obtain the target compound N$^6$,N$^6$-dipropyl-N$^2$-((6-(2-(methylsulfonyl)pyrimidin-5-yl)hex-5-ynoyl)-L-valine)-L-lysine (compound A3.5, 50 mg, yield 28%) as a pale yellow solid.

LC-MS (ESI) [M+H]$^+$: 580.0:
1HNMR (400 MHz, DMSO-d6) δ 8.24 (s, 2H), 7.98-7.93 (m, 2H), 4.24-4.16 (m, 1H), 4.10 (d, *J* = 5.2 Hz, 1H), 3.41 (s, 3H), 2.79-2.64 (m, 6H), 2.55 (t, *J* = 7.1 Hz, 2H), 2.45-2.26 (m, 2H), 2.06-1.91 (m, 1H),1.89-1.78 (m, 2H), 1.76-1.66 (m, 1H), 1.64-1.57 (m, 1H), 1.57-1.42 (m, 6H). 1.37-1.24 (m, 2H), 0.93-0.78 (m, 12H).

**Example 1.4: Synthesis of linker-toxin (LP62)**

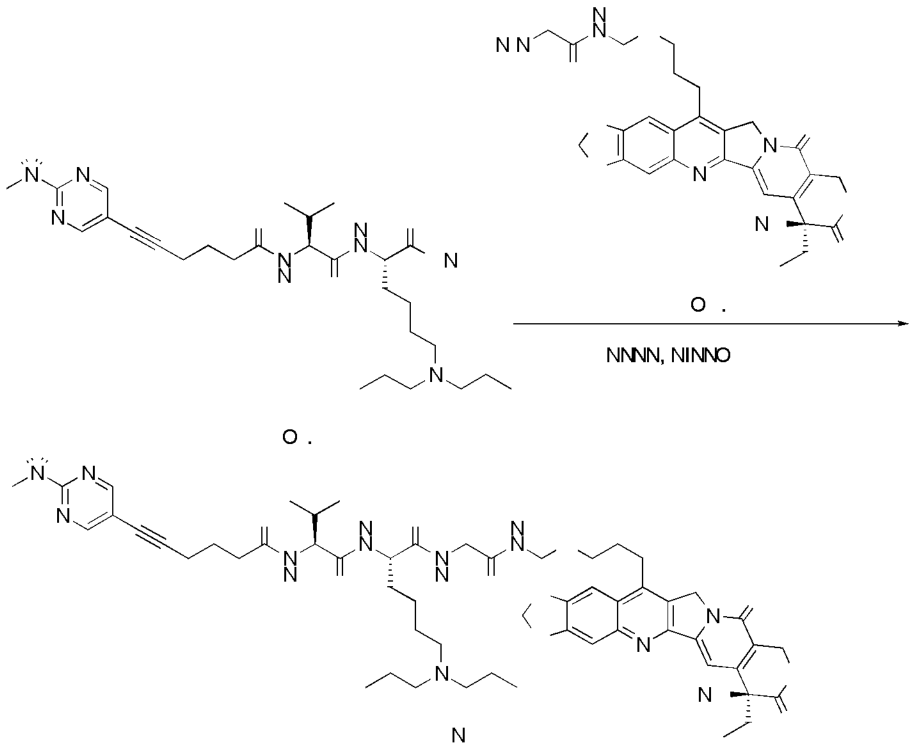


Step 1: Compound A3.5 (43 mg, 0.074 mmol) and compound A2.3 (40 mg, 0.074 mmol) were dissolved in 1 mL of DMF, and HBTU (28 mg, 0.075 mmol) and N,N-diisopropylethylamine (24 mg, 0.187 mmol) were then added sequentially. The reaction liquid was stirred at room temperature for 1 hour. After the reaction was complete as detected by LC-MS, the reaction liquid was directly purified by preparative chromatography (a 0.01% trifluoroacetic acid aqueous solution, acetonitrile) to give the target compound LP62 as a trifluoroacetate salt (8.5 mg, yield 10%) as a yellow solid.

LC-MS (ESI) $[M+H]^+$: 1098.6:

$^1H$ NMR (400 MHz, DMSO-$d_6$): δ 9.10 (s, 2H), 9.03 (s, 1H), 8.64 (t, *J* = 6.4 Hz, 1H), 8.19 (m, *J= 5.9* Hz, 1H), 8.08 (d, *J* = 7.4 Hz,1H), 7.92 (d, *J* = 8.5 Hz,1H), 7.59 (s, 1H), 7.51 (s, 1H), 7.24 (s, 1H), 6.49 (s, 1H), 6.29 (s, 2H), 5.42 (s, 2H), 5.24 (s, 2H), 4.66-4.52 (m, 2H), 4.31-4.21 (m, 1H), 4.19-4.10 (m, 1H), 3.74 (d, *J* = 5.5 Hz, 2H), 3.49-3.48 (m,2H),3.40 (s, 3H), 3.15-3.06 (m,2H), 3.02-2.95 (m,6H), 2.59-2.52 (m, 3H), 2.41-2.29 (m, 2H), 2.50-1.90 (m, 2H), 1.91-1.77 (m, 6H), 1.63-1.57 (m, 6H), 1.31-1.29 (m,2H), 0.92-0.80 (m,15H).

**Example 2. Preparation of antibody**

Unless otherwise specified, the anti-EGFR antibody used in the present invention is MAB07, and the heavy chain and light chain amino acid sequences thereof are described in the Sequence Listing.

In general, the antibody was prepared as follows: A stable CHO cell line expressing and secreting MAB07 was constructed. The stable CHO cell line was cultured in a suitable culture medium. A culture supernatant was harvested and subjected to sequentially depth filtration, protein A column affinity chromatography, virus inactivation, filtration, anion chromatography, cation chromatography, nanofiltration/ultrafiltration, and other processes. Finally, a target antibody was obtained.

**Example 3. Preparation and detection of antibody-drug conjugate**

**Example 3.1: Preparation of MAB07-LP62**

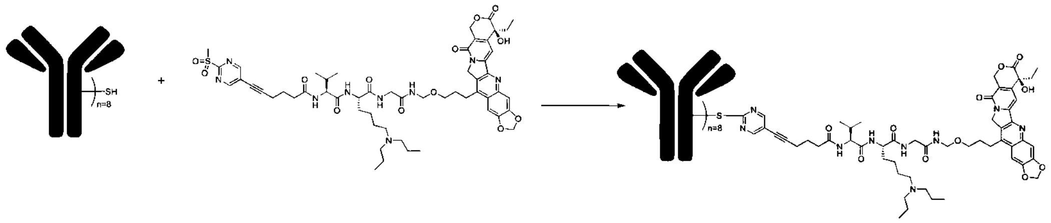

wherein n = 8, indicating the molar ratio of the linker-loaded drug to the antibody.

2-5 mL of the anti-EGFR antibody MAB07 (Anti-EGFR_mAb, 15-25 mg/mL) was taken and placed in a reaction tube. EDTA at a final concentration of 10-20 mM was added, and the pH was then adjusted to 6.0-7.5 with a 0.5-1 M disodium hydrogen phosphate solution. 10-20 mM TCEP (tris(2-carboxyethyl)phosphine, 4-10 eq., 0.1-0.4 mL) was slowly added, and the mixture was mixed uniformly and reacted at room temperature for 50-80 minutes. After the reduction reaction of MAB07 was complete, a solution of linker-toxin LP62 (4-12 eq., 1-8 μmol) previously dissolved in dimethylsulfoxide (DMSO) or DMAC solvent was added to the above solution system, and the system was stirred and mixed uniformly and reacted at room temperature for 100-150 minutes. After purification by cation chromatography, the system buffer was replaced with 10-50 mM PB or histidine-histidine hydrochloride, pH 5.5-7.5, by using Amicon Ultra-15 to give MAB07-LP62, a conjugate product of MAB07 and the linker-toxin LP62. The DAR value and monomer content thereof were verified by LC-MS, HIC, and HPLC-SEC analysis and met the standards.

**Example 3.2: Preparation of reference ADC Cetuximab-LP62.**

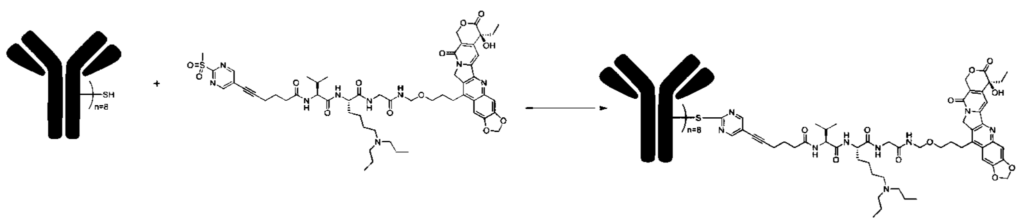

wherein n = 8, indicating the molar ratio of the linker-loaded drug to the antibody.

2-5 mL of cetuximab (15-25 mg/mL) was taken and placed in a reaction tube. EDTA at a final concentration of 10-20 mM was added, and the pH was then adjusted to 6.0-7.5 with a 0.5-1 M disodium hydrogen phosphate solution. 10-20 mM TCEP (tris(2-carboxyethyl)phosphine, 4-10 eq., 0.1-0.4 mL) was slowly added, and the mixture was mixed uniformly and reacted at room temperature for 50-80 minutes. After the reduction reaction of cetuximab was complete, a solution of linker-toxin LP62 (4-12 eq., 1-8 μmol) previously dissolved in dimethylsulfoxide (DMSO) or DMAC solvent was added to the above solution system, and the system was stirred and mixed uniformly and reacted at room temperature for 100-150 minutes. After cation chromatography, the system buffer was replaced with 10-50 mM PB or histidine-histidine hydrochloride, pH 5.5-7.5, by using Amicon Ultra-15 to give Cetuximab-LP62, a conjugate product of cetuximab and the linker-toxin LP62. The DAR value and monomer content thereof were verified by LC-MS, HIC, and HPLC-SEC analysis and met the standards.

**Example 3.3: Preparation of reference ADC MAB07-VC-MMAE**

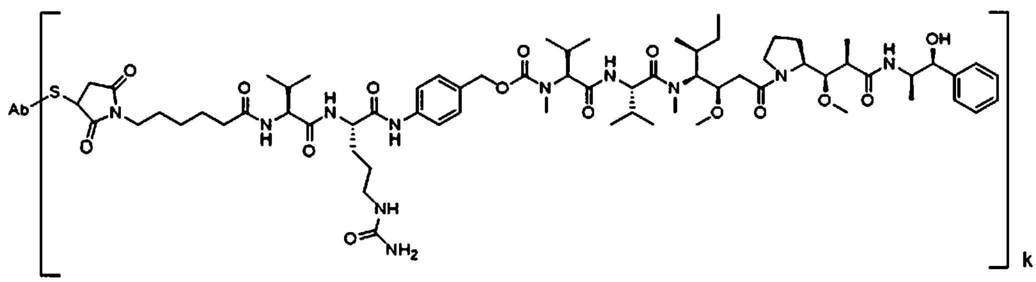

Structure of MAB07-VC-MMAE (in which Ab was MAB07, and k was the DAR value)

MAB07-VC-MMAE was prepared according to a general method in the prior art. Specifically, 2-5 mL of the anti-EGFR antibody MAB07 (Anti-EGFR_mAb, 15-25 mg/mL) was taken and placed in a reaction tube. 10-20 mM EDTA was added, and the pH was then adjusted to 6.0-7.5 with a 0.5-1 M disodium hydrogen phosphate solution. 10-20 mM TCEP (tris(2-carboxyethyl)phosphine, 4-10 eq., 0.1-0.4 mL) was slowly added, and the mixture was mixed uniformly and reacted at room temperature for 50-80 minutes. After the reduction reaction of MAB07 was complete, a solution of linker-toxin MC-VC-PABC-MMAE (the structure of which was as shown below) (4-12 eq., 1-8 μmol) previously dissolved in a dimethylsulfoxide (DMSO) or DMAC solvent was added to the above solution system, and the system was stirred and mixed uniformly and placed at room temperature for 100-150 minutes. The system buffer was replaced with 10-50 mM PB or histidine-histidine hydrochloride, pH 5.5-7.5, by using Amicon Ultra-15 to give MAB07-VC-MMAE, a conjugate product of MAB07 and the linker-toxin MC-VC-PABC-MMAE. The DAR value and monomer content thereof were verified by LC-MS, HIC, and HPLC-SEC analysis and met the standards.

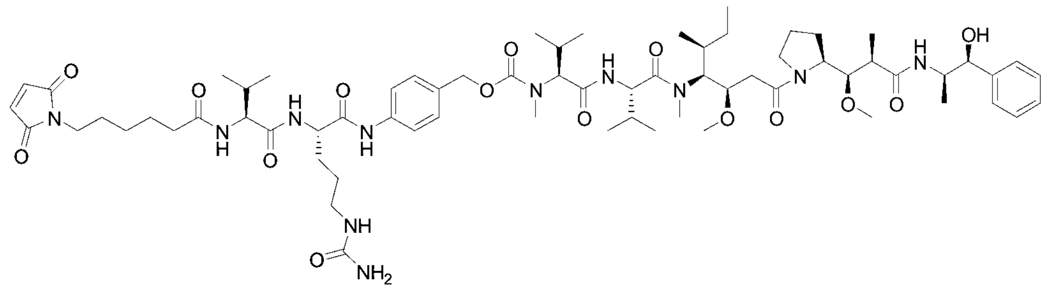

MC-VC-PABC-MMAE

**Example 3.4: Preparation of reference ADC MAB07-GGFG-DXD**

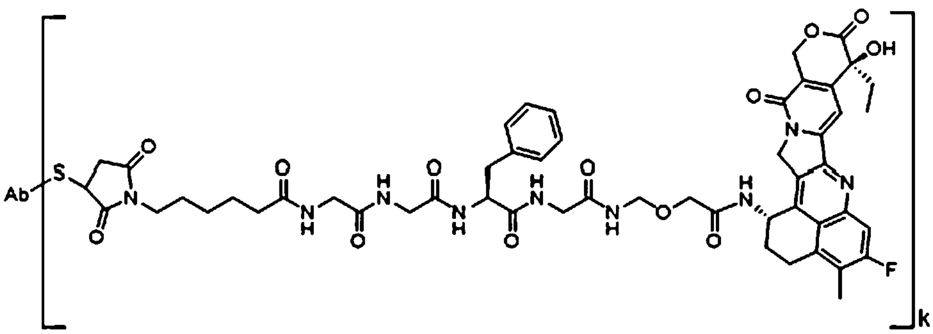

Structure of MAB07-GGFG-DXD (in which Ab was MAB07, and k was the DAR value)

MAB07-GGFG-DXD was prepared according to a general method in the prior art. Specifically, 2-5 mL of the anti-EGFR antibody MAB07 (Anti-EGFR_mAb, 15-25 mg/mL) was taken and placed in a reaction tube. EDTA at a final concentration of 10-20 mM was added, and the pH was then adjusted to 6.0-7.5 with a 0.5-1 M disodium hydrogen phosphate solution. 10-20 mM TCEP (tris(2-carboxyethyl)phosphine, 4-10 eq., 0.1-0.4 mL) was slowly added, and the mixture was mixed uniformly and reacted at room temperature for 50-80 minutes. After the reduction reaction of MAB07 was complete, a solution of linker-toxin MC-GGFG-DXD (the structure of which was as shown below) (8-12 eq., 2-10 μmol) previously dissolved in a dimethylsulfoxide (DMSO) or DMAC solvent was added to the above solution system, and the system was stirred and mixed uniformly and placed at room temperature for 100-150 minutes. The system buffer was replaced with 10-50 mM PB or histidine-histidine hydrochloride, pH 5.5-7.5, by using Amicon Ultra-15 to give MAB07-GGFG-DXD, a conjugate product of MAB07 and the linker-toxin MC-GGFG-DXD. The DAR value and monomer content thereof were verified by LC-MS, HIC, and HPLC-SEC analysis and met the standards.

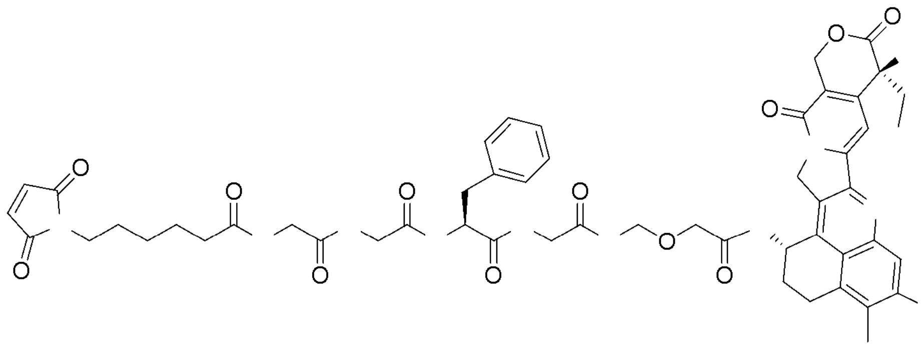

MC-GGFG-DXD

**Example 3.5: Size exclusion chromatography (HPLC-SEC) for monomer and conjugate aggregation analysis**

Size exclusion chromatography analysis was carried out on Agilent 1260 liquid chromatography system equipped with a TSKgel® G3000SWxL column (Tosoh Bioscience LLC, 7.8 mm × 30 cm, 5 μm pore size). The naked antibody or conjugate sample was eluted off the column with 0.1 M sodium phosphate containing 0.15 M sodium chloride and 10%-20% isopropanol (pH 7.4) at a flow rate of 0.5-1.0 mL/minute for about 30-60 minutes. All data were analyzed using Agilent ChemStation software. The percent aggregation of the naked antibody or conjugate was calculated as follows:

$$\text{Percent aggregation} = \frac{PA_{\text{aggregate}}}{PA_{\text{total}}} \times 100\%$$

wherein: *PA* = Integrated peak area

The calculated monomer purity of the resulting conjugate is listed below:

| Table 2: Size exclusion chromatography analysis of anti-EGFR antibody-drug conjugate | | | |
|---|---|---|---|
| Sample name | Aggregate (%) | Main peak (%) | Fragment (%) |
| MAB07-LP62 | 0.4 | 98.9 | 0.6 |
| Cetuximab-LP62 | 0.3 | 99.2 | 0.5 |

The results showed that the resulting anti-EGFR antibody-drug conjugate existed primarily in the monomeric state and exhibited excellent anti-aggregation properties.

**Example 3.6: Analysis of antibody-drug conjugate by LC-MS for loaded drug : antibody ratio (DAR)**

An antibody-drug conjugate sample was reduced with DTT at 37°C for 10 minutes, and the DAR value of the conjugate was then analyzed using LC-MS. Specifically, the reduced sample was injected into an LC-MS system (Agilent 1290 Infinity II LC system and Agilent 6545XT AdvanceBio Q-TOF) equipped with an Acquity UPLC BEH C4 reverse phase column (Waters Corporation, 2.1 mm × 50 mm, 1.7 μm, 300 Å). The drug : antibody ratio (DAR) was calculated using the provided deconvoluted LC-MS results.

| Table 3: Analysis of anti-EGFR antibody-drug conjugate by LC-MS for drug : antibody ratio (DAR) | | | |
|---|---|---|---|
| Sample name | Chain | DAR for LC/HC | DAR for IgG |
| MAB07-LP62 | LC (light chain) | 1.00 | 8.00 |
| | HC (heavy chain) | 3.00 | |
| Cetuximab-LP62 | LC (light chain) | 1.00 | 8.00 |
| | HC (heavy chain) | 3.00 | |

**Example 4. Assay for affinity of anti-EGFR antibody-drug conjugate for EGFR-positive cells**

Normally grown and passaged A549 and SW480 tumor cells were respectively collected, washed with PBS, resuspended, and inoculated into a 96-well plate at $5 \times 10^5$ cells/well/50 μl. The test sample was then diluted with a gradient using, for example, an FACS buffer (PBS + 2% FBS), and after dilution, the test sample to be tested and the control sample were added sequentially to the 96-well plate inoculated with the cells, wherein the control sample IgG1 was an isotype control (purchased from Beijing Sino Biological, Inc.). The sample plate was incubated at 4°C for 0.5 h and centrifuged, followed by supernatant removal and washing with 300 μl/well of FACS buffer. Subsequently, a fluorescently labeled goat anti-human IgG antibody detection solution was added to the sample plate (100 μl/well) in a sequential manner for incubation with the cells. The sample plate was incubated at 4°C for 0.5 h and washed with 300 μl/well of FACS buffer, and finally, the cells were resuspended. The fluorescence numerical value for each sample was detected by a flow cytometer. The data were analyzed on Graphpad prism 8.1 and fitted to a four-parameter equation.

As shown in FIG. 1A, the anti-EGFR antibody-drug conjugate MAB07-LP62 exhibited better affinity for EGFR-positive cells A549, regardless of whether the DAR was high (DAR8) or low (DAR4), and the affinity thereof was comparable to the affinity of the unconjugated antibody. Similarly, as shown in FIG. 1B, MAB07-LP62 (DAR8) also exhibited comparable affinity to the unconjugated antibody for EGFR-positive cells SW480.

**Example 5. Assay for internalization of anti-EGFR antibody-drug conjugate into EGFR-positive cells**

A volume of normally grown and passaged A549 cells was taken and centrifuged, followed by supernatant removal and resuspension in a cell culture medium (RPMI-1640 culture medium + 10% FBS). 50 μl of the cell suspension was inoculated into a 96-well plate for use ($1 \times 10^5$ cells/well). A pH-sensitive Zenon™ pHrodo™ iFL IgG labeling reagent (purchased from ThermoFisher) was formulated with the cell culture medium to form 4x working solution 1 (480 nM). A conjugate of this pH-sensitive dye and IgG did not fluoresce extracellularly, but fluoresced brightly in an acidic environment, including lysosomes. The test material was formulated with the cell culture medium to form 4x working solution 2 (160 nM). 25 μl of working solution 1 and 25 μl of working solution 2 were taken separately and added to a 96-well plate, followed by mixing and incubation. The mixed liquid was pipetted into 50 μl of the cell suspension, mixed uniformly, and placed in an incubator for culturing. Samples were taken at 0, 2, 16, 24 h time points and centrifuged, and the supernatants were removed. The resulting material was then washed with 300 μl/well of FACS buffer and resuspended, and the fluorescence value of each sample was measured using a flow cytometer.

As shown in FIG. 2, as compared with the data of the IgG1 isotype control, the proportion of MAB07-LP62 and MAB07 internalization increased as the incubation time increased and reached the peak at 16 h, indicating that the anti-EGFR antibody-drug conjugate MAB07-LP62 can effectively bind the EGFR antigen on the cell surface and be internalized into the intracellular lysosomal pathway.

**Example 6. Assay for cell killing activity of anti-EGFR antibody-drug conjugate**

Cells in the exponential growth phase were collected, and viable cells were counted using Vi-Cell XR cytometer. The cell suspension was adjusted to an appropriate concentration with a culture medium. 80 μl/well of the cell suspension was added to a cell culture plate. The test sample was diluted with a gradient (5-fold dilutions). 20 μl of each of the diluted test articles was taken and added to the cell suspension in the cell culture plate, and the cell culture plate was placed in an incubator and cultured for 72 hours. After 72 hours, 50 μl/well of a CellTiter-Glo solution (purchased from Promega) that had been thawed in advance and equilibrated to room temperature was added according to CellTiter-Glo operation instructions. The wells were mixed uniformly with a microwell plate shaker for 2 minutes and maintained at room temperature for 10 minutes. The fluorescence signal was measured using Envision 2104 plate reader.

As shown in FIG. 3, MAB07-LP62 showed stronger cell killing effects on the MAB07-insensitive EGFR-positive tumor cells NCI-H1993 and EBC-1.

**Example 7. Cell killing activity comparison between anti-EGFR antibody-drug conjugates**

The human colon adenocarcinoma cell line SW480 in the exponential growth phase was collected, and viable cells were counted using Vi-Cell XR cytometer. The cell suspension was adjusted to an appropriate concentration with a culture medium. 80 μl/well of the cell suspension was added to a cell culture plate. The test sample was diluted with a gradient (5-fold dilutions). 20 μl of each of the diluted test articles was taken and added to the cell suspension in the cell culture plate, and the cell culture plate was placed in an incubator and cultured for 72 hours. After 72 hours, 50 μl/well of a CellTiter-Glo solution (purchased from Promega) that had been thawed in advance and equilibrated to room temperature was added according to CellTiter-Glo operation instructions. The wells were mixed uniformly with a microwell plate shaker for 2 minutes and maintained at room temperature for 10 minutes. The fluorescence signal was measured using Envision

2104 plate reader.

As shown in Table 4, MAB07-LP62 outperformed Cetuximab-LP62 in terms of in vitro cell killing effect.

Table 4. Cell killing activity of anti-EGFR antibody-drug conjugates against human colon adenocarcinoma cell line SW480

| ADC sample | DAR value | IC50 (nM) |
|---|---|---|
| MAB07-LP62 | 8 | 1161 |
| Cetuximab-LP62 | 8 | 2702 |

**Example 8. Cross-binding activity of anti-EGFR antibody-drug conjugate to EGFRs from different species**

Extracellular domains (ECDs) of EGFRs derived from human, cynomolgus monkey, rat, and mouse were separately diluted with 1×PBS to a working concentration of 2 μg/mL, as ELISA coating solutions. The test plate was washed 3 times with PBST (PBS with 0.05% Tween 20). The plate was incubated with a blocking solution SuperBlock T20 (PBS) Blocking Buffer (60 μL/well) at room temperature, the test sample was then diluted with a gradient, and the diluted samples were then loaded separately into a test plate and incubated at room temperature. The test plate was washed, and HRP enzyme-labeled goat anti-human IgG was added as a secondary antibody, followed by incubation. The test plate was washed, and a stop solution (30 μL/well) was added, followed by centrifugation. The light absorbance at a wavelength of 450 nm was read. The raw data were analyzed using Graphpad prism 8.1 and fitted to a four-parameter equation.

As shown in FIG. 4, the binding affinity of MAB07-LP62 for cynomolgus EGFR ECD was consistent with that for human EGFR ECD, with EC50 of 0.074 nM and 0.033 nM, respectively. MAB07-LP62 (DAR8) showed weak specific binding to both rat and mouse EGFR ECDs.

**Example 9. Evaluation of anti-tumor activity of anti-EGFR antibody-drug conjugate in BALB/c nude mouse model subcutaneously xenografted with human epidermal cancer cell line A431**

A431 cells were cultured in a growth culture medium (DMEM 1640 + 10% FBS). Cells in the logarithmic growth phase were collected and resuspended in PBS and Matrixgel matrix for subcutaneous inoculation in mice. The mice to be inoculated were 7-8 weeks old, which were inoculated subcutaneously on the right back with A431 cells ($5 \times 10^6$/mouse). Once the tumor had grown to an average volume of 152.78 $mm^3$, the mice were randomly grouped depending on the tumor size and dosed, ensuring similar tumor volumes between groups. The day of grouping was defined as day 0. After the start of dosing, the body weight and tumor size of the mice were measured twice a week, and the mice were dosed once a week, for three doses. Clinical symptoms observed during the experiment were recorded. The tumor volume was calculated according to the following formula: tumor volume ($mm^3$) = $1/2 \times (a \times b^2)$ (wherein a represented the long diameter, and b represented the short diameter).

As shown in FIG. 5, on day 21 post-dose, Cetuximab-LP62 showed no tumor inhibitory effect (TGI -15.47%) relative to the control group (PBS). MAB07-LP62 exhibited a tumor inhibitory effect (TGI 42.66%). It was shown that MAB07-LP62 had superior anti-tumor activity in vivo than Cetuximab-LP62.

**Example 10. Evaluation of anti-tumor activity of anti-EGFR antibody-drug conjugate in BALB/c nude mouse model subcutaneously xenografted with human non-small cell lung cancer cell line NCI-H1993**

NCI-H1993 cells were cultured in a growth culture medium (RPMI 1640 + 10% FBS). Cells in the logarithmic growth phase were collected and resuspended in PBS and Matrixgel matrix for subcutaneous inoculation in mice. The mice to be inoculated were 6-7 weeks old, which were inoculated subcutaneously on the right back with NCI-H1993 cells ($1 \times 10^7$/mouse). Once the tumor had grown to an average volume of 199.56 $mm^3$, the mice were randomly grouped depending on the tumor size and dosed, ensuring similar tumor volumes between groups. The day of grouping was defined as day 0. After the start of dosing, the body weight and tumor size of the mice were measured twice a week, and the mice were dosed once a week, for three doses. Clinical symptoms observed during the experiment were recorded. The tumor volume was calculated according to the following formula: tumor volume ($mm^3$) = $1/2 \times (a \times b^2)$ (wherein a represented the long diameter, and b represented the short diameter).

As shown in FIG. 6, on day 24 post-dose, compared with the control group (MAB07), MAB07-LP62 (DAR4) (3 mg/kg and 8 mg/kg) and MAB07-LP62 (DAR8) (3 mg/kg and 8 mg/kg) both showed significant tumor growth inhibition, with TGI% of 47.0% ($p = 0.18$), 81.5% ($p < 0.001$), 53.4% ($p = 0.0106$), and 84.7% ($p < 0.001$), respectively. Both MAB07-LP62 (DAR8) and MAB07-LP62 (DAR4) exhibited significant dose-dependent anti-tumor effects. Compared with the antibody-drug conjugates MAB07-VC-MMAE (DAR4) (day 24, 3 mg/kg TGI% 38.6%) and MAB07-GGFG-DXD (DAR8) (day 24, 3

mg/kg, TGI% 28.4%; 8 mg/kg 73.3%) constructed based on a conjugation technique in the prior art, MAB07-LP62 (DAR8) showed significantly excellent anti-tumor activity. None of the animals experienced significant body weight loss throughout the treatment period.

**Example 11. Evaluation of anti-tumor activity of anti-EGFR antibody-drug conjugate in BALB/c nude mouse model subcutaneously xenografted with human non-small cell lung cancer cell line EBC-1**

EBC-1 cells were cultured in a growth culture medium (MEM + 10% FBS + 1% NEAA). Cells in the logarithmic growth phase were collected and resuspended in PBS and Matrixgel matrix for subcutaneous inoculation in mice. The mice to be inoculated were 7-8 weeks old, which were inoculated subcutaneously on the right back with EBC-1 cells ($3 \times 10^6$/mouse). Once the tumor had grown to an average volume of 200.41 $mm^3$, the mice were randomly grouped depending on the tumor size and dosed, ensuring similar tumor volumes between groups. The day of grouping was defined as day 0. After the start of dosing, the body weight and tumor size of the mice were measured twice a week, and the mice were dosed once a week, for three doses. Clinical symptoms observed during the experiment were recorded. The tumor volume was calculated according to the following formula: tumor volume ($mm^3$) = $1/2 \times (a \times b2)$ (wherein a represented the long diameter, and b represented the short diameter).

As shown in FIG. 7, on day 17 post-dose, as compared with the control group (MAB07), MAB07-LP62 (DAR4) (3 mg/kg and 8 mg/kg), MAB07-LP62 (DAR8) (3 mg/kg and 8 mg/kg), MAB07-GGFG-DXd (DAR8) (8 mg/kg), and MAB07-VC-MMAE (DAR4) (3 mg/kg) all exhibited significant tumor inhibitory effects, with TGI% of 67.06% ($p = 0.00126$), 96.58% ($p < 0.001$), 97.83% ($p < 0.001$), 100.00% ($p < 0.001$), 82.12% ($p < 0.001$), and 99.21% ($p < 0.001$), respectively. The test drugs in this experiment all exhibited dose-dependent anti-tumor effects. As compared with the other test drugs, MAB07-LP62 (DAR8) achieved a complete response (CR) in this tumor model. In contrast, the other test drugs all showed tumor growth to different extents during the observation period of 4 weeks after the end of dosing. MAB07-LP62 (DAR8) was shown to have a very strong tumor inhibitory effect. None of the animals experienced significant body weight loss throughout the treatment period.

**Example 12. Evaluation of anti-tumor effect of anti-EGFR antibody-drug conjugate in male BALB/c nude mouse model subcutaneously xenografted with HuPrime® lung cancer LU3075**

A tumor tissue was harvested from HuPrime® lung cancer xenograft model LU3075 tumor-bearing mice and cut into tumor blocks of 2-3 mm in diameter. BALB/c nude mice aged 8-9 weeks were inoculated subcutaneously with the resulting tumor blocks at the right anterior scapula. Once the tumor had grown to an average volume of 181.60 $mm^3$, the mice were randomly grouped depending on the tumor size and dosed, ensuring similar tumor volumes between groups. The day of grouping was defined as day 0. After the start of dosing, the body weight and tumor size of the mice were measured twice a week, and the mice were dosed once a week, for three doses. Clinical symptoms observed during the experiment were recorded. The tumor volume was calculated according to the following formula: tumor volume ($mm^3$) = $1/2 \times (a \times b^2)$ (wherein a represented the long diameter, and b represented the short diameter).

As shown in FIG. 8, on day 28 post-dose, MAB07-LP62 (DAR8) exhibited stronger tumor inhibitory effects at all of 1 mg/kg (TGI 79.88%), 3 mg/kg (TGI 76.55%), and 8 mg/kg (TGI 91.47%). In addition, the group dosed with a combination of 3 mg/kg MAB07-LP62 (DAR8) and 20 mg/kg osimertinib (day 28, TGI 93.80%) showed superior efficacy than MAB07-LP62 (DAR8) alone (day 28, TGI 76.55%) or osimertinib alone (day 28, TGI 61.00%). 8 mg/kg MAB07 showed a TGI of 35.20% on day 28 post-dose. This example demonstrated the advantages of MAB07-LP62 in combination with small molecule inhibitors such as osimertinib. None of the animals experienced significant body weight loss throughout the treatment period.

**Example 13. Evaluation of anti-tumor effect of anti-EGFR antibody-drug conjugate in human lung cancer PDX (patient-derived xenograft) xenografted NCG mouse model**

Osimertinib-resistant EGFR exon19del/T790M/C797S mutant human lung cancer LD1-0025-200717 PDX model was revived in NCG mice. Once the tumor had grown to 500-800 $mm^3$, the tumor tissue was surgically aseptically excised, and non-tumor tissues and necrotic tissues were removed. The tumor tissue was stripped and evenly cut into about 3 mm $\times$ 3 mm $\times$ 3 mm tissue blocks, and then, the NCG mice were inoculated subcutaneously on the right back with the small tumor tissue blocks. 6 days after inoculation, $5 \times 10^6$ PBMCs were injected intraperitoneally per mouse. Once the tumor had grown to an average volume of about 110 $mm^3$, the mice were randomly grouped depending on the tumor size and dosed, ensuring similar tumor volumes between groups. The day of grouping was defined as day 0. After the start of dosing, the body weight and tumor size of the mice were measured twice a week, and the mice were dosed once or dosed for three weeks. Clinical symptoms observed during the experiment were recorded. The tumor volume was calculated according to the following formula: tumor volume ($mm^3$) = $1/2 \times (a \times b^2)$ (wherein a represented the long diameter, and b

represented the short diameter).

As shown in FIG. 9, in osimertinib-resistant lung cancer PDX model LD1-0025-200717, as compared with the PBS group, IgG1-LP62 TGI% was 58.1% ($p < 0.0001$), osimertinib and serplulimab had no apparent tumor inhibitory effects, and MAB07-LP62 (DAR8), whether given alone or in combination (with osimertinib or serplulimab), resulted in sustained remission of the tumor at 1 mg/kg, 3 mg/kg, and 8 mg/kg doses. None of the animals experienced significant body weight loss throughout the treatment period.

**Example 14. Evaluation of anti-tumor activity of anti-EGFR antibody-drug conjugate in NCG mouse model having humanized PBMC immune system in mice and subcutaneously allografted with human colon cancer HT-29 cells**

HT-29 cells were cultured in a growth culture medium (McCoy's 5A + 10% FBS). Human PBMCs were inoculated into NCG mice to complete the reconstitution of the immune system in the mice. After 3 weeks, HT-29 cells in the logarithmic growth phase were collected and resuspended in PBS and Matrixgel matrix for subcutaneous inoculation in mice. The mice to be inoculated were 6-8 weeks old, which were inoculated subcutaneously on the right back with HT-29 cells ($3 \times 10^6$/mouse). Once the tumor had grown to an average volume of 140.5 $mm^3$, the mice were randomly grouped depending on the tumor size and dosed, ensuring similar tumor volumes between groups. The day of grouping was defined as day 0. After the start of dosing, the body weight and tumor size of the mice were measured twice a week, and the mice were dosed once a week, for three doses. Clinical symptoms observed during the experiment were recorded. The tumor volume was calculated according to the following formula: tumor volume ($mm^3$) = $1/2 \times (a \times b^2)$ (wherein a represented the long diameter, and b represented the short diameter).

As shown in FIG. 10, on day 17 post-dose, compared with the control group (PBS), MAB07-LP62 (DAR8) (at both 1 mg/kg and 3 mg/kg) showed significant tumor growth inhibition and dose-dependent anti-tumor effects, with TGI% of 49.3% ($p = 0.0017$) and 94.6% ($p < 0.001$), respectively. In addition, a group dosed with a combination of 10 mg/kg of serplulimab + 1 mg/kg MAB07-LP62 (DAR8) showed superior tumor growth inhibition than 10 mg/kg serplulimab or 1 mg/kg MAB07-LP62 (DAR8), with TGI % of 82.1%, 18.5%, and 49.3%, respectively. The combination of serplulimab and MAB07-LP62 (DAR8) was shown to have significant synergistic effect. None of the animals experienced significant body weight loss throughout the treatment period.

**Example 15. Evaluation of anti-tumor effect of anti-EGFR antibody-drug conjugate in human head and neck squamous carcinoma LD1-2023-411020 PDX xenograft NCG mouse model**

A human head and neck squamous carcinoma PDX model was revived in NCG mice. Once the tumor had grown to 500-800 $mm^3$, the tumor tissue was stripped and evenly cut into about 3 mm $\times$ 3 mm $\times$ 3 mm tissue blocks, and then, the NCG mice were inoculated subcutaneously on the right back with the small tumor tissue blocks. Once the tumor had grown to an average volume of about 138.88 $mm^3$, the mice were randomly grouped depending on the tumor size and dosed, ensuring similar tumor volumes between groups. The day of grouping was defined as day 0. After the start of dosing, the body weight and tumor size of the mice were measured twice a week, and the mice were dosed once a week, for three doses. The tumor volume was calculated according to the following formula: tumor volume ($mm^3$) = $1/2 \times (a \times b^2)$ (wherein a represented the long diameter, and b represented the short diameter). As shown in FIG. 11, on day 21, MAB07-LP62 (DAR8) exhibited a stronger tumor inhibitory effect at the 8 mg/kg dose, with a TGI of 121.62% ($p < 0.001$). MAB07-LP62 (DAR8) was shown to have a very strong tumor inhibitory effect. None of the animals experienced significant body weight loss throughout the treatment period.

**Example 16. Evaluation of anti-tumor effect of anti-EGFR antibody-drug conjugate in human gastric cancer LD1-0017-411335 PDX xenograft NU/NU mouse model**

A DS-8201 (fam-trastuzumab deruxtecan-nxki, trade name ENHERTU®)-resistant human gastric cancer PDX model was revived in NU/NU mice. Once the tumor had grown to 500-800 $mm^3$, the tumor tissue was stripped and evenly cut into about 3 mm $\times$ 3 mm $\times$ 3 mm tissue blocks, and then, the NU/NU mice were inoculated subcutaneously on the right back with the small tumor tissue blocks. Once the tumor had grown to an average volume of about 176.58 $mm^3$, the mice were randomly grouped depending on the tumor size and dosed, ensuring similar tumor volumes between groups. The day of grouping was defined as day 0. After the start of dosing, the body weight and tumor size of the mice were measured twice a week, and the mice were dosed once a week, for four doses. Clinical symptoms observed during the experiment were recorded. The tumor volume was calculated according to the following formula: tumor volume ($mm^3$) = $1/2 \times (a \times b^2)$ (wherein a represented the long diameter, and b represented the short diameter).

As shown in FIG. 12, on day 28, MAB07-LP62 (DAR8) exhibited stronger tumor inhibitory effects at both 3 mg/kg and 8 mg/kg doses, with TGIs of 87.04% ($p = 0.0003$) and 98.01% ($p < 0.0001$), respectively. In contrast, the naked anti-MAB07 exhibited no significant anti-tumor activity at 8 mg/kg, with a TGI of 27.59% ($p = 0.1976$); whereas the free loaded drug A1.3 showed a TGI of 30.68%. MAB07-LP62 (DAR8) was shown to have a very strong tumor inhibitory effect. None of the animals experienced significant body weight loss throughout the treatment period.

**Example 17. Evaluation of anti-tumor effect of anti-EGFR antibody-drug conjugate in NOD/SCID mouse model subcutaneously xenografted with human esophageal cancer KYSE-150 cell line**

KYSE-150 cells were cultured in a growth culture medium (RPMI 1640/F12 + 2% FBS). Cells in the exponential growth phase were collected and resuspended in PBS and Matrixgel matrix for subcutaneous inoculation in mice. The mice to be inoculated were 7-8 weeks old, which were inoculated subcutaneously on the right back with KYSE-150 cells ($1 \times 10^7$/mouse). Once the tumor had grown to an average volume of 199.66 $mm^3$, the mice were randomly grouped depending on the tumor size and dosed, ensuring similar tumor volumes between groups. The day of grouping was defined as day 0. After the start of dosing, the body weight and tumor size of the mice were measured twice a week, and the mice were dosed once a week, for three doses. The tumor volume was calculated according to the following formula: tumor volume ($mm^3$) = $1/2 \times (a \times b^2)$ (wherein a represented the long diameter, and b represented the short diameter).

As shown in FIG. 13, on day 21, MAB07-LP62 (DAR8) exhibited dose-dependent tumor inhibitory effects in the 1 mg/kg, 3 mg/kg, and 8 mg/kg dose groups, with TGIs of 28.6% ($p = 0.482$), 94.5% ($p < 0.001$), and 97.9% ($p < 0.001$), respectively. However, the naked anti-MAB07 exhibited only partial anti-tumor activity at 8 mg/kg, with a TGI of 66.4% ($p < 0.001$). MAB07-LP62 (DAR8) was shown to have a very strong tumor inhibitory effect. None of the animals experienced significant body weight loss throughout the treatment period.

In addition to the depicted and claimed various embodiments, the disclosed subject matter is also directed to other embodiments having other combinations of the features disclosed and claimed herein. As such, the particular features presented herein can be combined with each other in other manners within the scope of the disclosed subject matter such that the disclosed subject matter includes any suitable combination of the features disclosed herein. The foregoing description of specific embodiments of the disclosed subject matter has been presented for purposes of illustration and description. The above description is not intended to be exhaustive or to limit the disclosed subject matter to those embodiments disclosed.

It will be apparent to those skilled in the art that various modifications and variations can be made in the compositions and methods of the disclosed subject matter without departing from the spirit or scope of the disclosed subject matter. Thus, it is intended that the disclosed subject matter include modifications and variations that are within the scope of the appended claims and their equivalents.

Various publications, patents and patent applications are cited herein, the contents of which are hereby incorporated by reference in their entireties.

## Claims

1. An antibody-drug conjugate, a prodrug, pharmaceutically acceptable salt, or solvate thereof, or a solvate of the pharmaceutically acceptable salt, **characterized in that** the antibody-drug conjugate has a structure represented by formula (I):

$$TP\text{-}[L1\text{-}L2\text{-}L3\text{-}D]_k \qquad (I)$$

wherein:

TP is a targeting moiety selectively binding or recognizing EGFR;
L1 is an extension unit connecting TP and L2;
L2 is an optional amino acid residue or a short peptide consisting of 2-10 amino acid residues;
L3 is a spacer element;
D is a biologically active molecule; and
k represents any numerical value between 0.1 and 10.0.

2. The antibody-drug conjugate, the prodrug, pharmaceutically acceptable salt, or solvate thereof, or the solvate of the pharmaceutically acceptable salt according to claim 1, **characterized in that** the TP is an antibody or an antigen-binding fragment thereof.

3. The antibody-drug conjugate, the prodrug, pharmaceutically acceptable salt, or solvate thereof, or the solvate of the pharmaceutically acceptable salt according to claim 2, **characterized in that** the antigen-binding fragment is selected

from a full-length immunoglobulin, a single-chain Fv (scFv) fragment, an Fab fragment, an Fab' fragment, $F(ab')_2$, an Fv fragment, a disulfide-stabilized Fv fragment (dsFv), $(dsFv)_2$, an Fv-Fc fusion, an scFv-Fc fusion, an scFv-Fv fusion, a diabody, a tribody, a tetrabody, or any combination thereof.

4. The antibody-drug conjugate, the prodrug, pharmaceutically acceptable salt, or solvate thereof, or the solvate of the pharmaceutically acceptable salt according to any one of the preceding claims, **characterized in that** the TP is a humanized antibody or an antigen-binding fragment thereof.

5. The antibody-drug conjugate, the prodrug, pharmaceutically acceptable salt, or solvate thereof, or the solvate of the pharmaceutically acceptable salt according to any one of the preceding claims, **characterized in that** the TP comprises a heavy chain variable region (VH) and a light chain variable region (VL), wherein the heavy chain variable region (VH) comprises heavy chain complementarity determining region 1 (HCDR1), HCDR2, and HCDR3 contained in SEQ ID NO: 7, and the light chain variable region (VL) comprises light chain complementarity determining region 1 (LCDR1), LCDR2, and LCDR3 contained in SEQ ID NO: 8.

6. The antibody-drug conjugate, the prodrug, pharmaceutically acceptable salt, or solvate thereof, or the solvate of the pharmaceutically acceptable salt according to claim 5, **characterized in that** the amino acid sequence of HCDR1 is SEQ ID NO: 1, the amino acid sequence of HCDR2 is SEQ ID NO: 2, and the amino acid sequence of HCDR3 is SEQ ID NO: 3; and the amino acid sequence of LCDR1 is SEQ ID NO: 4, the amino acid sequence of LCDR2 is SEQ ID NO: 5, and the amino acid sequence of LCDR3 is SEQ ID NO: 6.

7. The antibody-drug conjugate, the prodrug, pharmaceutically acceptable salt, or solvate thereof, or the solvate of the pharmaceutically acceptable salt according to any one of the preceding claims, **characterized in that** the TP comprises an Fc sequence of human IgG.

8. The antibody-drug conjugate, the prodrug, pharmaceutically acceptable salt, or solvate thereof, or the solvate of the pharmaceutically acceptable salt according to any one of the preceding claims, **characterized in that** the IgG is selected from IgG1, IgG2, IgG3, and IgG4.

9. The antibody-drug conjugate, the prodrug, pharmaceutically acceptable salt, or solvate thereof, or the solvate of the pharmaceutically acceptable salt according to any one of the preceding claims, **characterized in that** the TP comprises a heavy chain variable region (VH), and the amino acid sequence of the heavy chain variable region (VH) has at least 70%, at least 80%, at least 90%, at least 95%, at least 99%, or 100% identity to SEQ ID NO: 7.

10. The antibody-drug conjugate, the prodrug, pharmaceutically acceptable salt, or solvate thereof, or the solvate of the pharmaceutically acceptable salt according to any one of the preceding claims, **characterized in that** the TP comprises a light chain variable region (VL), and the amino acid sequence of the light chain variable region (VL) has at least 70%, at least 80%, at least 90%, at least 95%, at least 99%, or 100% identity to SEQ ID NO: 8.

11. The antibody-drug conjugate, the prodrug, pharmaceutically acceptable salt, or solvate thereof, or the solvate of the pharmaceutically acceptable salt according to any one of the preceding claims, **characterized in that** the TP comprises a heavy chain (HC), and the amino acid sequence of the heavy chain (HC) has at least 70%, at least 80%, at least 90%, at least 95%, at least 99%, or 100% identity to SEQ ID NO: 9.

12. The antibody-drug conjugate, the prodrug, pharmaceutically acceptable salt, or solvate thereof, or the solvate of the pharmaceutically acceptable salt according to any one of the preceding claims, **characterized in that** the TP comprises a light chain (LC), and the amino acid sequence of the light chain (LC) has at least 70%, at least 80%, at least 90%, at least 95%, at least 99%, or 100% identity to SEQ ID NO: 10.

13. The antibody-drug conjugate, the prodrug, pharmaceutically acceptable salt, or solvate thereof, or the solvate of the pharmaceutically acceptable salt according to any one of the preceding claims, **characterized in that** the TP is a multispecific antibody.

14. The antibody-drug conjugate, the prodrug, pharmaceutically acceptable salt, or solvate thereof, or the solvate of the pharmaceutically acceptable salt according to any one of the preceding claims, **characterized in that** the TP is an afucosylated antibody.

15. The antibody-drug conjugate, the prodrug, pharmaceutically acceptable salt, or solvate thereof, or the solvate of the

pharmaceutically acceptable salt according to any one of the preceding claims, **characterized in that** the Fc region comprises a C-terminal lysine.

**16.** The antibody-drug conjugate, the prodrug, pharmaceutically acceptable salt, or solvate thereof, or the solvate of the pharmaceutically acceptable salt according to any one of the preceding claims, **characterized in that** the Fc region comprises a deletion of the C-terminal lysine.

**17.** The antibody-drug conjugate, the prodrug, pharmaceutically acceptable salt, or solvate thereof, or the solvate of the pharmaceutically acceptable salt according to any one of the preceding claims, **characterized in that** the L1 is selected from

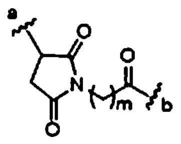
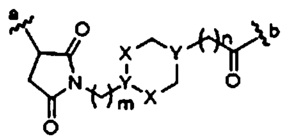
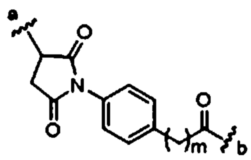

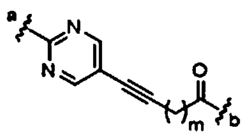
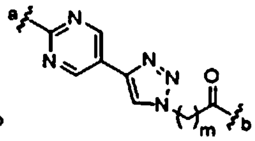
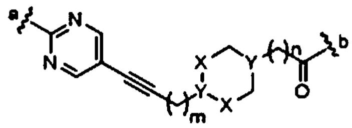

wherein L1 is connected to TP at position a via an S atom and to L2 at position b, m is any integer between 1 and 10, n is any integer between 1 and 10, and X and Y are each independently C, O, S, or N.

**18.** The antibody-drug conjugate, the prodrug, pharmaceutically acceptable salt, or solvate thereof, or the solvate of the pharmaceutically acceptable salt according to any one of the preceding claims, **characterized in that** the L 1 is

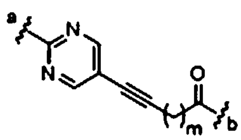

wherein L1 is connected to TP at position a via an S atom and to L2 at position b, and m is selected from 1, 2, 3, 4, or 5.

**19.** The antibody-drug conjugate, the prodrug, pharmaceutically acceptable salt, or solvate thereof, or the solvate of the pharmaceutically acceptable salt according to any one of the preceding claims, **characterized in that** the L1 is

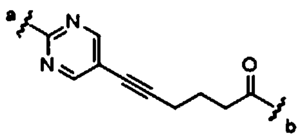

wherein L1 is connected to TP at position a via an S atom and to L2 at position b.

**20.** The antibody-drug conjugate, the prodrug, pharmaceutically acceptable salt, or solvate thereof, or the solvate of the pharmaceutically acceptable salt according to any one of the preceding claims, **characterized in that** the L2 is selected from

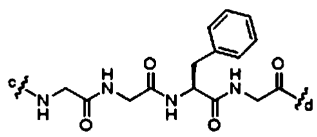
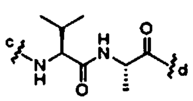
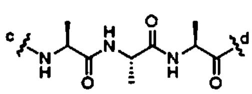

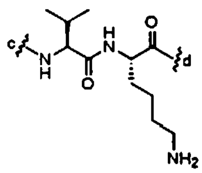
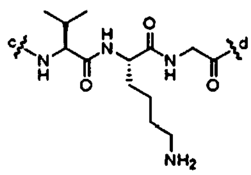
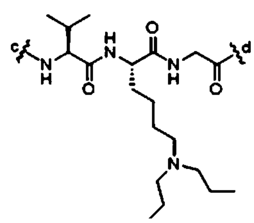

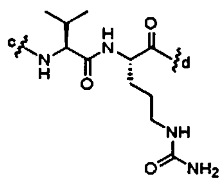
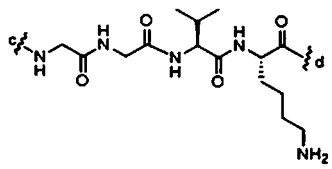
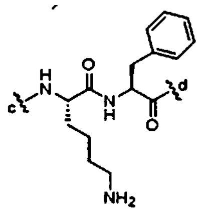

wherein L2 is connected to L1 at position c and to L3 at position d.

**21.** The antibody-drug conjugate, the prodrug, pharmaceutically acceptable salt, or solvate thereof, or the solvate of the pharmaceutically acceptable salt according to any one of the preceding claims, **characterized in that** the L2 is

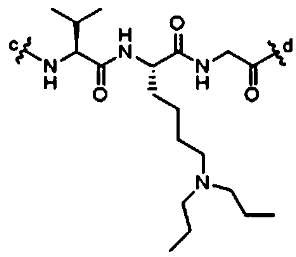

wherein L2 is connected to L1 at position c and to L3 at position d.

**22.** The antibody-drug conjugate, the prodrug, pharmaceutically acceptable salt, or solvate thereof, or the solvate of the pharmaceutically acceptable salt according to any one of the preceding claims, **characterized in that** the L3 is a single bond or a group selected from:

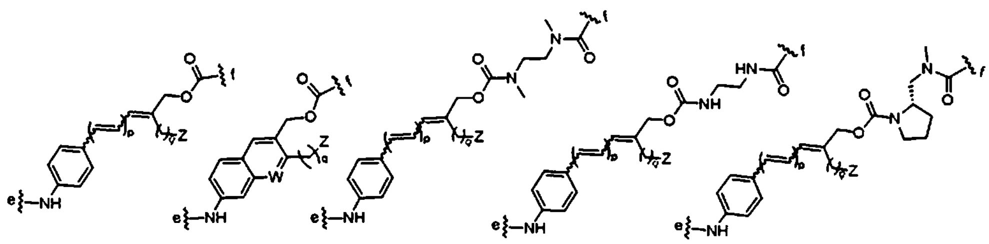

wherein L3 is connected to L2 at position e and to D at position f,
p and q are each independently an integer between 0 and 10,
W is C, O, S, or N, and
Z is selected from hydrogen, halogen, $C_1$-$C_{20}$ alkane, $C_1$-$C_{20}$ haloalkane, cyano, sulfonic acid, carboxylic acid, $C_1$-$C_{20}$ alkoxy, $C_2$-$C_{10}$ unsaturated alkane, a natural amino acid, an unnatural amino acid, and a short peptide consisting of the above amino acids.

**23.** The antibody-drug conjugate, the prodrug, pharmaceutically acceptable salt, or solvate thereof, or the solvate of the pharmaceutically acceptable salt according to any one of the preceding claims, **characterized in that** Z is selected from:

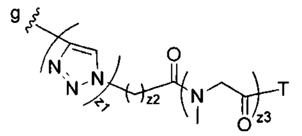
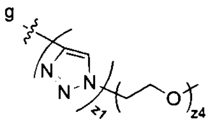
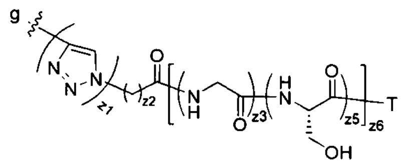
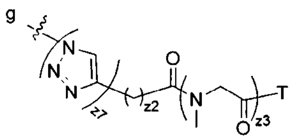
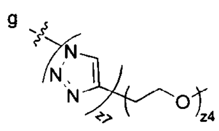
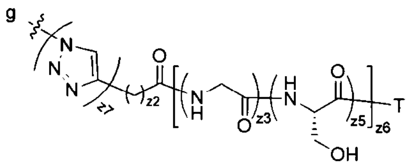
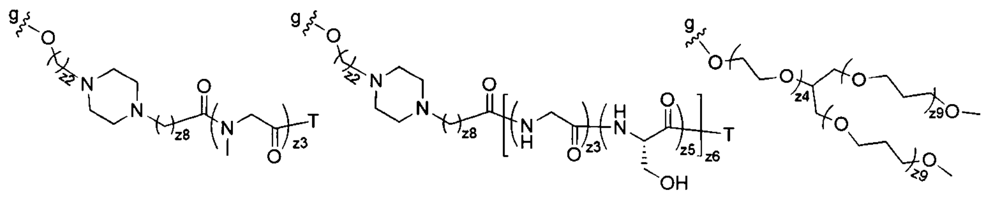

wherein Z is connected to the remainder of L3 at position g, z1-z9 are each independently an integer between 0 and 10, and T is $NH_2$ or OH.

**24.** The antibody-drug conjugate, the prodrug, pharmaceutically acceptable salt, or solvate thereof, or the solvate of the pharmaceutically acceptable salt according to any one of the preceding claims, **characterized in that** D is a cytotoxic agent.

**25.** The antibody-drug conjugate, the prodrug, pharmaceutically acceptable salt, or solvate thereof, or the solvate of the pharmaceutically acceptable salt according to any one of the preceding claims, **characterized in that** the D is selected from

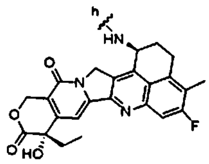
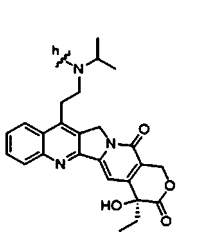
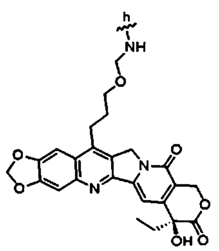
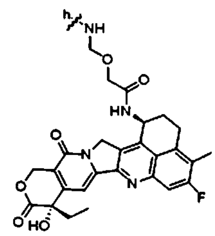

wherein D is connected to L3 at position h.

**26.** The antibody-drug conjugate, the prodrug, pharmaceutically acceptable salt, or solvate thereof, or the solvate of the pharmaceutically acceptable salt according to any one of the preceding claims, **characterized in that** the antibody-drug conjugate is selected from:

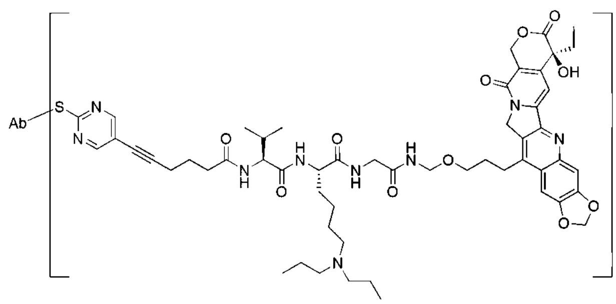
**(A-1)**

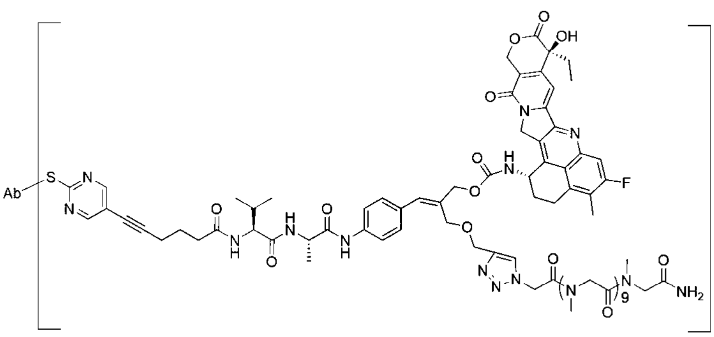

(A-2)

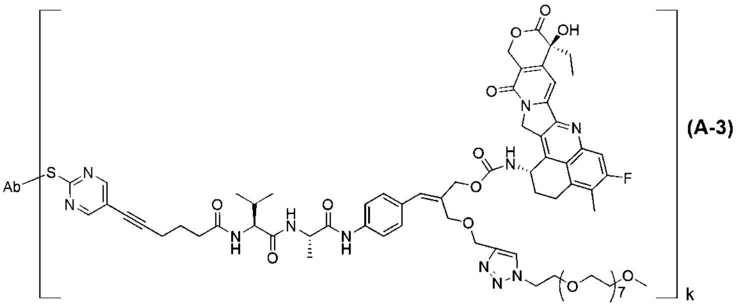

(A-3)

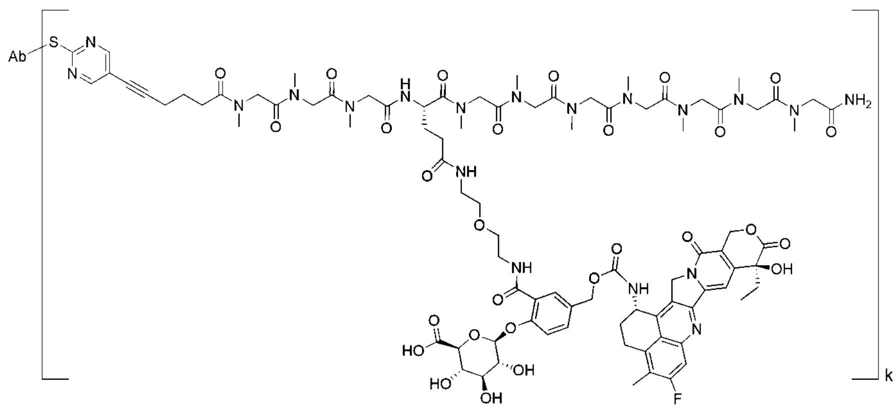

(A-4)

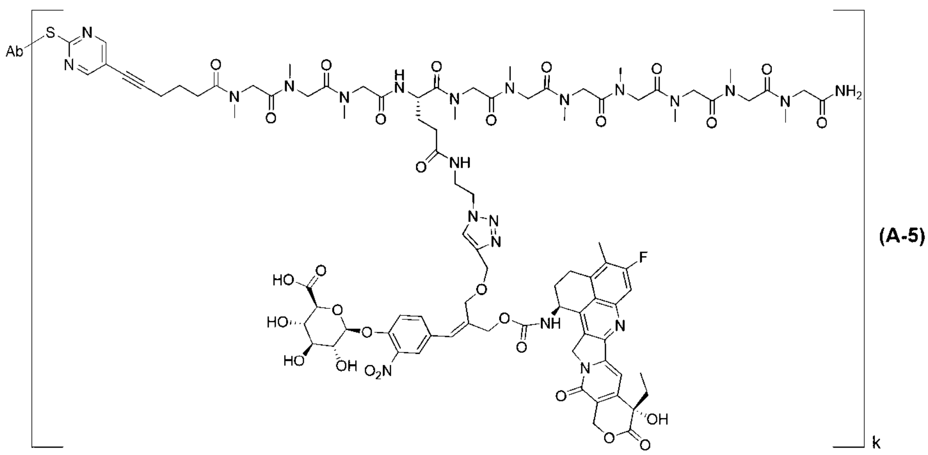

(A-5)

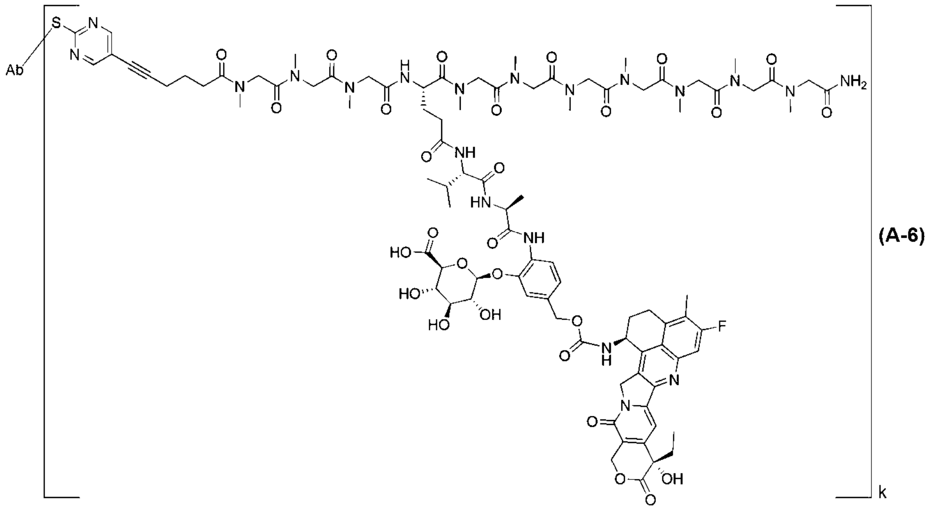

**(A-6)**

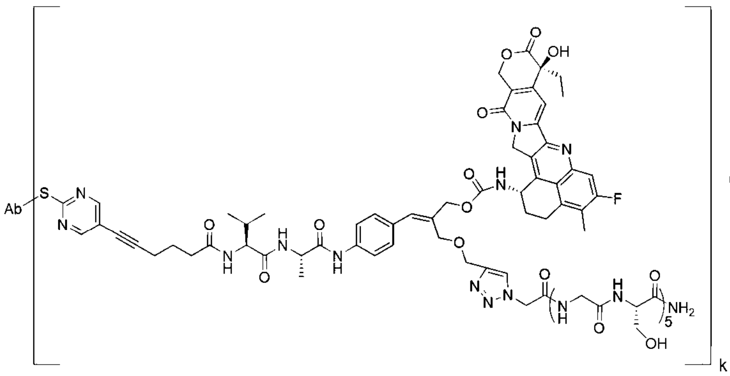

**(A-7)**

wherein Ab represents the antibody or the antigen-binding fragment thereof.

**27.** The antibody-drug conjugate, the prodrug, pharmaceutically acceptable salt, or solvate thereof, or the solvate of the pharmaceutically acceptable salt according to any one of the preceding claims, **characterized in that** k is about 4.0 to about 8.0, preferably k is about 4.0, about 6.0, or about 8.0.

**28.** A pharmaceutical composition comprising the antibody-drug conjugate, the prodrug, pharmaceutically acceptable salt, or solvate thereof, or the solvate of the pharmaceutically acceptable salt according to any one of claims 1-27, and optionally, a pharmaceutically acceptable carrier, diluent, or excipient.

**29.** The antibody-drug conjugate, the prodrug, pharmaceutically acceptable salt, or solvate thereof, or the solvate of the pharmaceutically acceptable salt according to any one of claims 1-27 or the pharmaceutical composition according to claim 28, for use as a drug.

**30.** The antibody-drug conjugate, the prodrug, pharmaceutically acceptable salt, or solvate thereof, or the solvate of the pharmaceutically acceptable salt according to any one of claims 1-27 or the pharmaceutical composition according to claim 28, for use in the treatment or prevention of a tumor or cancer.

**31.** The antibody-drug conjugate, the prodrug, pharmaceutically acceptable salt, or solvate thereof, or the solvate of the pharmaceutically acceptable salt, or the pharmaceutical composition for use in the treatment or prevention of a tumor or cancer according to claim 30, **characterized in that** the tumor or cancer is an EGFR-related tumor or cancer.

**32.** The antibody-drug conjugate, the prodrug, pharmaceutically acceptable salt, or solvate thereof, or the solvate of the pharmaceutically acceptable salt, or the pharmaceutical composition for use in the treatment or prevention of a tumor or cancer according to claim 30, **characterized in that** the tumor or cancer is selected from epidermal cancer, colon cancer, rectal cancer, head and neck cancer, lung cancer, ovarian cancer, cervical cancer, bladder cancer, esophageal cancer, breast cancer, kidney cancer, prostate cancer, gastric cancer, pancreatic cancer, and glioma.

**33.** The antibody-drug conjugate, the prodrug, pharmaceutically acceptable salt, or solvate thereof, or the solvate of the pharmaceutically acceptable salt, or the pharmaceutical composition for use in the treatment or prevention of a tumor or cancer according to claim 32, **characterized in that** the lung cancer is small cell lung cancer or non-small cell lung cancer.

**34.** The antibody-drug conjugate, the prodrug, pharmaceutically acceptable salt, or solvate thereof, or the solvate of the pharmaceutically acceptable salt, or the pharmaceutical composition for use in the treatment or prevention of a tumor or cancer according to claim 32, **characterized in that** the lung cancer is osimertinib-resistant lung cancer.

**35.** The antibody-drug conjugate, the prodrug, pharmaceutically acceptable salt, or solvate thereof, or the solvate of the pharmaceutically acceptable salt, or the pharmaceutical composition for use in the treatment or prevention of a tumor or cancer according to claim 32, **characterized in that** the gastric cancer is ENHERTU-resistant gastric cancer.

**36.** The antibody-drug conjugate, the prodrug, pharmaceutically acceptable salt, or solvate thereof, or the solvate of the pharmaceutically acceptable salt, or the pharmaceutical composition for use in the treatment or prevention of a tumor or cancer according to any one of claims 30-35, **characterized in that** the tumor or cancer is a tumor or cancer with a KRAS gene mutation and/or a BRAF gene mutation.

**37.** The antibody-drug conjugate, the prodrug, pharmaceutically acceptable salt, or solvate thereof, or the solvate of the pharmaceutically acceptable salt, or the pharmaceutical composition for use in the treatment or prevention of a tumor or cancer according to claim 36, **characterized in that** the tumor or cancer with the KRAS gene mutation and/or the BRAF gene mutation is colon cancer, rectal cancer, lung cancer, or pancreatic cancer with the KRAS gene mutation and/or the BRAF gene mutation.

**38.** An article of manufacture or kit comprising the antibody-drug conjugate, the prodrug, pharmaceutically acceptable salt, or solvate thereof, or the solvate of the pharmaceutically acceptable salt according to any one of claims 1-27 or the pharmaceutical composition according to claim 28, and instructions for use.

**39.** A method for preventing or treating a tumor or cancer, **characterized by** comprising administering to a subject in need thereof a prophylactically or therapeutically effective amount of the antibody-drug conjugate, the prodrug, pharmaceutically acceptable salt, or solvate thereof, or the solvate of the pharmaceutically acceptable salt according to any one of claims 1-27 or the pharmaceutical composition according to claim 28.

**40.** The method according to claim 39, **characterized in that** the tumor or cancer is an EGFR-related tumor or cancer.

**41.** The method according to claim 39, **characterized in that** the tumor or cancer is selected from epidermal cancer, colon cancer, rectal cancer, head and neck cancer, lung cancer, ovarian cancer, cervical cancer, bladder cancer, esophageal cancer, breast cancer, kidney cancer, prostate cancer, gastric cancer, pancreatic cancer, and glioma.

**42.** The method according to claim 41, **characterized in that** the lung cancer is small cell lung cancer or non-small cell lung cancer.

**43.** The method according to claim 41, **characterized in that** the lung cancer is osimertinib-resistant lung cancer.

**44.** The method according to claim 41, **characterized in that** the gastric cancer is ENHERTU-resistant gastric cancer.

**45.** The method according to any one of claims 39-44, **characterized in that** the tumor or cancer is a tumor or cancer with a KRAS gene mutation and/or a BRAF gene mutation.

**46.** The method according to claim 45, **characterized in that** the tumor or cancer with the KRAS gene mutation and/or the BRAF gene mutation is a colon cancer, rectal cancer, lung cancer, or pancreatic cancer with the KRAS gene mutation and/or the BRAF gene mutation.

**47.** The method according to any one of claims 39-46, **characterized in that** the method comprises administering to the subject an additional therapeutic agent selected from an anti-tumor agent, a chemotherapeutic agent, a growth inhibitor, and a cytotoxic agent.

**48.** The method according to claim 47, **characterized in that** the additional therapeutic agent is osimertinib.

**49.** The method according to claim 47, **characterized in that** the additional therapeutic agent is a TKI inhibitor or a PD1/PDL1 inhibitor.

**50.** The method according to claim 47, **characterized in that** the additional therapeutic agent is an anti-PD1 antibody.

**51.** The method according to claim 50, **characterized in that** the anti-PD1 antibody is serplulimab.

**52.** The method according to any one of claims 39-51, **characterized in that** the method comprises further administering to the subject a radiotherapy.

**53.** Use of the antibody-drug conjugate, the prodrug, pharmaceutically acceptable salt, or solvate thereof, or the solvate of the pharmaceutically acceptable salt according to any one of claims 1-27 or the pharmaceutical composition according to claim 28 in the preparation of a medicament for use in the treatment or prevention of a tumor or cancer.

**54.** Use of the antibody-drug conjugate, the prodrug, pharmaceutically acceptable salt, or solvate thereof, or the solvate of the pharmaceutically acceptable salt according to any one of claims 1-27 or the pharmaceutical composition according to claim 28 in combination with an additional therapeutic agent in the preparation of a medicament for treating or preventing an EGFR-related disease or disorder or an osimertinib-resistant tumor or cancer.

**55.** The use according to claim 53 or 54, **characterized in that** the additional therapeutic agent is osimertinib or an anti-PD1 antibody.

**56.** The use according to claim 55, **characterized in that** the anti-PD1 antibody is serplulimab.

**57.** A method for preventing or treating an osimertinib-resistant tumor or cancer, **characterized by** comprising administering to a subject in need thereof a prophylactically or therapeutically effective amount of the antibody-drug conjugate, the prodrug, pharmaceutically acceptable salt, or solvate thereof, or the solvate of the pharmaceutically acceptable salt according to any one of claims 1-27 or the pharmaceutical composition according to claim 28.

**58.** The method according to claim 57, **characterized in that** the tumor or cancer is selected from epidermal cancer, colon cancer, rectal cancer, head and neck cancer, lung cancer, ovarian cancer, cervical cancer, bladder cancer, esophageal cancer, breast cancer, kidney cancer, prostate cancer, gastric cancer, pancreatic cancer, and glioma.

**59.** The method according to claim 58, **characterized in that** the lung cancer is small cell lung cancer or non-small cell lung cancer.

**60.** The method according to any one of claims 57-59, **characterized in that** the method comprises administering to the subject an additional therapeutic agent selected from an anti-tumor agent, a chemotherapeutic agent, a growth inhibitor, and a cytotoxic agent.

**61.** The method according to claim 60, **characterized in that** the additional therapeutic agent is osimertinib.

**62.** The method according to claim 60, **characterized in that** the additional therapeutic agent is a TKI inhibitor or a PD1/PDL1 inhibitor.

**63.** The method according to claim 60, **characterized in that** the additional therapeutic agent is an anti-PD1 antibody.

**64.** The method according to claim 63, **characterized in that** the anti-PD1 antibody is serplulimab.

**65.** The method according to any one of claims 57-64, **characterized in that** the method comprises further administering to the subject a radiotherapy.

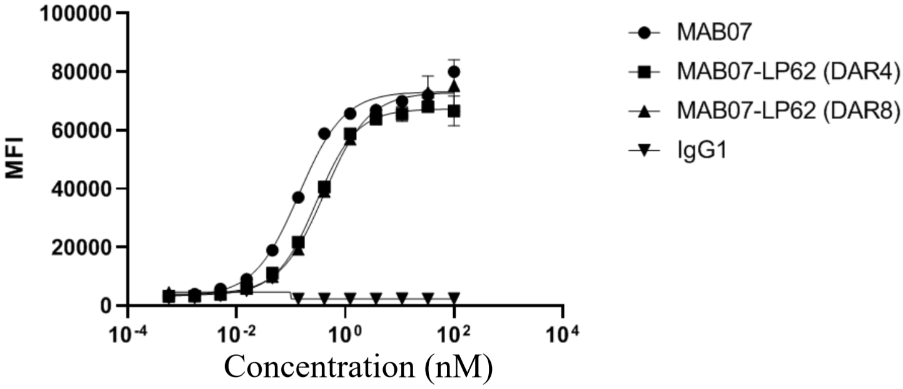


*FIG. 1A*

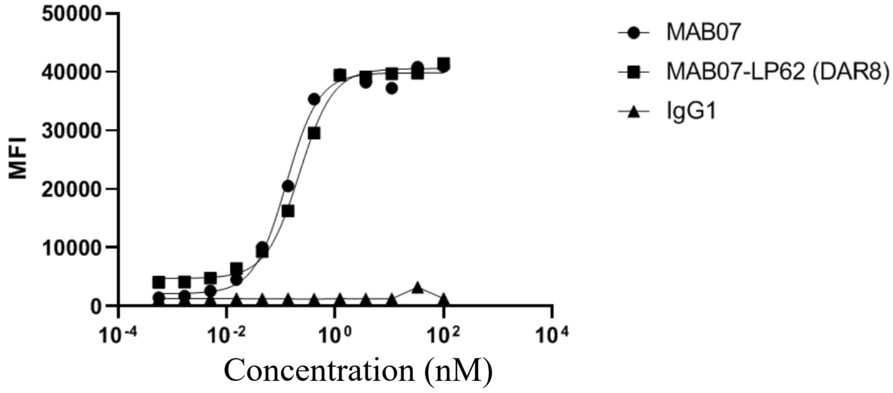


*FIG. 1B*

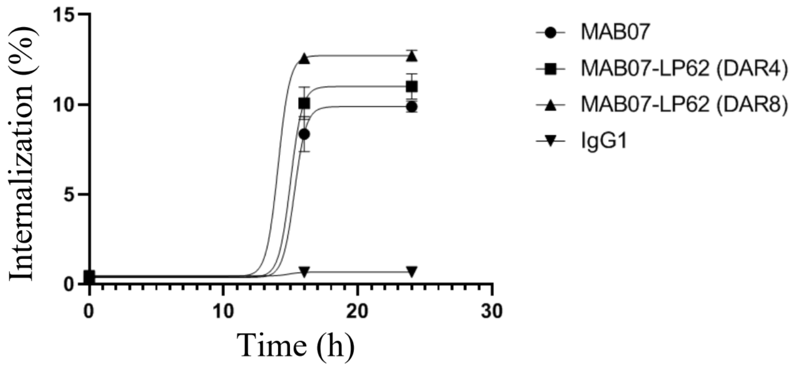


*FIG. 2*

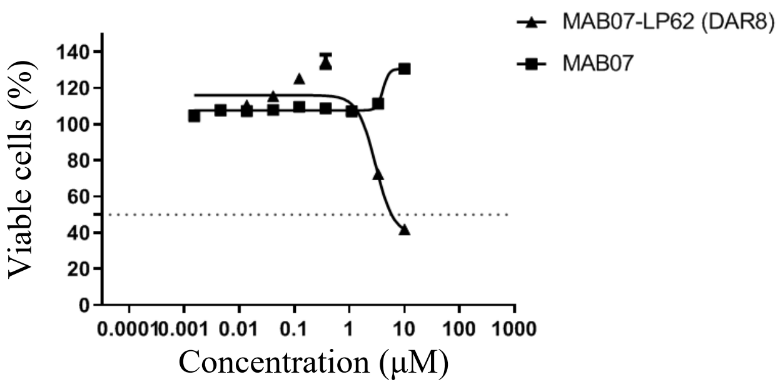


*FIG. 3A*

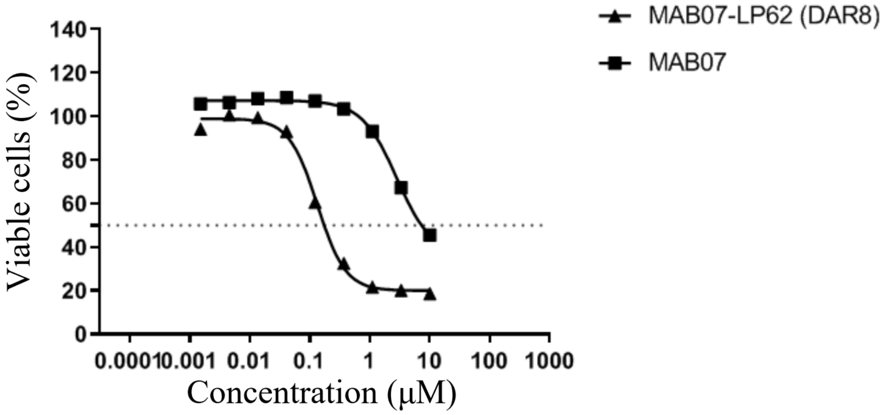


*FIG. 3B*

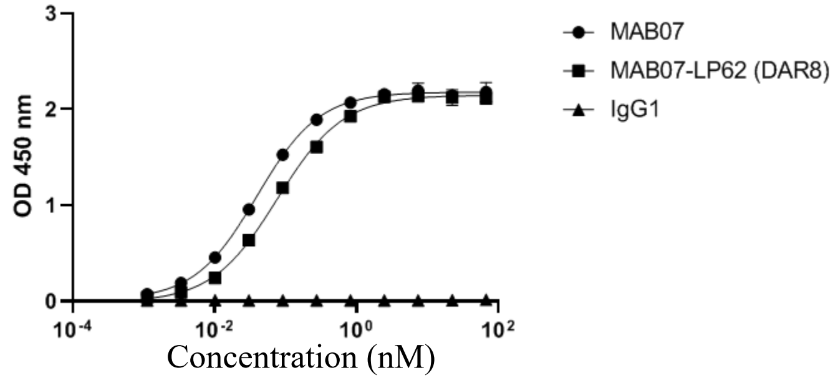


*FIG. 4A*

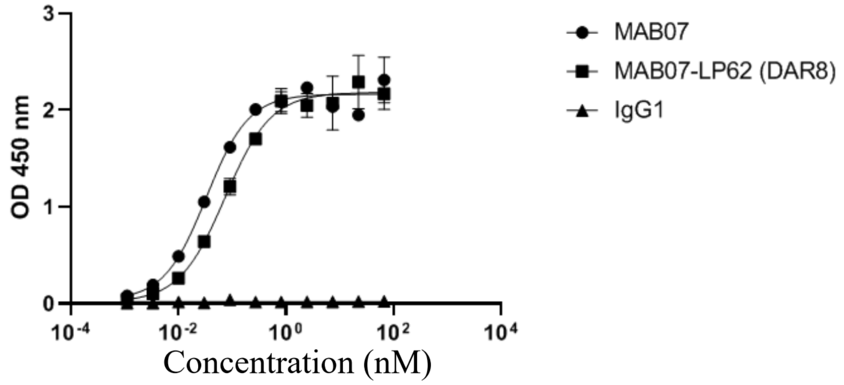


*FIG. 4B*

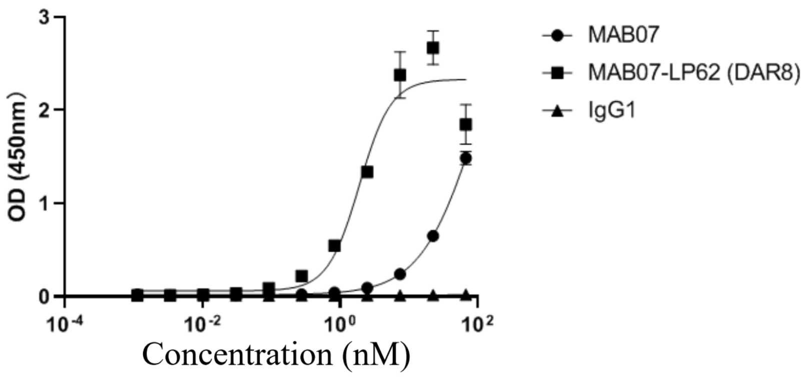


*FIG. 4C*

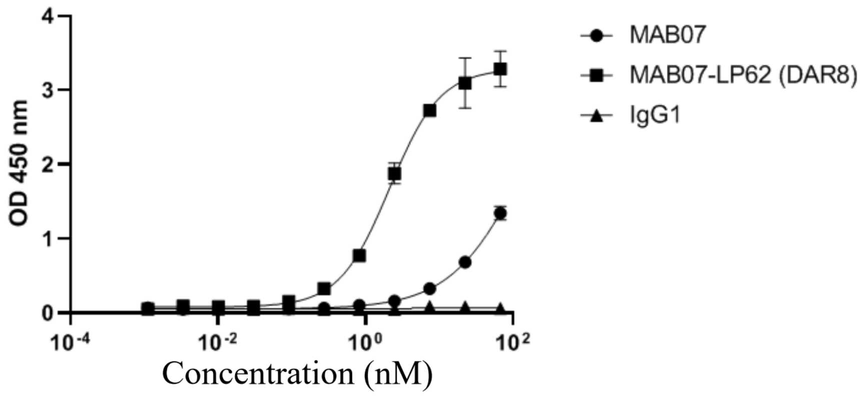


*FIG. 4D*

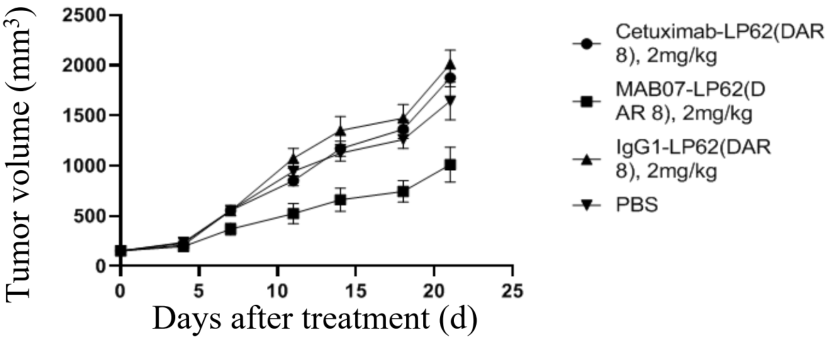


*FIG. 5*

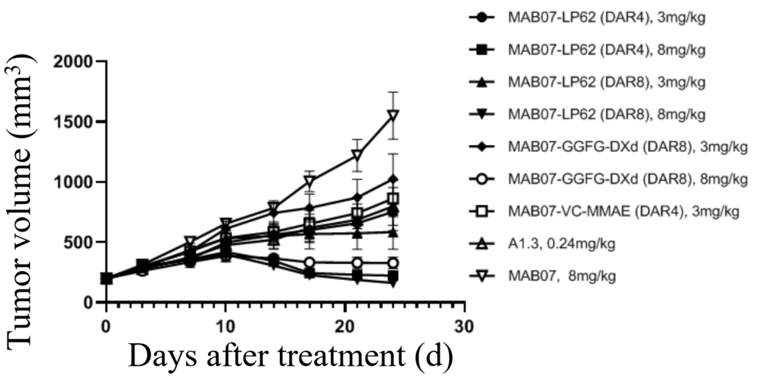


*FIG. 6*

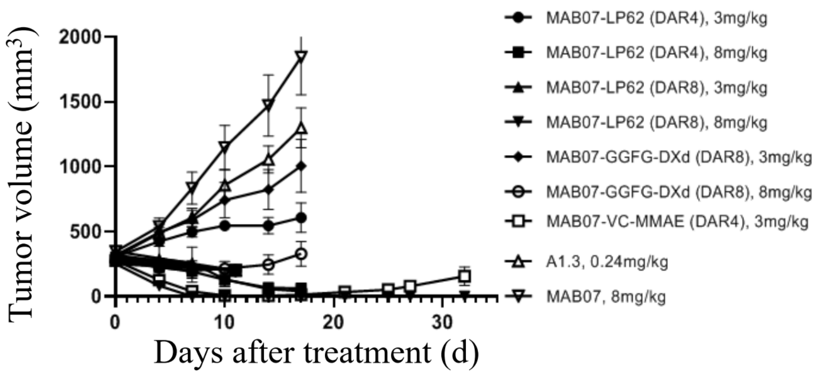


*FIG. 7*

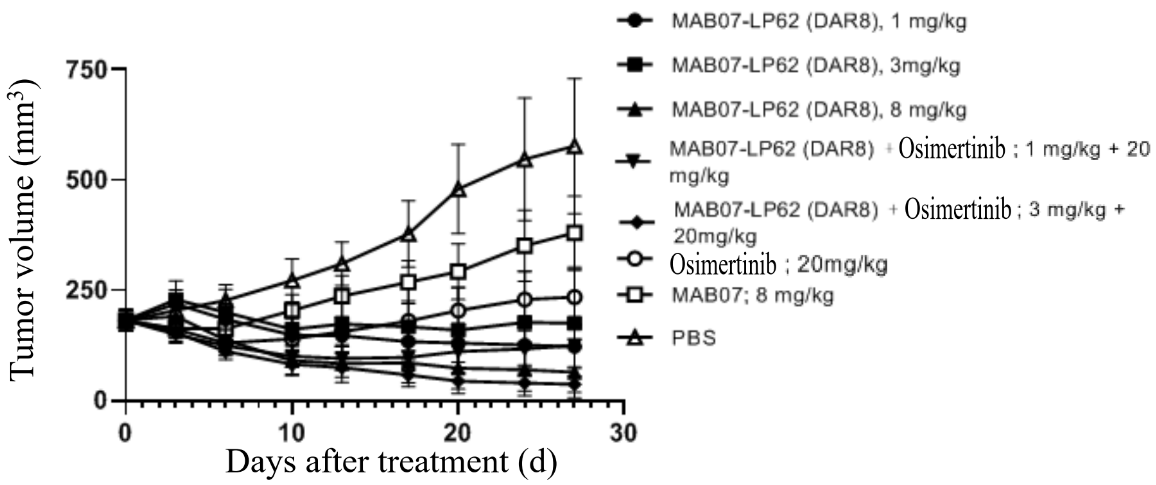


*FIG. 8*

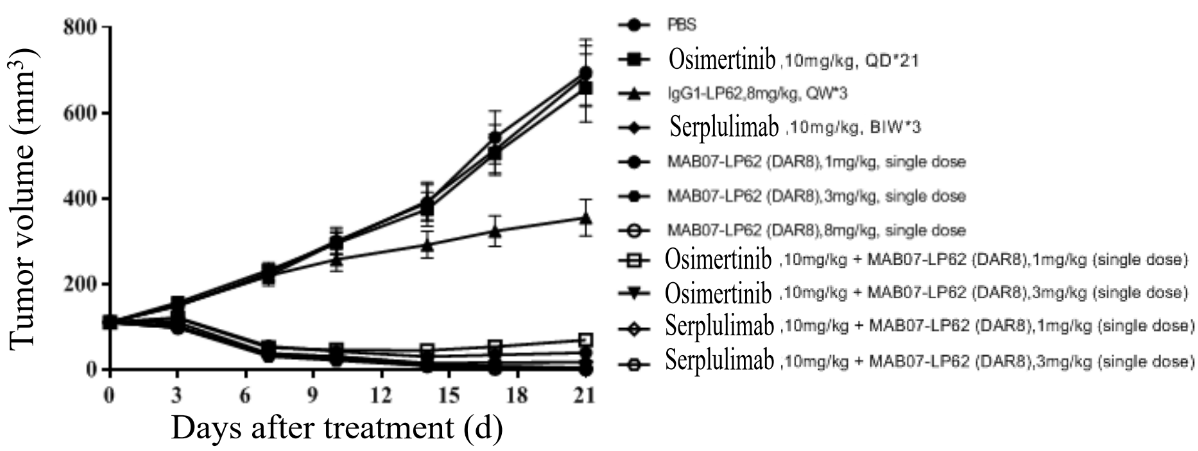


*FIG. 9*

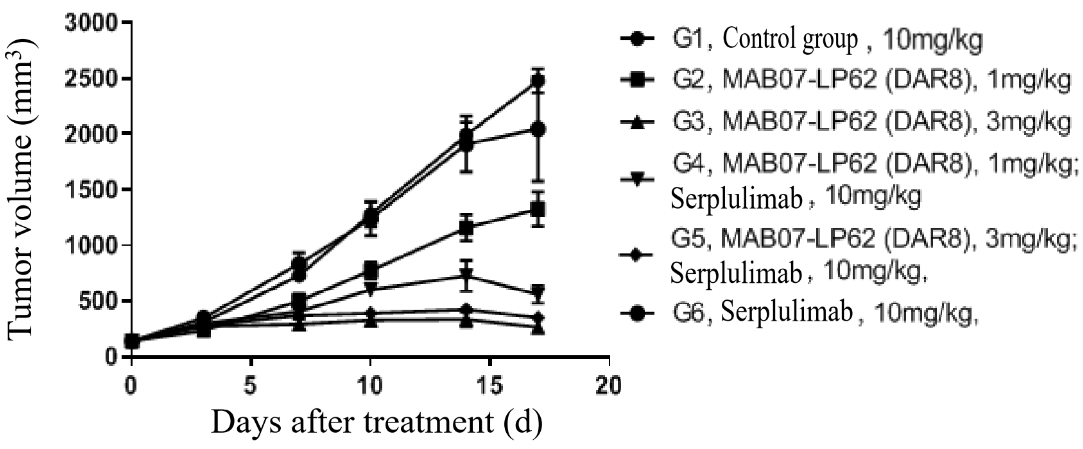


*FIG. 10*

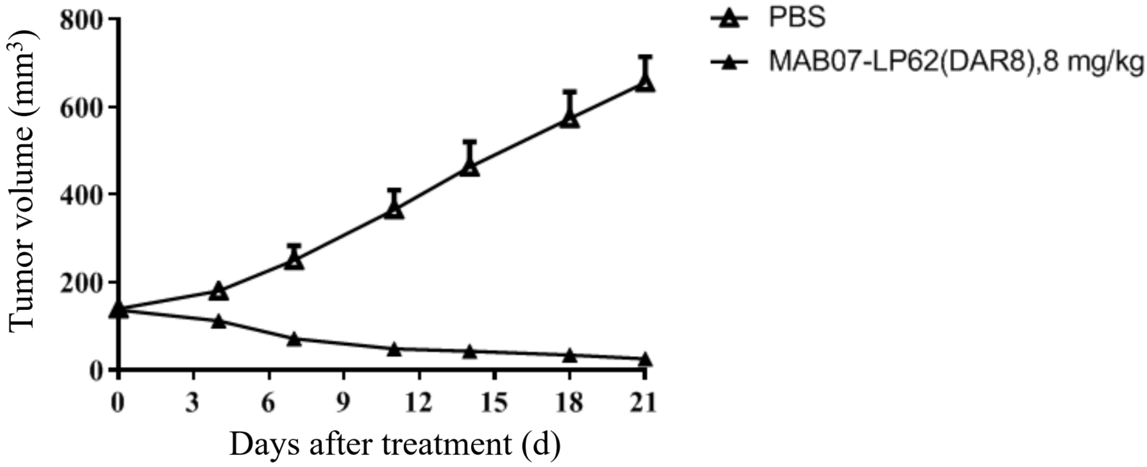


*FIG. 11*

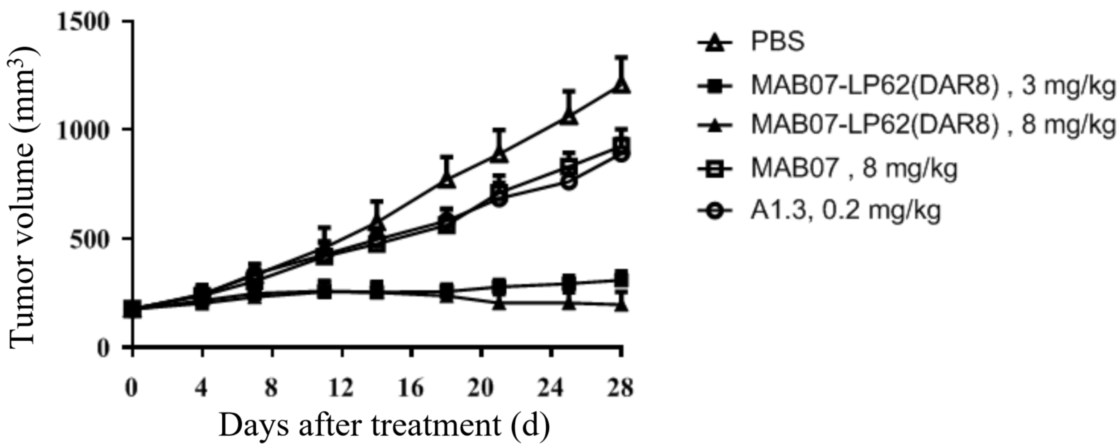


*FIG. 12*

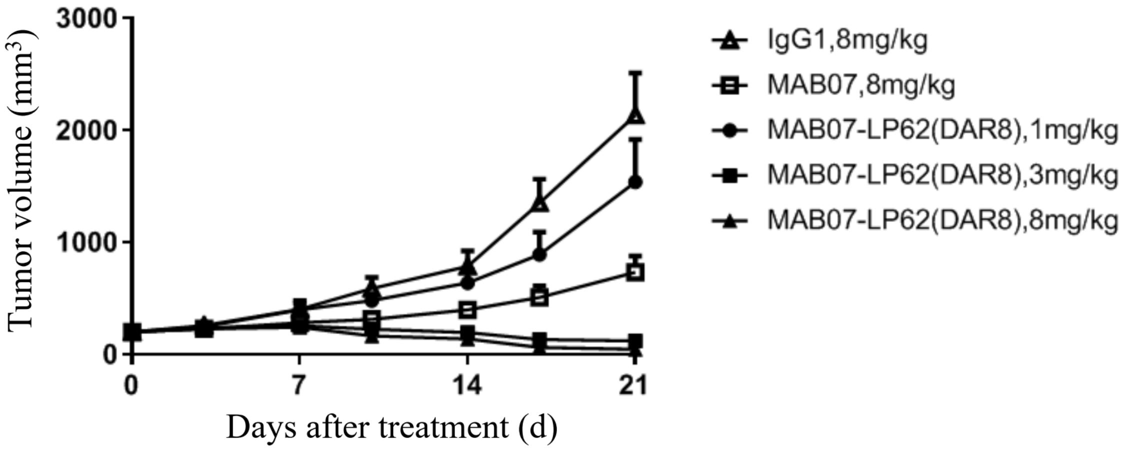


*FIG. 13*

**INTERNATIONAL SEARCH REPORT**

International application No.
**PCT/CN2024/091817**

**A. CLASSIFICATION OF SUBJECT MATTER**

A61K47/68(2017.01)i; C07D491/22(2006.01)i; C07K16/28(2006.01)i; A61P35/00(2006.01)i

According to International Patent Classification (IPC) or to both national classification and IPC

**B. FIELDS SEARCHED**

Minimum documentation searched (classification system followed by classification symbols)

A61K, C07D, C07K, A61P

Documentation searched other than minimum documentation to the extent that such documents are included in the fields searched

Electronic data base consulted during the international search (name of data base and, where practicable, search terms used)

CNABS, CNTXT, DWPI, VEN, ENTXTC, ENTXT, CNKI, PUBMED, ISI_Web of Science, Science Direct, STNext: 上海复宏汉霖生物技术股份有限公司, 缀合物, 偶联物, conjugate, EGFR, 喜树碱, SEQ ID NOs: 1-8, 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| X | WO 2022170971 A1 (MEDILINK THERAPEUTICS (SUZHOU) CO., LTD.) 18 August 2022 (2022-08-18)<br>abstract, claims 1-51, and description, pages 52 and 312-313 | 1-4, 7-8, 13-65 |
| Y | WO 2022170971 A1 (MEDILINK THERAPEUTICS (SUZHOU) CO., LTD.) 18 August 2022 (2022-08-18)<br>abstract, claims 1-51, and description, pages 52 and 312-313 | 5-65 |
| Y | CN 106714830 A (SHANGHAI HENLIUS BIOTECH CO., LTD.) 24 May 2017 (2017-05-24)<br>abstract, claims 1-30, and description, paragraphs 13, 17, 19-20, 41 and 146 | 5-65 |
| Y | WO 2020236817 A2 (NOVARTIS AG. et al.) 26 November 2020 (2020-11-26)<br>abstract, and claims 1-24 | 23-65 |
| Y | CN 113952450 A (SHANGHAI HENLIUS BIOTECH CO., LTD.) 21 January 2022 (2022-01-21)<br>abstract, and claims 1-10 | 5-65 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 August 2024** | **19 August 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/091817**

**C. DOCUMENTS CONSIDERED TO BE RELEVANT**

| Category* | Citation of document, with indication, where appropriate, of the relevant passages | Relevant to claim No. |
|---|---|---|
| A | WO 2023046202 A1 (SUZHOU SINOVENT PHARMACEUTICALS CO., LTD.) 30 March 2023 (2023-03-30)<br>abstract, and claims 1-28 | 1-65 |

Form PCT/ISA/210 (second sheet) (July 2022)

**INTERNATIONAL SEARCH REPORT**

International application No.
**PCT/CN2024/091817**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2024/091817**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **39-52 and 57-65**
because they relate to subject matter not required to be searched by this Authority, namely:
Claims 39-52 and 57-65 relate to the prevention or treatment of diseases, which falls within subject matter for which no search is required by the International Searching Authority as defined in PCT Rule 39.1(iv). A search is carried out on the basis that the designation of the subject matter thereof is a pharmaceutical use.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/091817**

| Patent document cited in search report | Publication date (day/month/year) | Patent family member(s) | Publication date (day/month/year) |
|---|---|---|---|
| WO 2022170971 A1 | 18 August 2022 | MX 2023008895 A | 09 August 2023 |
| | | CA 3206117 A1 | 18 August 2022 |
| | | KR 20230145038 A | 17 October 2023 |
| | | AU 2022220512 A1 | 13 July 2023 |
| | | JP 2024508081 A | 22 February 2024 |
| | | EP 4257154 A1 | 11 October 2023 |
| | | IL 304804 A | 01 September 2023 |
| | | US 2024108745 A1 | 04 April 2024 |
| | | TW 202241521 A | 01 November 2022 |
| CN 106714830 A | 24 May 2017 | SI 3148581 T1 | 28 February 2020 |
| | | WO 2015184403 A2 | 03 December 2015 |
| | | WO 2015184403 A3 | 21 January 2016 |
| | | SG 11201609959 UA | 29 December 2016 |
| | | CY 1122436 T1 | 27 January 2021 |
| | | EP 3148581 A2 | 05 April 2017 |
| | | EP 3148581 A4 | 01 November 2017 |
| | | EP 3148581 B1 | 09 October 2019 |
| | | ES 2761675 T3 | 20 May 2020 |
| | | US 2017218073 A1 | 03 August 2017 |
| | | US 10584171 B2 | 10 March 2020 |
| | | ES 2905777 T3 | 12 April 2022 |
| | | EP 3613433 A1 | 26 February 2020 |
| | | EP 3613433 B1 | 05 January 2022 |
| | | HRP 20192038 T1 | 07 February 2020 |
| | | HUE 047113 T2 | 28 April 2020 |
| | | KR 20170010855 A | 01 February 2017 |
| | | KR 102366644 B1 | 22 February 2022 |
| | | TW 201613971 A | 16 April 2016 |
| | | TWI 689520 B | 01 April 2020 |
| | | AU 2015266664 A1 | 08 December 2016 |
| | | AU 2015266664 B2 | 30 April 2020 |
| | | PT 3148581 T | 06 January 2020 |
| | | DK 3148581 T3 | 13 January 2020 |
| | | PL 3148581 T3 | 18 May 2020 |
| | | JP 2017521054 A | 03 August 2017 |
| | | JP 6691872 B2 | 13 May 2020 |
| | | US 2020223929 A1 | 16 July 2020 |
| | | US 11261255 B2 | 01 March 2022 |
| WO 2020236817 A2 | 26 November 2020 | BR 112021023229 A2 | 31 May 2022 |
| | | SG 11202112056 PA | 30 December 2021 |
| | | WO 2020236825 A2 | 26 November 2020 |
| | | WO 2020236825 A3 | 18 February 2021 |
| | | WO 2020236825 A8 | 18 March 2021 |
| | | US 2023081720 A1 | 16 March 2023 |
| | | DOP 2021000238 A | 31 March 2022 |
| | | EP 3972651 A2 | 30 March 2022 |
| | | CA 3138058 A1 | 26 November 2020 |
| | | KR 20220024106 A | 03 March 2022 |
| | | CA 3136088 A1 | 26 November 2020 |
| | | JP 2022532918 A | 20 July 2022 |

Form PCT/ISA/210 (patent family annex) (July 2022)

**INTERNATIONAL SEARCH REPORT**
**Information on patent family members**

International application No.
**PCT/CN2024/091817**

| Patent document cited in search report | Publication date (day/month/year) | Patent family member(s) | Publication date (day/month/year) |
|---|---|---|---|
| | | JP 2022533400 A | 22 July 2022 |
| | | MX 2021014094 A | 11 February 2022 |
| | | IL 288117 A | 01 January 2022 |
| | | US 2023092679 A1 | 23 March 2023 |
| | | AU 2020279979 A1 | 25 November 2021 |
| | | JOP 20210289 A1 | 30 January 2023 |
| | | TW 202100184 A | 01 January 2021 |
| | | CL 2021003042 A1 | 15 July 2022 |
| | | AR 122270 A1 | 31 August 2022 |
| | | PE 20220218 A1 | 02 February 2022 |
| | | WO 2020236817 A3 | 30 December 2020 |
| | | WO 2020236817 A8 | 20 May 2021 |
| | | IL 288110 A | 01 January 2022 |
| | | KR 20220017931 A | 14 February 2022 |
| | | UY 38700 A | 31 December 2020 |
| | | AU 2020279230 A1 | 02 December 2021 |
| | | CO 2021016552 A2 | 08 April 2022 |
| | | EP 3972649 A2 | 30 March 2022 |
| CN 113952450 A | 21 January 2022 | None | |
| WO 2023046202 A1 | 30 March 2023 | AU 2022350588 A1 | 02 May 2024 |
| | | KR 20240082348 A | 10 June 2024 |
| | | CA 3233254 A1 | 30 March 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202310519224
- CN 202311314455
- US 6075181 A
- US 6150584 A
- WO 8912624 A
- US 6214345 B
- US 5122368 A
- US 5824805 A
- US 5622929 A
- US 4880935 A
- US 19025968 W
- US 20050238649 A
- US 20060074008 A


### Non-patent literature cited in the description


- **MODJTAHEDI et al.** *Br. J. Cancer*, 1996, vol. 73, 228-235
- **HERBST** ; **SHIN**. *Cancer*, 2002, vol. 94, 1593-1611
- **ATALAY et al.** *Ann. Oncology*, 2003, vol. 14, 1346-1363
- **TSAO** ; **HERBST**. *Signal*, 2003, vol. 4, 4-9
- **KABAT et al.** *J. Biol. Chem.*, 1977, vol. 252, 6609-6616
- **KABAT et al.** Sequences of proteins of immunological interest. U.S. Department of Health and Human Services, 1991
- **CHOTHIA et al.** *J. Mol. Biol.*, 1987, vol. 196, 901-917
- **AL-LAZIKANI et al.** *J. Mol. Biol.*, 1997, vol. 273, 927-948
- **MACCALLUM et al.** *J. Mol. Biol.*, 1996, vol. 262, 732-745
- **ABHINANDAN** ; **MARTIN**. *Mol. Immunol.*, 2008, vol. 45, 3832-3839
- **LEFRANC M.P. et al.** *Dev. Comp. Immunol.*, 2003, vol. 27, 55-77
- **HONEGGER** ; **PLÜCKTHUN**. *J. Mol. Biol.*, 2001, vol. 309, 657-670
- **EHRENMANN F. et al.** *Nucleic Acids Res.*, 2010, vol. 38, D301-D307
- **ADOLF-BRYFOGLE J. et al.** *Nucleic Acids Res.*, 2015, vol. 43, D432-D438
- **JONES et al.** *Nature*, 1986, vol. 321, 522-525
- **RIECHMANN et al.** *Nature*, 1988, vol. 332, 323-329
- **PRESTA**. *Curr. Op. Struct. Biol.*, 1992, vol. 2, 593-596
- **HOOGENBOOM** ; **WINTER**. *J. Mol. Biol.*, 1991, vol. 227, 381
- **MARKS et al.** *J. Mol. Biol.*, 1991, vol. 222, 581
- **COLE et al.** Monoclonal Antibodies and Cancer Therapy. Alan R. Liss, 1985, 77
- **BOERNER et al.** *J. Immunol.*, 1991, vol. 147 (1), 86-95
- **VAN DIJK** ; **VAN DE WINKEL**. *Curr. Opin. Pharmacol.*, 2001, vol. 5, 368-74
- **LI et al.** *Proc. Natl. Acad. Sci. USA*, 2006, vol. 103, 3557-3562
- **EDGAR, R.C.** *Nucleic Acids Research*, 2004, vol. 32 (5), 1792-1797
- **EDGAR, R.C.** *BMC Bioinformatics*, 2004, vol. 5 (1), 113
- **BURTON**. *Molec. Immunol.*, 1985, vol. 22, 161-206
- **BURTON**. *Molec Immunol.*, 1985, vol. 22, 161-206
- **M. DAËRON**. *Annu. Rev. Immunol.*, 1997, vol. 15, 203-234
- **KINET**. *Annu. Rev. Immunol.*, 1991, vol. 9, 457-92
- **CAPEL et al.** *Immunomethods*, 1994, vol. 4, 25-34
- **HAAS et al.** *J. Lab.Clin. Med.*, 1995, vol. 126, 330-41
- *J. boil. Chem.*, 1968, vol. 243, 3558
- Monoclonal Antibodies For Immunotargeting Of Drugs In Cancer Therapy. **ARNON et al.** Monoclonal Antibodies And Cancer Therapy. Alan R. Liss, Inc., 1985
- Antibodies For Drug Delivery. **HELLSTROM et al.** Controlled DrugDelivery. Marcel Dekker, Inc., 1987
- AntibodyCarriers Of Cytotoxic Agents In Cancer Therapy: A Review. **THORPE et al.** Monoclonal Antibodies'84: Biological And Clinical Applications. 1985

- Analysis, Results, and Future Prospective of the Therapeutic Use of Radiolabeled Antibody In Cancer Therapy. Monoclonal Antibodies For CancerDetection And Therapy. Academic Press, 1985
- **THORPE et al.** *Immunol. Rev.*, 1982, vol. 62, 119-58
- **DUBOWCHIK** ; **WALKER**. *Pharm. Therapeutics*, 1999, vol. 83, 67-123
- **NEVILLE et al.** *Biol. Chem.*, 1989, vol. 264, 14653-14661
- **THORPE et al.** *Cancer Res.*, 1987, vol. 47, 5924-5931
- **WAWRZYNCZAK et al.** Immunoconjugates: Antibody Conjugates in Radioimagery and Therapy of Cancer. Oxford U. Press, 1987
- **JOHNSON et al.** *Anticancer Res.*, 1995, vol. 15, 1387-93
- **LAU et al.** *Bioorg-Med-Chem.*, 1995, vol. 3 (10), 1299-1304
- **LAU et al.** *Bioorg-Med-Chem.*, 1995, vol. 3 (10), 1305-12
- Handbook of Pharmaceutical Additives. Synapse Information Resources, Inc., 2001
- Remington’s Pharmaceutical Sciences. Lippincott, Williams & Wilkins, 2000
- Handbook of Pharmaceutical Excipients. 1994